(19) Europäisches Patentamt
European Patent Office
Office européen des brevets

(11) Veröffentlichungsnummer: **0 019 589**
**B1**

# (12) EUROPÄISCHE PATENTSCHRIFT

(45) Veröffentlichungstag der Patentschrift: **09.11.83**

(51) Int. Cl.³: **C 07 C 103/52,** C 12 Q 1/36

(21) Anmeldenummer: **80810155.4**

(22) Anmeldetag: **08.05.80**

(54) **Tripeptidderivate und Verfahren zur Bestimmung von Enzymen mittels derselben.**

(30) Priorität: **11.05.79 CH 4412/79**
**06.05.80 CH 3515/80**

(43) Veröffentlichungstag der Anmeldung:
**26.11.80 Patentblatt 80/24**

(45) Bekanntmachung des Hinweises auf die Patenterteilung:
**09.11.83 Patentblatt 83/45**

(84) Benannte Vertragsstaaten:
**AT BE CH DE FR GB IT LI LU NL SE**

(56) Entgegenhaltungen:
**DE - A - 2 527 932**
**DE - A - 2 629 067**
**FR - A - 2 183 188**
**FR - A - 2 353 063**
**US - A - 4 061 625**
**US - A - 4 147 692**

(73) Patentinhaber: **Pentapharm A.G., Engelgasse 109, CH-4052 Basel (CH)**

(72) Erfinder: **Gundro, Svendsen Lars, Reichensteinerstrasse 15, CH-4153 Reinach BL (CH)**

EP 0 019 589 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Tripeptidderivate und Verfahren zur Bestimmung von Enzymen mittels derselben

Die vorliegende Erfindung betrifft neue Tripeptidderivate, die als Substrate zur quantitativen Bestimmung von proteolytischen Enzymen, insbesondere Enzymen, der Enzymklasse 3.4.21, z. B. von Organ- oder Glandulärkallikreinen, Thrombin und Plasmin, verwendbar sind.

Sogenanntes Organkallikrein oder Glandulärkallikrein wird von verschiedenen Organen und Drüsen, z. B. Pankreasdrüse, Speicheldrüse, Niere, Schleimhaut des Verdauungstraktes, etc., erzeugt und in Form von Proenzym oder in aktiver Form ausgeschieden. Diese Organ- oder Glandulärkallikreine sind in chemischer und physiologischer Hinsicht von Plasmakallikrein verschieden. Bei gewissen pathologischen Zuständen sinkt oder steigt der Organkallikrein-Sekretspiegel unter bzw. über den Normalwert. So sinkt beispielsweise die Kallikreinausscheidung im Harn von Nierenkranken wesentlich unter den Normalwert. Bei Kranken mit Schrumpfnieren ist die Kallikreinausscheidung nahezu vollständig unterbunden. Bei Kranken mit essentiellem Bluthochddruck ist die Ausscheidung von Kallikrein im 24-Stunden-Harn signifikant, durchschnittlich um 50% des Normalwertes, herabgesetzt (siehe z. B. H. S. Margolius in »Chemistry and Biology of the Kallikrein-Kinin System in Health and Disease«, 1974, S. 399–409). Es ist deshalb wichtig, über einfache Methoden zur raschen quantitativen Bestimmung der Organkallikreine zu verfügen. Es ist bekannt, z. B. Harnkallikrein durch Esterolyse von gewissen Argininestern, z. B. Tosyl-argininmethylester (TAME), zu bestimmen. Bei einer verbesserten esterolytischen Methode wird mit Tritium markieter Tosyl-argininmethylester verwendet und die Radioaktivität des esterolytisch freigesetzten markierten Methanols gemessen. Diese esterolytischen Methoden besitzen den Nachteil, daß sie unbiologisch sind, insofern als die Kallikreine proteolytische Enzyme sind, die natürliche Peptidketten amidolytisch und nicht esterolytisch spalten. Estersubstrate besitzen ferner den Nachteil, daß sie durch zahlreiche andere Enzyme unspezifisch, d. h. durch Kallikrein nicht spezifisch gespalten werden. Die unter Verwendung von mit Tritium markiertem TAME durchgeführte Bestimmungsmethode ist insofern umständlich, als vor der Messung der Radioaktivität des mit Tritium markierten Methanols das letztere aus dem Esterolysegemisch mit einer mit Wasser nicht mischbaren Flüssigkeit extrahiert werden muß, da sonst der noch im Esterolysegemisch vorhandene markierte TAME die Messung verunmöglichen würde.

In der deutschen Offenlegungsschrift Nr. 2 527 932 sind Substrate der Formel $R^1-Pro-X-Arg-NH-R^2$ beschrieben, in welcher $R^1$ eine blockierende Gruppe, $-NH-R^2$ eine chromogene Gruppe und X eine Phenylalanyl-, β-Cyclohexylalanyl-, Phenylglycyl- oder Tyrosylgruppe darstellen. Diese Substrate werden durch Plasmakallikrein sehr leicht gespalten und liefern ein farbiges Spaltprodukt $R^2-NH_2$, dessen Menge durch photometrische, spektrophotometrische oder fluoreszenzphotometrische Methoden gemessen werden kann. Man hat versucht, diese Substrate auch zur Bestimmung von Organ- oder Glandulärkallikreinen zu verwenden. Es hat sich jedoch gezeigt, daß die genannten Substrate überraschenderweise gegenüber Organ- oder Glandulärkallikreinen und übrigens auch gegenüber Thrombin unempfindlich sind, d. h. von den letzteren nicht oder nur in geringem Ausmaß gespalten werden.

In der deutschen Offenlegungsschrift Nr. 2 629 067 sind Tripeptidderivate beschrieben, die als Substrate zur Bestimmung von gewissen proteolytischen Enzymen, z. B. Glandulärkallikreinen und Plasmin, verwendbar sind. Zwei Beispiele sind dort genannt, nämlich H-D-Valyl-leucyl-arginyl-p-nitroanilid.dihydrochlorid und H-D-Valyl-leucyl-lysyl-p-nitroanilid.dihydrochlorid. Das erste Tripeptidderivat ist ein Substrat für Glandulärkallikrein, während das zweite Tripeptidderivat ein Substrat für Plasmin darstellt. Die beiden Verbindungen werden durch die genannten Enzyme gespalten, wobei sich p-Nitroanilin bildet, dessen Menge photometrisch oder spektrophotometrisch gemessen werden kann. Die Suszeptibilität des H-D-Valyl-leucyl-arginyl-p-nitroanilid.dihydrochlorids erreicht jedoch gerade knapp den Grenzwert, der für die genaue Bestimmung des Urinkallikreins in unkonzentriertem Urin erforderlich ist. Verwendet man jedoch das genannte Amidsubstrat in konzentriertem Urin, so wird die photometrische Messung des p-Nitroanilins durch die Eigenfarbe des Urins stark gestört.

Die beiden ersten Aminosäuren der Tripeptidkette der in der obengenannten Auslegeschrift beschriebenen zwei Tripeptidderivate tragen hydrophobe Isopropylgruppen in α- bzw. β-Stellung. Man hat versucht, durch Ersetzen der Isopropylgruppen durch aromatische Gruppen, z. B. Phenylreste, jedoch unter Beibehaltung der Grundstruktur der Dipeptidkette, die Hydrophobizität des Substrates und damit dessen Suszeptibilität gegenüber Glandulärkallikrein zu erhöhen. Dieser Versuch ist jedoch fehlgeschlagen. Die durch Einführung von aromatischen Gruppen erhaltenen Tripeptidsubstrate werden durch Glandulärkallikreine überhaupt nicht oder höchstens in sehr geringem Ausmaß gespalten. Es hat sich jedoch gezeigt, daß diese aromatische Gruppen tragenden Tripeptidsubstrate eine überraschend hohe Suszeptibilität gegenüber Plasmin aufweisen. Es wurde ferner unerwarteterweise gefunden, daß man durch Hydrierung der aromatischen Reste in den genannten Tripeptid-Plasminsubstraten zu neuen Tripeptidsubstraten gelangt, die eine erstaunlich hohe Suszeptibilität gegenüber Organ- oder Glandulärkallikreinen und auch Thrombin aufweisen. Man hatte bis jetzt geglaubt, daß man zum Aufbau der Tripeptidketten nur natürlich vorkommende Aminosäuren verwenden könne, um Substrate zu erhalten, die von proteolytischen Enzymen gespalten würden. Es war deshalb überraschend, daß man durch Verwendung von Cyclohexylreste enthaltenden

Aminosäuren, die in der Natur nicht vorkommen, chromogene Substrate erhalten kann, die unerwarteterweise durch Organ- oder Glandulärkallikreine und andere proteolytische Enzyme, z. B. Thrombin und Plasmin, leicht gespalten werden.

In der französischen OS 2 183 188 sind Tripeptidderivate beschrieben, die eine chromogene Gruppe aufweisen, welche durch gewisse proteolytische Enzyme, insbesondere Thrombin, Plasmin und Trypsin, unter Bildung einer farbigen Verbindung abspaltbar ist. Diese Tripeptidderivate werden jedoch durch Organ- oder Glandulärkallikrein nicht gespalten und sind im übrigen gegenüber Thrombin und Plasmin signifikant weniger empfindlich als die im vorliegenden Patent beschriebenen Tripeptidderivate, nämlich etwa 4mal weniger empfindlich gegenüber Thrombin und etwa 10mal weniger empfindlich gegenüber Plasmin.

Im US-Patent 4 061 625 sind Tripeptidderivate mit einer chromogenen Gruppe beschrieben, die von Thrombin und thrombinähnlichen Enzymen unter Bildung einer farbigen Verbindung gespalten werden. Diese Tripeptidderivate sind gegenüber Thrombin um etwa 60% weniger empfindlich als die im vorliegenden Patent beschriebenen, zur Bestimmung von Thrombin vorgesehenen Tripeptidderivate.

In der französischen OS 2 353 063 sind Tripeptidderivate beschrieben, welche durch Plasminogen und Trypsin gespalten werden und daher zur Bestimmung dieser Enzyme geeignet sind, aber durch Organ- oder Glandulärkallikrein, Thrombin und Plasmin nicht oder höchstens äußerst langsam gespalten werden und deshalb zur Bestimmung der zuletzt genannten Enzyme unbrauchbar sind.

Im US-Patent 4 147 692 sind Dipeptidderivate beschrieben, welche als abspaltbare chromogene Gruppe eine 4-Methylcumarylgruppe enthalten und zur Bestimmung von X-Prolyl-dipeptidyl-aminopeptidase bestimmt sind. Diese Derivate enthalten keine Arginyl- oder Lysylgruppe, welche für die Spaltbarkeit durch Enzyme der Klasse 3.4.21. (Organ- oder Glandulärkallikrein, Thrombin und Plasmin) wesentlich ist.

Die Erfindung betrifft neue chromogene Substrate, die eine hohe Suszeptibilität gegenüber gewissen proteolytischen Enzymen, insbesondere Enzymen der Enzymklasse E.C. 3.4.21., z. B. Organ- oder Glanulärkallikreinen, Plasmin und Thrombin, aufweisen und somit zur quantitativen Bestimmung dieser Enzyme verwendbar sind. Diese Substrate sind Tripeptidderivate der Formel

$$H-D-X-Y-Z-R \qquad I$$

in welcher Z eine Arginyl- oder Lysylgruppe und R eine p-Nitrophenylamino-, 2-Naphthylamino-, 4-Methoxy-2-naphthyl-amino-, 4-Methyl-cumaryl-(7)-amino-, 1,3-Di(methoxycarbonyl)-phenyl-(5)-amino-, Chinonylamino- oder Nitrochinonylamino-Gruppe darstellen, und

a) X eine Cyclohexylglycyl-, Cyclohexylalanyl- oder p-Hydroxycyclohexylalanyl-Gruppe und Y eine Alanyl-, α-Aminobutyryl-, Valyl-, Norvalyl-, Leucyl-, Norleucyl-, Isoleucyl-, Prolyl- oder Pipecolinoyl-Gruppe darstellen, oder
b) X eine Cyclohexylglycyl-, Cyclohexylalanyl-, p-Hydroxycyclohexylalanyl-, Phenylalanyl- oder Phenylglycyl-Gruppe und Y eine Phenylalanyl-, Phenylglycyl- oder Tyrosyl-Gruppe darstellen, oder
c) X eine Alanyl-, α-Aminobutyryl-, Valyl-, Norvalyl-, Leucyl-, Norleucyl- oder Isoleucyl-Gruppe und Y eine Cyclohexylglycyl-, Cyclohexylalanyl-, p-Hydroxycyclohexylalanyl-, Phenylglycyl- oder Tyrosyl-Gruppe darstellen, oder
d) X eine Phenylalanyl-, Phenylglycyl- oder Cyclohexylglycyl-Gruppe und Y eine Cyclohexylalanyl-, Cyclohexylglycyl- oder p-Hydroxycyclohexylalanyl-Gruppe darstellen,

und deren Salze mit Säuren.

In der Formel I bezeichnet D die D-Form der durch X dargestellten N-terminalen Aminosäure.

Die Tripeptidderivate der Formel I sind als solche in wäßrigen Medien schwer löslich und werden deshalb vorzugsweise in Form ihrer Salze mit Säuren verwendet, insbesondere ihre Salze mit Mineralsäuren, z. B. HCl, HBr, $H_2SO_4$, $H_3PO_4$, oder mit organischen Säuren, z. B. Ameisensäure, Essigsäure, Propionsäure, Trimethylessigsäure, Methoxyessigsäure, halogenierte Essigsäuren, wie Trichlor- oder Trifluoressigsäure, Glykokollsäure, Milchsäure, Oxalsäure, Malonsäure, Zitronensäure, Benzoesäure, im Kern substituierte aromatische Säuren, wie Toluylsäuren, Chlor- oder Brombenzoesäuren, Methoxybenzoesäuren und Aminobenzoesäuren, Phthalsäure, etc. Die Art der verwendeten Säure ist nicht kritisch, da sie an der Reaktion zwischen den Substraten und den Enzymen nicht teilnimmt.

Die Substrate der Formel I bzw. deren Salze mit Säuren werden unter der Einwirkung von gewissen proteolytischen Enzymen, insbesondere Enzymen der Enzymklasse E.C. 3.4.21. (s. »Enzyme Nomenclature«, Elsevier Scientific Publishing Company, Amsterdam 1973, S. 238 u. f.), z. B. Organ- und Glandulärkallikreinen, Plasmin und Thrombin, hydrolytisch gespalten, wobei ein farbiges oder fluoreszierendes Spaltprodukt der Formel R–H entsteht, dessen Menge durch photometrische, spektrophotometrische, fluoreszenzspektrophotometrische oder elektrochemische Methoden gemessen werden kann. Die neuen Substrate sind somit zur quantitativen Bestimmung von proteolytischen Enzymen, insbesondere Enzymen der Enzymklasse E.C. 3.4.21., welche natürliche

Peptidketten an der Carboxylseite sowohl von Arginin als auch Lysin spalten, z. B. Organ- oder Glandulärkallikreinen, Plasmin, Thrombin sowie deren Inhibitoren und Proenzyme und auch anderer Faktoren, die an der Bildung oder Inhibition der genannten Enzyme beteiligt sind, geeignet.

Die Tripeptidderivate, die in den nachfolgenden Ausführungsbeispielen 3, 5, 7, 9, 12, 13, 14, 15, 16, 19, 22, 30, 32, 33, 34, 35, 36, 37, 59, 65, 66 und 67 beschrieben sind, eignen sich besonders als Substrate zur Bestimmung von Urinkallikrein.

Zur Bestimmung von Kallikrein in menschlichem Sputum kann man die in den nachfolgenden Ausführungsbeispielen 1, 3, 5, 7, 9, 12, 13, 15, 16, 22, 23, 30, 32, 33, 34, 35, 36, 37, 48, 49, 50, 51, 52, 53, 59, 61, 62, 63, 64, 65, 66, 67, 69, 70 und 71 beschriebenen Tripeptidderivate verwenden.

Die in den nachfolgenden Ausführungsbeispielen 1, 2, 4, 6, 8, 10, 16, 17, 18, 19, 22, 25, 28, 29, 31, 32, 33, 35, 37, 38, 39, 40, 41, 42, 43, 46, 52, 57, 58, 61, 62, 65, 67 und 68 beschriebenen Tripeptidderivate bilden eine Gruppe von Substraten, die zur Bestimmung von Plasmin eingesetzt werden können.

Die in den nachfolgenden Ausführungsbeispielen 19, 22, 23, 24, 44, 45, 46, 47, 51, 52, 53, 59, 60, 62, 63, 64, 69, 70 und 71 beschriebenen Tripeptidderivate stellen sehr empfindliche Substrate für die Bestimmung von Thrombin dar.

Die Erfindung bezieht sich ferner auf ein Verfahren zur quantitativen Bestimmung von proteolytischen Enzymen der Enzymklasse E.C. 3.4.21., welche natürliche Peotidketten auf der Carboxylseite sowohl von Arginin als auch von Lysin spalten, z. B. von Organ- oder Glandulärkallikreinen, Plasmin und Thrombin. Das erfindungsgemäße Verfahren ist dadurch gekennzeichnet, daß man Materialien, welche die obengenannten Enzyme enthalten oder in welchen die letzteren gebildet oder verbraucht werden, mit einem Tripeptidderivat der Formel I zur Reaktion bringt und die Menge des durch die hydrolytische Einwirkung des Enzyms auf das Tripeptidderivat gebildeten farbigen oder fluoreszierenden Spaltproduktes R—H durch photometrische, spektrophotometrische, fluoreszenzspektrophotometrische oder elektrochemische Methoden mißt. Man kann nach diesem Verfahren beispielsweise den Enzymgehalt von Enzympräparaten oder den Enzymspiegel in Körperflüssigkeiten des Menschen, z. B. in Urin, Pankreassaft, Darmschleim, Milchdrüsensekret, Schweißdrüsensekret, Sputum und Blut, und der Säugetiere bestimmen. Das erfindungsgemäße Verfahren eignet sich insbesondere zur quantitativen Bestimmung von Organ- oder Glandulärkallikreinen in den obengenannten Körperflüssigkeiten. Mittels dieses Verfahrens können freie Organkallikreine und Schleimkallikreine sowie die sich aus Präkallikreinen bildenden Kallikreine und ferner physiologische oder nicht-physiologische Inhibitoren der Kallikreine und physiologische oder nicht-physiologische Aktivatoren der Präkallikreine bestimmt werden.

Die Tripeptidderivate der Formel I können nach den im folgenden beschriebenen Methoden hergestellt werden:

1. Die chromogene Gruppe R wird an die Carboxylgruppe des C-terminalen Arginins oder Lysins angehängt, wobei deren $\alpha$-Aminogruppe durch eine Schutzgruppe, z. B. eine Carbobenzoxy- oder tert.-Butoxycarbonylgruppe, die $\delta$-Guanidylgruppe im Fall des Arginins durch Protonisierung, z. B. mit HCl, Nitrierung oder Tosylierung, und die $\varepsilon$-Aminogruppe im Fall des Lysins mit einer Carbobenzoxygruppe oder einer p-Methyl-, p-Methoxy- oder p-Chlor-benzyloxycarbonylgruppe oder einer tert.-Butoxycarbonylgruppe geschützt werden. Die C-terminale R—NH-Gruppe dient während des stufenweisen Aufbaus der Peptidkette ebenfalls als Schutzgruppe. Die anderen Schutzgruppen können je nach Bedarf selektiv abgespalten werden, um die weiteren Aminosäurederivate anzuknüpfen, bis die gewünschte Peptidkette vollständig aufgebaut ist. Zum Schluß können die verbleibenden Schutzgruppen vollständig abgespalten werden, ohne daß die R—NH-Gruppe in Mitleidenschaft gezogen wird (siehe z. B. Miklos Bodansky et al., »Peptide Synthesis«, Interschience Publishers, 1966, S. 163—165).
2. Zuerst wird die Peptidkette (nach Bodansky, loc. cit.) aufgebaut, wobei jedoch die C-terminale Carboxylgruppe des Arginins bzw. Lysins mit einer üblichen Estergruppe, z. B. einer Methoxy-, Äthoxy- oder Benzyloxygruppe im Fall des Arginins oder einer tert.-Butoxygruppe im Fall des Lysins, geschützt wird. Die Estergruppen können durch alkalische Hydrolyse abgespalten werden, mit Ausnahme der tert.-Butoxygruppe, die selektiv mittels Trifluoressigsäure abgespalten werden muß. Falls die $\delta$-Guanidylgruppe des Arginins protonisiert ist, wird die genannte Estergruppe mittels Trypsin enzymatisch abgespalten, wobei keine Racemisierung eintritt. Hierauf wird die chromogene Gruppe R—NH— angeknüpft. Wenn die $\delta$-Guanidinogruppe des Arginins durch eine Nitro- oder Tosylgruppe bzw. die $\varepsilon$-Aminogruppe des Lysins durch eine Carbobenzoxy- oder tert.-Butoxygruppe und die N-terminale $\alpha$-Aminogruppe des Tripeptidderivates durch eine Carbobenzoxygruppe oder eine p-Methyl-, p-Methoxy- oder p-Chlor-benzyloxycarbonylgruppe oder eine tert.-Butoxygruppe geschützt sind, so werden diese Schutzgruppen gleichzeitig abgespalten. Die Abspaltung kann durch Behandlung des geschützten Tripeptidderivats mit wasserfreiem HF bei Raumtemperatur durchgeführt werden, wobei alle obengenannten Amino- bzw. $\delta$-Guanidino-Schutzgruppen abgespalten werden. Die Abspaltung kann auch durch Behandlung mit 2N HBr in Eisessig bei Raumtemperatur durchgeführt werden, wenn das geschützte Tripeptidderivat keine Nitro- oder Tosyl-Schutzgruppe enthält.

Die Herstellung der erfindungsgemäßen Tripeptidderivate ist in den nachfolgenden Ausführungsbeispielen ausführlich beschrieben.

Die Analysen der gemäß den Beispielen erhaltenen Eluate und Produkte wurden durch Dünnschichtchromatographie unter Verwendung von mit Siliciumdioxidgel überzogenen Glasplatten (Merck, F 254) durchgeführt. Die Dünnschichtchromatogramme wurden mittels der folgenden Lösungsmittelsysteme entwickelt:

A Chloroform/Methanol (9 : 1)
B n-Propanol/Essigsäureäthylester/Wasser (7 : 1 : 2)
C n-Butanol/Essigsäure/Wasser (3 : 1 : 1)

Es werden die folgenden Abkürzungen verwendet:

| | | |
|---|---|---|
| AcOH | = | Essigsäure |
| Ala | = | Alanin |
| Arg | = | Arginin |
| BOC | = | tert.-Butoxycarbonyl |
| But | = | α-Aminobuttersäure |
| Cbo | = | Carbobenzoxy |
| CHA | = | Cyclohexylalanin |
| CHG | = | Cyclohexylglycin |
| DMF | = | Dimethylformamid |
| DPA | = | 1,3-Di(methoxycarbonyl)-phenyl-(5)-amid |
| DSC | = | Dünnschichtchromatogramm |
| $Et_3N$ | = | Triäthylamin |
| HCA | = | p-Hydroxycyclohexylalanin |
| HMPTA | = | N,N,N′,N″,N″-Hexamethyl-phosphorsäuretriamid |
| Ile | = | Isoleucin |
| Leu | = | Leucin |
| LMS | = | Lösungsmittelsystem |
| Lys | = | Lysin |
| MCA | = | 4-Methyl-cumaryl-(7)-amid |
| MeOH | = | Methanol |
| 4-MeO-2-NA | = | 4-Methoxy-2-naphthylamid |
| NLeu oder Norleu | = | Norleucin |
| NVal oder Norval | = | Norvalin |
| OpNP | = | p-Nitrophenoxy |
| Phe | = | Phenylalanin |
| Ph′Gly | = | Phenylglycin |
| Pip | = | Pipecolinsäure |
| pNA | = | p-Nitroanilid |
| Pro | = | Prolin |
| THF | = | Tetrahydrofuran |
| Tyr | = | Tyrosin |
| Val | = | Valin |

Wenn nichts anderes vermerkt ist, besitzen die Aminosäuren in den Peptidketten die L-Form.

## Beispiel 1

### H-D-CHG-CHA-Arg-pNA · 2 HBr

### 1a. Cbo-Arg-pNA · HCl

In einem Dreihalsrundkolben von 250 ml Inhalt wurden 16,0 g (47,0 mMol) über $P_2O_5$ im Vakuum getrocknetes Cbo-Arg-OH · HCl in 90 ml absolutem HMPTA unter Feuchtigkeitsausschluß bei 20° C gelöst. Bei Zimmertemperatur wurden der erhaltenen Lösung zuerst eine Lösung von 4,74 g (47,0 mMol) $Et_3N$ in 10 ml HMPTA und dann 16,4 g (100 mMol) p-Nitrophenylisocyanat (100%iger Überschuß) portionenweise zugesetzt. Nach 24 Stunden Reaktionszeit bei 20° C wurde das HMPTA im Vakuum größtenteils abdestilliert. Der Rückstand wurde mehrmals mit 30%iger AcOH extrahiert. Der Rückstand wurde verworfen. Die vereinigten Essigsäureextrakte wurden zur weiteren Reinigung auf eine mit 30%iger AcOH äquilibrierte »Sephadex G-15«-Säule aufgetragen und mit 30%iger AcOH eluiert. Diejenige Fraktion des AcOH-Eluates, die sich durch Trypsinbehandlung unter Freisetzung von

p-Nitroanilin spalten ließ, wurde gefriergetrocknet. Man erhielt 12,6 g eines amorphen Pulvers, das im DSC im LMS C einheitlich war. Elementaranalyse und Berechnung aus der Bruttoformel $C_{20}H_{25}N_6O_5Cl$ ergaben die folgenden Werte (die aus der Bruttoformel ermittelten Werte sind in Klammern gesetzt): C = 51,29% (51,67%), H = 5,48% (5,42%), N = 17,92% (18,08%), Cl = 7,50% (7,63%).

1b. 2 HBr · H · Arg-pNA

Unter Feuchtigkeitsausschluß wurden 4,65 g (10 mMol) der Verbindung 1a mit 40 ml 2N HBr in Eisessig unter Rühren 45 Min. bei 20°C behandelt. Das Aminosäurederivat löste sich dabei unter $CO_2$-Entwicklung. Die Reaktionslösung wurde unter intensivem Rühren zu 250 ml absolutem Äther zugetropft, wobei 2 HBr · H-Arg-pNA ausfiel. Die Ätherphase wurde abgesaugt, worauf die feste Phase noch viermal mit je 100 ml absolutem Äther gewaschen wurde, um das als Nebenprodukt gebildete Benzylbromid sowie den Überschuß an HBr und AcOH weitgehend zu entfernen. Der Rückstand wurde in 50 ml Methanol gelöst, mit $Et_3N$ auf pH 4,5 eingestellt und zur Trocknung im Vakuum bei 30°C eingeengt. Dieses so erhaltene Produkt wurde in 75 ml MeOH gelöst und durch eine mit MeOH äquilibrierte Säule von »Sephadex LH-20« (vernetztes Dextrangel) laufen gelassen. Aus einer Fraktion des Eluates erhielt man 4,18 g (91,6% der Theorie) der amorphen Verbindung 1b, die im DSC im LMS C einheitlich war. Elementaranalyse und Berechnung aus der Bruttoformel $C_{12}H_{20}N_6O_3Br_2$ ergaben die folgenden Werte: C = 31,15% (31,60%), H = 4,35% (4,42%), N = 18,84% (18,43%) und Br = 34,81% (35,03%).

1c. Cbo-CHA-Arg-pNA · HBr

4,56 g (10 mMol) der Verbindung 1 b wurden in 30 ml frisch destilliertem DMF gelöst und nach Kühlung auf −10°C unter Rühren mit 1,40 ml (10 mMol) $Et_3N$ versetzt. Das gebildete $Et_3N$ · HBr wurde abfiltriert und mit wenig kaltem DMF gewaschen. Zum Filtrat wurden unter Rühren bei −10°C 4,69 g (11 mMol) Cbo-CHA-OpNP gegeben und unter Feuchtigkeitsausschluß 2−3 Stunden lang reagieren gelassen, wobei die Temperatur der Reaktionslösung allmählich auf etwa 20°C stieg. Die Lösung wurde wieder auf −10°C gekühlt, mit 0,70 ml (5 mMol) $Et_3N$ gepuffert und etwa 2 Stunden bei −10°C und 3 Stunden bei Raumtemperatur reagieren gelassen. Diese Prozedur wurde nochmals mit 0,70 ml $Et_3N$ wiederholt, und nach weiteren 16 Stunden wurde die Reaktionslösung im Vakuum bei 50°C zur Trockne eingeengt. Der Rückstand wurde in 75 ml 50%iger AcOH gelöst und durch Gelfiltration auf einer mit 50%iger AcOH äquilibrierten Säule von »Sephadex G-15« gereinigt. Diejenige Hauptfraktion des AcOH-Eluates, die sich durch Trypsinbehandlung unter Freisetzung von p-Nitroanilin spalten ließ, wurde im Vakuum bei 40°C zur Trockne eingeengt. Der Rückstand wurde in 150 ml MeOH gelöst und nochmals zur Trockne eingeengt. Nach Trocknung des erhaltenen Rückstandes im Vakuumtrockenschrank bei 60°C über $P_2O_5$ erhielt man 5,85 g (88,3% der Theorie) der amorphen Verbindung 1c, die im DSC im LMS C einheitlich war. Elementaranalyse und Berechnung aus der Bruttoformel $C_{29}H_{40}N_7O_6Br$ ergaben die folgenden Werte: C = 52,28% (52,57%), H = 6,16% (6,09%), N = 15,09% (14,80%) und Br = 11,85% (12,06%).

1d. 2 HBr · H-CHA-Arg-pNA

5,30 g (8 mMol) der Verbindung 1c wurden unter Feuchtigkeitsausschluß mit 32 ml 2N HBr in Eisessig unter Rühren 40 Min. lang bei 20°C behandelt. Das Dipeptidderivat löste sich dabei allmählich unter $CO_2$-Entwicklung. Die Reaktionslösung wurde unter intensivem Rühren zu 250 ml absolutem Äther zugetropft, wobei 2 HBr · H-CHA-Arg-pNA ausfiel. Die Ätherphase wurde abgesaugt, worauf die fete Phase noch viermal mit je 100 ml absolutem Äther gewaschen wurde, um das als Nebenprodukt gebildete Benzylbromid sowie den Überschuß an HBr und AcOH weitgehend zu entfernen. Der Rückstand wurde in 50 ml MeOH gelöst. Nach Einstellung des pH auf 4,5 mit $Et_3N$ wurde die Lösung im Vakuum bei 30°C zur Trockne eingeengt. Der so erhaltene Rückstand wurde in 50 ml MeOH gelöst und auf einer mit MeOH äquilibrierten Säule von »Sephadex LH-20« gereinigt. Diejenige Fraktion des MeOH-Eluates, die sich durch Trypsinbehandlung unter Freisetzung von p-Nitroanilin spalten ließ, wurde im Vakuum bei 30°C zur Trockne eingeengt. Nach Trocknung des erhaltenen Rückstandes im Vakuumtrockenschrank bei 40°C über $P_2O_5$ erhielt man 4,48 g (91,9% der Theorie) der amorphen Verbindung 1d, die im DSC im LMS C einheitlich war. Elementaranalyse und Berechnung aus der Bruttoformel $C_{21}H_{35}N_7O_4Br$ ergaben die folgenden Werte: C = 41,80% (41,39%), H = 5,86% (5,79%), N = 16,31% (16,09%) und Br = 25,85% (26,23%).

1e. Cbo-D-CHG-CHA-Arg-pNA · HBr

3,05 g (5 mMol) der Verbindung 1d wurden in 20 ml frisch destilliertem DMF gelöst und nach Kühlung auf −10° C unter Rühren mit 0,70 ml (5 mMol) $Et_3N$ versetzt. Das gebildete $Et_3N$ · HBr wurde abfiltriert und mit wenig kaltem DMF gewaschen. Zum Filtrat wurden unter Rühren bei −10° C 2,27 g (5,5 mMol) Cbo-D-CHG · OpNP gegeben. Man ließ das Reaktionsgemisch unter Feuchtigkeitsausschluß 2−3 Stunden lang reagieren, worauf die Temperatur der Reaktionslösung allmählich auf etwa 20° C stieg. Die Lösung wurde wieder auf −10° C gekühlt, mit 0,35 ml (2,5 mMol) $Et_3N$ gepuffert und etwa 2 Stunden bei −10° C und weitere 3 Stunden bei Raumtemperatur reagieren gelassen. Diese Prozedur wurde nochmals mit 0,35 ml $Et_3N$ wiederholt, und nach weiteren 16 Stunden wurde die Reaktionslösung im Vakuum bei 50° C zur Trockne eingeengt. Der Rückstand wurde in 50 ml 50%iger AcOH gelöst und durch Gelfiltrierung auf einer mit 50%iger AcOH äquilibrierten Säule von »Sephadex G-15« gereinigt. Diejenige Hauptfraktion des AcOH-Eluates, die sich durch Trypsinbehandlung unter Freisetzung von p-Nitroanilin spalten ließ, wurde im Vakuum bei 40° C zur Trockne eingeengt. Der Rückstand wurde in 100 ml MeOH gelöst, worauf die Lösung nochmals zur Trockne eingeengt wurde. Nach Trocknung des erhaltenen Rückstandes im Vakuumtrockenschrank bei 60° C über $P_2O_5$ erhielt man 3,24 g (80,8% der Theorie) der amorphen Verbindung 1e, die im DSC im LMS C einheitlich war. Elementaranalyse und Berechnung aus der Brottuformel $C_{37}H_{53}N_8O_7Br$ ergaben die folgenden Werte: C = 55,72% (55,43%), H = 6,73% (6,66%), N = 14,25% (13,98%) und Br = 9,86% (9,97%).

1f. 2 HBr · H-D-CHG-CHA-Arg-pNA

2,41 g (3 mMol) der Verbindung 1e wurden unter Feuchtigkeitsausschluß mit 12 ml 2N HBr in Eisessig unter Rühren 40 Minuten lang bei 20° C behandelt. Das Tripeptidderivat löste sich dabei allmählich unter Decarboxylierung und gleichzeitiger $CO_2$-Entwicklung. Die Reaktionslösung wurde unter kräftigem Rühren zu 120 ml absolutem Äther zugetropft, wobei 2 HBr · H-D-CHG-CHA-Arg-pNA ausfiel. Die Ätherphase wurde mit einem Filtrierstab abgesaugt, und danach wurde die feste Phase noch viermal mit je 50 ml absolutem Äther gewaschen. Der so erhaltene Rückstand wurde in 40 ml MeOH gelöst. Nach Einstellung des pH auf 4,5 mit $Et_3N$ wurde die Lösung im Vakuum bei 30° C zur Trockne eingeengt. Der Rückstand wurde in 30 ml MeOH gelöst und auf einer mit MeOH äquilibrierten Säule von »Sephadex LH-20« gereinigt. Diejenige Fraktion des MeOH-Eluates, die sich durch Trypsinbehandlung unter Freisetzung von p-Nitroanilin spalten ließ, wurde im Vakuum bei 30° C zur Trockne eingeengt. Zur weiteren Reinigung wurde der vorgereinigte Rückstand in 30 ml 33%iger AcOH gelöst und durch Gelfiltrierung auf einer mit 33%iger AcOH äquilibrierten Säule von »Sephadex G-15« gereinigt. Die Hauptfraktion des AcOH-Eluates, die sich durch Trypsinbehandlung unter Freisetzung von p-Nitroanilin spalten ließ, wurde im Vakuum bei 40° C zur Trockne eingeengt. Nach Trocknung des so erhaltenen Rückstandes im Vakuumtrockenschrank bei 40° C über $P_2O_5$ erhielt man 1,68 g (74,8% der Theorie) der amorphen Verbindung 1f, die im DSC im LMS C einheitlich war. Elementaranalyse und Berechnung aus der Bruttoformel $C_{29}H_{48}N_8O_5Br_2$ ergaben die folgenden Werte: C = 46,18% (46,53%), H = 6,55% (6,46%), N = 15,18% (14,97%) und Br = 21,12% (21,35%).

Die Aminosäureanalyse ergab die zu erwartenden Aminosäuren im richtigen Verhältnis:

Arg: 1,00 — CHA: 0,96 — D-CHG: 0,98.

Beispiel 2

2 HBr · H-D-CHG-Phe-Lys-pNA

2a. BOC-Lys(ε-Cbo)-pNA

In einem Dreihalsrundkolben von 500 ml Inhalt wurden 38,05 g (0,1 Mol) getrocknetes Öl von BOC−Lys(ε-Cbo)−OH in 150 ml absolutem HMPTA unter Feuchtigkeitsausschluß bei 20° C gelöst. Bei Raumtemperatur wurden der erhaltnen Lösung zuerst eine Lösung von 10,12 g (0,1 Mol) $Et_3N$ in 25 ml HMPTA und dann 24,62 g (0,15 Mol) p-Nitrophenylisocyanat (50%iger Überschuß) portionenweise zugesetzt, wobei jedesmal eine heftige $CO_2$-Entwicklung auftrat. Nach 24 Stunden Reaktionszeit bei 20° C wurde das HMPTA im Vakuum größtenteils abdestilliert. Der Rückstand wurde mehrmals mit 2%iger $NaHCO_3$-Lösung und anschließend mit dest. $H_2O$ digeriert. Der so erhaltene Rückstand wurde im Vakuum bei 40° C getrocknet und danach mit warmem MeOH mehrmals extrahiert, bis der Rückstand nur noch das schwerlösliche Nebenprodukt N,N'-bis(p-Nitrophenyl)-harnstoff enthielt. Die MeOH-Extrakte wurden auf 300 ml konzentriert, wobei einige Verunreinigungen flockig anfielen. Nach Filtration wurde das Filtrat (330 ml) auf einer mit MeOH äquilibrierten Säule von »Sephadex LH-20« gereinigt. Die Hauptfraktion des MeOH-Eluates wurde im Vakuum bei 30° C auf ein kleines Volumen eingeengt, wobei eine nadelförmige Substanz auskristallisierte. Die erhaltenen Kristalle wurden

abfiltriert und mit 50 ml eiskaltem MeOH portionenweise gewaschen. Nach Trocknung im Vakuumtrockenschrank über $P_2O_5$ bei 40°C erhielt man 31,1 g (62,1% der Theorie) der kristallinen Verbindung 2a, die im DSC in den LMS A und B einheitlich war. Die Mutterlauge lieferte noch 5,8 g (11,6% der Theorie) der Substanz 2a, die im DSC in den LMS A und B einheitlich war. Elementaranalyse und Berechnung aus der Bruttoformel $C_{25}H_{32}N_4O_7$ ergaben die folgenden Werte: C = 60,23% (59,99%), H = 6,50% (6,44%) und N = 11,38% (11,19%).

2b. $CF_3COOH$ · H-Lys(ε-Cbo)-pNA

25,03 g (50 mMol) der Verbindung 2a wurden unter Feuchtigkeitsausschluß unter intensivem Rühren 60 Minuten bei 20°C mit 50 ml frisch destillierter wasserfreier Trifluoressigsäure behandelt, wobei die BOC-Gruppe unter $CO_2$-Entwicklung und Freisetzung von Isobutylen selektiv abgespalten wurde. Die Reaktionslösung wurde unter kräftigem Rühren zu 750 ml absolutem Äther zugetropft, wobei $CF_3COOH$ · H-Lys(ε-Cbo)-pNA flockig ausfiel. Die Ätherphase wurde mit einem Filtrierstab abgesaugt. Die feste Phase wurde noch viermal mit je 100 ml absolutem Äther behandelt. Der erhaltene Rückstand wurde in 200 ml MeOH gelöst. Nach Einstellung des pH auf 4,5 mit $Et_3N$ wurde die Lösung im Vakuum bei 30°C zur Trockne eingeengt. Der Rückstand wurde in 200 ml MeOH gelöst und auf einer mit MeOH äquilibrierten Säule von »Sephadex LH-20« gereinigt. Die Hauptfraktion des MeOH-Eluates, die sich im DSC im LMS C einheitlich verhielt, wurde im Vakuum bei 30°C eingeengt. Nach Trocknung des so erhaltenen Rückstandes im Vakuumtrockenschrank bei 40°C über $P_2O_5$ erhielt man 22,64 g (88,0% der Theorie) der amorphen Verbindung 2b. Elementaranalyse und Berechnung aus der Bruttoformel $C_{22}H_{25}N_4O_7F_3$ ergaben die folgenden Werte: C = 51,66% (51,36%), H = 4,88% (4,90%) und N = 11,08% (10,89%).

2c. BOC-Phe-Lys(ε-Cbo)-pNA

7,72 g (15 mMol) der Verbindung 2b wurden in 50 ml frisch destilliertem DMF gelöst und nach Kühlung auf −10°C unter Rühren mit 6,38 g (16,5 mMol) BOC-Phe-OpNP und 2,09 ml (15 mMol) $Et_3N$ versetzt. Man ließ das Gemisch unter Feuchtigkeitsausschluß 3 Stunden reagieren, wobei die Reaktionstemperatur allmählich auf Raumtemperatur stieg. Die Lösung wurde erneut auf −10°C gekühlt und mit 1,05 ml (7,5 mMol) $Et_3N$ gepuffert. Nach 5 Stunden Reaktionszeit wurde diese Prozedur nochmals mit 1,05 ml $Et_3N$ wiederholt. Nach 16 Stunden Reaktionszeit bei 20°C wurde die Reaktionslösung im Vakuum bei 50°C zur Trockne eingeengt. Der Rückstand wurde in 150 ml MeOH gelöst und durch Gelfiltrierung auf einer mit MeOH äquilibrierten Säule von »Sephadex LH-20« gereinigt. Die erste Hauptfraktion des MeOH-Eluates, die sich im DSC in den LMS A und B einheitlich verhielt, wurde im Vakuum bei 30°C auf ein kleines Volumen eingeengt, wobei die gewünschte Substanz auskristallisierte. Die Kristalle wurden abfiltriert und mit 30 ml eiskaltem MeOH portionenweise nachgewaschen. Aus der Mutterlauge ließ sich eine zusätzliche Menge von 1,0 g kristalliner Substanz gewinnen. Nach Trocknung im Vakuumtrockenschrank bei 40°C über $P_2O_5$ erhielt man 7,72 g (79,5% der Theorie) der Verbindung 2c, die im DSC in den LMS A und B einheitlich war. Elementaranalyse und Berechnung aus der Bruttoformel $C_{34}H_{41}N_5O_8$ ergaben die folgenden Werte: C = 62,88% (63,05%), H = 6,42% (6,38%) und N = 11,06% (10,81%).

2d. $CF_3COOH$ · Phe-Lys(ε-Cbo)-pNA

3,24 g (5 mMol) der Verbindung 2c wurden unter Feuchtigkeitsausschluß mit 10 ml frisch destillierter wasserfreier Trifluoressigsäure unter intensivem Rühren 60 Minuten bei 20°C behandelt. Die Reaktionslösung wurde unter kräftigem Rühren zu 100 ml absolutem Äther zugetropft, wobei $CF_3COOH$ · H-Phe-Lys(ε-Cbo)-pNA amorph ausfiel. Die Ätherphase wurde abgesaugt. Der feste Rückstand wurde noch dreimal mit je 30 ml absolutem Äther gewaschen. Der so erhaltene Rückstand wurde in 50 ml MeOH gelöst. Nach Einstellung des pH auf 4,5 mit $Et_3N$ wurde die Lösung im Vakuum bei 30°C zur Trockne eingeengt. Der Rückstand wurde in 75 ml MeOH gelöst und auf einer mit MeOH äquilibrierten Säule von »Sephadex LH-20« gereinigt. Die Hauptfraktion des MeOH-Eluates, die sich im DSC im LMS C einheitlich verhielt, wurde im Vakuum bei 30°C eingeengt. Nach Trocknung des Rückstandes im Vakuumtrockenschrank bei 40°C über $P_2O_5$ erhielt man 2,95 g (89,2% der Theorie) der amorphen Verbindung 2d. Elementaranalyse und Berechnung aus der Bruttoformel $C_{31}H_{34}N_5O_8F_3$ ergaben die folgenden Werte: C = 56,82% (56,27%), H = 5,16% (5,18%) und N = 10,63% (10,59%).

2e. Cbo-D-CHG-Phe-Lys(ε-Cbo)-pNA

1,99 g (3 mMol) der Verbindung 2d wurden in 15 ml frisch destilliertem DMF gelöst und nach Kühlung

auf $-10°C$ unter Rühren mit 1,36 g (3,3 mMol) Cbo-D-CHG-OpNP und 0,42 ml (3 mMol) $Et_3N$ versetzt. Man ließ das Gemisch unter Feuchtigkeitsausschluß während 3 Stunden reagieren, wobei die Temperatur allmählich auf 20°C stieg. Die Reaktionslösung wurde erneut auf $-10°C$ gekühlt und mit 0,21 ml (1,5 mMol) $Et_3N$ gepuffert. Nach einer Reaktionszeit von 5 Stunden bei $-10°C$ ließ man die Temperatur des Reaktionsgemisches allmählich auf Zimmertemperatur steigen. Diese Prozedur wurde nochmals mit 0,21 ml $Et_3N$ durchgeführt, wobei die Reaktionszeit etwa 16 Stunden betrug. Das Reaktionsgemisch wurde im Vakuum bei 50°C zur Trockne eingeengt, worauf der Rückstand in 50 ml MeOH gelöst und durch Gelfiltrierung auf einer mit MeOH äquilibrierten Säule von »Sephadex LH-20« gereinigt wurde. Die erste Hauptfraktion des MeOH-Eluates, die sich im DSC in den LMS A und B einheitlich verhielt, wurde im Vakuum bei 30°C zur Trockne eingeengt. Nach Trocknung des Rückstandes im Vakuumtrockenschrank bei 40°C über $P_2O_5$ erhielt man 2,03 g (82,4% der Theorie) der teilweise kristallinen Verbindung 2e. Elementaranalyse und Berechnung aus der Bruttoformel $C_{45}H_{52}N_6O_9$ ergaben die folgenden Werte: C = 66,22% (65,84%), H = 6,32% (6,38%) und N = 10,49% (10,24%).

2f. 2 HBr · H-D-CHG-Phe-Lys-pNA

1,64 g (2 mMol) der Verbindung 2e wurden unter Feuchtigkeitsausschluß unter Rühren 40 Minuten bei 20°C mit 12 ml 2N HBr in Eisessig behandelt. Das Tripeptidderivat löste sich allmählich unter gleichzeitiger Abspaltung der beiden Schutzgruppen Cbo und BOC, wobei $CO_2$-Entwicklung auftrat. Die Reaktionslösung wurde unter kräftigem Rühren zu 100 ml absolutem Äther getropft, wobei 2 HBr · H-D-CHG-Phe-Lys-pNA flockig ausfiel. Die Ätherphase wurde nach 30 Minuten abgesaugt, worauf die feste Phase noch viermal mit je 25 ml abs. Äther gewaschen wurde. Der erhaltene Rückstand wurde in 40 ml MeOH gelöst. Nach Einstellung des pH auf 4,5 mit $Et_3N$ wurde die Lösung im Vakuum bei 30°C zur Trockne eingeengt. Der Rückstand wurde in 30 ml MeOH gelöst und auf einer mit MeOH äquilibrierten Säule von »Sephadex LH-20« gereinigt. Diejenige Hauptfraktion des MeOH-Eluates, die sich durch Trypsinbehandlung unter Freisetzung von p-Nitroanilin spalten ließ, wurde im Vakuum bei 30°C zur Trockne eingeengt. Zur weiteren Reinigung wurde das vorgereinigte Produkt in 25 ml 33%iger AcOH gelöst und durch Gelfiltrierung auf einer mit 33%iger AcOH äquilibrierten Säule von »Sephadex G-15« gereinigt. Diejenige Fraktion des AcOH-Eluates, die sich durch Trypsinbehandlung unter Freisetzung von p-Nitroanilin spalten ließ, wurde im Vakuum bei 40°C zur Trockne eingeengt. Nach Trocknung des so erhaltenen Rückstandes im Vakuumtrockenschrank bei 40°C über $P_2O_5$ erhielt man 1,13 g (79,1% der Theorie) der amorphen Verbindung 2f, die im DSC im LMS C einheitlich war. Elementaranalyse und Berechnung aus der Bruttoformel $C_{29}H_{42}N_6O_5Br_2$ ergaben die folgenden Werte: C = 48,41% (48,75%), H = 6,02% (5,93%), N = 12,11% (11,76%) und Br = 22,02% (22,37%).

Die Aminosäureanalyse ergab die zu erwartenden Aminosäuren im richtigen Verhältnis:

Phe: 1,00 — Lys: 0,98 — D-CHG: 1,02.

Beispiel 3

2 HBr · H-D-Val-CHA-Arg-MCA

3b. 2 HBr · H-Arg-MCA

13,0 g (25,9 mMol) käufliches Cbo-Arg-MCA · HCl wurden mit 104 ml (208 mMol) einer Lösung von 2N HBr in Eisessig gemäß Beispiel 1b deblockiert. Der trockene Rückstand wurde in 400 ml MeOh gelöst und auf einer Säule von »Sephadex LH-20« gereinigt. Diejenige Fraktion des MeOH-Eluates, die sich durch Trypsinbehandlung unter Freisetzung von 4-Methyl-7-amino-cumarin spalten ließ, wurde im Vakuum bei 30°C zur Trockne eingeengt. Nach Trocknung des erhaltenen Rückstandes im Vakuumtrockenschrank bei 40°C über $P_2O_5$ erhielt man 11,2 g (87,7% der Theorie) der amorphen Verbindung 3b, die im DSC im LMS C einheitlich war. Elementaranalyse und Berechnung aus der Bruttoformel $C_{16}H_{23}N_5O_3Br_2$ ergaben die folgenden Werte: C = 39,40% (38,96%), H = 4,61% (4, 70%), N = 14,48% (14,20%) und Br = 31,90% (32,40%).

3c. Cbo-CHA-Arg-MCA · HBr

4,93 g (10 mMol) der Verbindung 3b und 4,69 g (11 mMol) Cbo-CHA-OpNP wurden zu 75 ml frisch destilliertem DMF gegeben. Nach Kühlung auf $-10°C$ wurden unter Rühren zuerst 1,40 ml (10 mMol) und anschließend 0,70 ml (5 mMol) $Et_3N$ zugegeben. Man ließt das Gemisch unter Feuchtigkeitsausschluß zuerst 3 Stunden bei $-10°C$ und dann weitere 4 Stunden bei Raumtemperatur reagieren. Die

Reaktionslösung wurde erneut auf −10° C gekühlt, mit 0,70 ml $Et_3N$ gepuffert und über Nacht bei 20° C gerührt. Das Reaktionsgemisch wurde im Vakuum bei 50° C zur Trockne eingeengt, worauf der Rückstand in 200 ml 50%iger AcOH gelöst und auf einer Säule von »Sephadex G-15« gereinigt wurde. Diejenige Fraktion des AcOH-Eluates, die sich durch Trypsinbehandlung unter Freisetzung von 4-Methyl-7-amino-cumarin spalten ließ, wurde im Vakuum bei 40° C zur Trockne eingeengt. Nach Trocknung des erhaltenen Rückstandes im Vakuumtrockenschrank bei 60° C über $P_2O_5$ erhielt man 5,95 g (85,0% der Theorie) der kristallinen Verbindung 3c, die im DSC im LMS C einheitlich war. Elementaranalyse und Berechnung aus der Bruttoformel $C_{33}H_{43}N_6O_6Br$ ergaben die folgenden Werte: C = 56,33% (56,65%), H = 6,28% (6,19%), N = 12,25% (12,01%) und Br = 11,30% (11,42%).

3d. 2 HBr · H-CHA-Arg-MCA

5,60 g (8 mMol) der Verbindung 3c wurden gemäß Beispiel 1d mit 32 ml 2N HBr in Eisessig deblockiert. Das erhaltene Rohprodukt wurde in 100 ml MeOH gelöst und auf einer Säule von »Sephadex LH-20« gereinigt. Diejenige Fraktion des MeOH-Eluates, die sich durch Trypsinbehandlung unter Freisetzung von 4-Methyl-7-amino-cumarin spalten ließ, wurde im Vakuum bei 30° C zur Trockne eingeengt. Nach Trocknung des erhaltenen Rückstandes im Vakuumtrockenschrank bei 40° C über $P_2O_5$ erhielt man 4,76 g (92,1% der Theorie) der amorpen Verbindung 3d, die im DSC im LMS C einheitlich war. Elementaranalyse und Berechnung aus der Bruttoformel $C_{25}H_{38}N_6O_4Br_2$ ergaben die folgenden Werte: C = 47,02% (46,45%), H = 6,02% (5,93%), N = 13,21% (13,00%) und Br = 24,48% (24,72%).

3e. Cbo-D-Val-CHA-Arg-MCA · HBr

3,23 g (5 mMol) der Verbindung 3d wurden gemäß Beispiel 1e mit 2,05 g (5,5 mMol) Cbo-D-Val-OpNP umgesetzt. Das erhaltene Rohprodukt wurde in 75 ml 50%iger AcOH gelöst und auf einer Säule von »Sephadex G-15« gereinigt. Diejenige Fraktion des AcOH-Eluates, die sich durch Trypsinbehandlung unter Freisetzung von 4-Methyl-7-amino-cumarin spalten ließ, wurde im Vakuum bei 40° C zur Trockne eingeengt. Nach Trocknung des Rückstandes im Vakuumtrockenschrank bei 60° C über $P_2O_5$ erhielt man 3,21 g (80,4% der Theorie) der amorphen Verbindung 3e, die im DSC im LMS C einheitlich war. Elementaranalyse und Berechnung aus der Bruttoformel $C_{38}H_{52}N_7O_7Br$ ergaben die folgenden Werte: C = 57,05% (57,14%), H = 6,61% (6,56%), N = 12,49% (12,28%) und Br = 9,82% (10,00%).

3f. 2 HBr · H-D-Val-CHA-Arg-MCA

2,40 g (3 mMol) der Verbindung 3d wurden gemäß Beispiel 1f mit 12 ml 2N HBr in Eisessig deblockiert. Das erhaltene Rohprodukt wurde in 50 ml MeOH gelöst und auf einer Säule von »Sephadex LH-20« gereinigt. Diejenige Fraktion des MeOH-Eluates, die sich durch Trypsinbehandlung unter Freisetzung von 4-Methyl-7-amino-cumarin spalten ließ, wurde im Vakuum bei 30° C zur Trockne eingeengt. Zur weiteren Reinigung wurde das vorgereinigte Produkt in 40 ml 50%iger AcOH gelöst und durch Gelfiltrierung an einer Säule von »Sephadex G-15« gereinigt. Die Hauptfraktion des AcOH-Eluates, die sich durch Trypsinbehandlung unter Freisetzung von 4-Methyl-7-amino-cumarin spalten ließ, wurde im Vakuum bei 40° C zur Trockne eingeengt. Nach Trocknung des Rückstandes im Vakuumtrockenschrank bei 40° C über $P_2O_5$ erhielt man 1,73 g (77,3% der Theorie) der amorpen Verbindung 3f, die im DSC im LMS C einheitlich war. Elementaranalyse und Berechnung aus der Bruttoformel $C_{30}H_{47}N_7O_5Br_2$ ergaben die folgenden Werte: C = 48,12% (48,33%), H = 6,43% (6,35%), N = 13,38% (13,15%) und Br = 21,18% (21,44%).

Die Aminosäureanalyse ergab die zu erwartenden Aminosäuren im richtigen Verhältnis:

Val: 1,00 — Arg: 1,02 — D-CHA: 0,97.

Beispiel 4

2 HBr · H-D-CHG-Phe-Lys-MCA

4a. BOC-Lys(ε-Cbo)-MCA

In einem Dreihalskolben von 1000 ml Inhalt wurden 38,05 g (0,1 Mol) getrocknetes BOC-Lys(ε-Cbo)-OH in einem Gemisch von 50 ml frisch destilliertem wasserfreiem DMF und 300 ml absolutem THF bei 20° C gelöst. Der auf −10° C gekühlten Lösung wurde unter Feuchtigkeitsausschluß und unter Rühren eine Lösung von 10,2 g (0,1 Mol) $Et_3N$ in 75 ml THF zugesetzt. Dann wurde innerhalb von 20

Minuten eine Lösung von 13,65 g (0,1 Mol) Chlorameisensäure-isobutylester in 50 ml THF zugetropft, wobei man die Reaktionstemperatur nie über −5° C steigen ließ. Nach einer Reaktionszeit von etwa 10 Minuten bei einer Temperatur von −10° C bis −5° C wurde eine Lösung von 17,52 g (0,1 Mol) 4-Methyl-7-amino-cumarin in 75 ml DMF innerhalb von 25 Minuten zugetropft, wobei man die Temperatur nie über −5° C steigen ließ. Das Reaktionsgemisch wurde anschließend 1 Stunde bei −5° C und über Nacht bei Raumtemperatur gerührt und dann wieder auf −10° C gekühlt. Das auskristallisierte $Et_3N \cdot HCl$ wurde abfiltriert. Das Filtrat wurde im Vakuum bei 50° C zur Trockne eingeengt. Der Rückstand wurde in 500 ml MeOH gelöst und durch Gelfiltrierung auf einer Säule von »Sephadex LH-20« gereinigt. Das MeOH-Eluat lieferte außer dem gewünschten Produkt BOC-Lys(ε-Cbo)-MCA noch drei weitere Fraktionen, welche das Nebenprodukt N-[4-Methylcumaryl)(7)]-karbaminsäureisobutylester und die Ausgangsprodukte BOC-Lys(ε-Cbo)-OH bzw. 7-Amino-4-methyl-cumarin enthielten. Die das Produkt BOC-Lys(ε-Cbo)-MCA enthaltende Fraktion wurde im Vakuum bei 30° C zur Trockne eingeengt. Nach Trocknung des Rückstandes im Vakuumtrockenschrank bei 50° C über $P_2O_5$ erhielt man 26,3 g (48,9% der Theorie) der teilweise kristallinen Verbindung 4a, die im DSC in den LMS A und B einheitlich war. Elementaranalyse und Berechnung aus der Bruttoformel $C_{29}H_{35}N_3O_7$ ergaben die folgenden Werte: C = 64,90% (64,79%), H = 6,52% (6,56%) und N = 7,88% (7,82%).

4b. $CF_3COOH \cdot$ H-Lys(ε-Cbo)-MCA

21,5 g (40 mMol) der Verbindung 4a wurden gemäß Beispiel 2b mit 60 ml Trifluoressigsäure deblockiert. Das nach Aufarbeitung erhaltene Rohprodukt wurde in 250 ml MeOH gelöst und durch Gelfiltrierung auf einer Säule von »Sephadex LH-20« gereinigt. Die erste Hauptfraktion des MeOH-Eluates, die sich im DSC im LMS C einheitlich verhielt, wurde im Vakuum bei 30° C zur Trockne eingeengt. Nach Trocknung des Rückstandes im Vakuumtrockenschrank bei 40° C über $P_2O_5$ erhielt man 19,5 g (88,4% der Theorie) der amorphen Verbindung 4b. Elementaranalyse und Berechnung aus der Bruttoformel $C_{26}H_{28}N_3O_7F_3$ ergaben die folgenden Werte: C = 57,02% (56,62%), H = 5,20% (5,12%) und N = 7,58% (7,62%).

4c. BOC-Phe-Lys(ε-Cbo)-MCA

5,52 g (10 mMol) der Verbindung 4b wurden gemäß Beispiel 2c mit 4,25 g (11 mMol) BOC-Phe-OpNP umgesetzt. Das nach Aufarbeitung erhaltene Rohprodukt wurde in 100 ml MeOH gelöst und durch Gelfiltrierung auf einer Säule von »Sephadex LH-20« gereinigt. Die Hauptfraktion des MeOH-Eluates, die sich im DSC in den LMS A und B einheitlich verhielt, wurde im Vakuum bei 30° C zur Trockne eingeengt. Nach Trocknung des Rückstandes im Vakuumtrockenschrank bei 50° C über $P_2O_5$ erhielt man 5,78 g (84,4% der Theorie) der teilweise kristallinen Verbindung 4c. Elementaranalyse und Berechnung aus der Bruttoformel $C_{38}H_{44}N_4O_8$ ergaben die folgenden Werte: C = 66,09% (66,65%), H = 6,44% (6,48%) und N = 8,32% (8,18%).

4d. $CF_3COOH \cdot$ H-Phe-Lys(ε-Cbo)-MCA

3,42 g (5 mMol) der Verbindung 4c wurden gemäß Beispiel 2d mit 15 ml Trifluoressigsäure deblockiert. Das nach Aufarbeitung erhaltene Rohprodukt wurde in 75 ml MeOH gelöst und durch Gelfiltrierung auf einer Säule von »Sephadex LH-20« gereinigt. Die Hauptfraktion des MeOH-Eluates, die sich im DSC im LMS C einheitlich verhielt, wurde im Vakuum bei 30° C zur Trockne eingeengt. Nach Trocknung des Rückstandes im Vakuumtrockenschrank bei 40° C über $P_2O_5$ erhielt man 3,41 g (97,6% der Theorie) der amorphen Verbindung 4d. Elementaranalyse und Berechnung aus der Bruttoformel $C_{35}H_{37}N_4O_8F_3$ ergaben die folgenden Werte: C = 59,54% (60,16%), H = 5,31% (5,34%) und N = 8,33% (8,02%).

4e. Cbo-D-CHG-Phe-Lys(ε-Cbo)-MCA

2,10 g (3 mMol) der Verbindung 4d wurden gemäß Beispiel 2e mit 1,36 g (3,3 mMol) Cbo-D-CHG-OpNP umgesetzt. Das nach Aufarbeitung erhaltene Rohprodukt wurde in 40 ml MeOH gelöst und durch Gelfiltrierung auf einer Säule von »Sephadex LH-20« gereinigt. Die erste Hauptfraktion des MeOH-Eluates, die sich im DSC in den LMS A und B einheitlich verhielt, wurde im Vakuum bei 30° C zur Trockne eingeengt. Nach Trocknung des Rückstandes im Vakuumtrockenschrank bei 60° C über $P_2O_5$ erhielt man 2,12 g (82,4% der Theorie) der amorphen Verbindung 4e. Elementaranalyse und Berechnung aus der Bruttoformel $C_{49}H_{55}N_5O_9$ ergaben die folgenden Werte: C = 69,03% (68,59%), H = 6,49% (6,46%) und N = 8,32% (8,16%).

4f. 2 HBr · H-D-CHG-Phe-Lys-MCA

1,72 g (2 mMol) der Verbindung 4e wurden gemäß Beispiel 2f mit 12 ml 2N HBr in Eisessig deblockiert. Das nach Aufarbeitung erhaltene Rohprodukt wurde in 30 ml MeOH gelöst und auf einer Säule von »Sephadex LH-20« gereinigt. Diehenige Fraktion des MeOH-Eluates, die sich durch Trypsinbehandlung unter Freisetzung von 4-Methyl-7-amino-cumarin spalten ließ, wurde im Vakuum bei 30°C zur Trockne eingeengt. Zur weiteren Reinigung wurde das vorgereinigte Produkt in 40 ml 50%iger AcOH gelöst und durch Gelfiltrierung auf einer Säule von »Sephadex G-15« gereinigt. Die Hauptfraktion des AcOH-Eluates, die sich durch Trypsinbehandlung unter Freisetzung von 4-Methyl-7-amino-cumarin spalten ließ, wurde im Vakuum bei 40°C zur Trockne eingeengt. Nach Trocknung des Rückstandes im Vakuumtrockenschrank bei 40°C über $P_2O_5$ erhielt man 1,10 g (73,2% der Theorie) der amorphen Verbindung 4f, die im DSC im LMS C einheitlich war. Elementaranalyse und Berechnung aus der Bruttoformel $C_{33}H_{45}N_5O_5Br_2$ ergaben die folgenden Werte: C = 53,20% (52,74%), H = 6,12% (6,04%), N = 9,18% (9,32%) und Br = 21,16% (21,26%).

Die Aminosäureanalyse ergab die zu erwartenden Aminosäuren im richtigen Verhältnis:

Phe: 1,00 — Lys: 0,99 — D-CHG: 0,97.

Beispiel 5

2 HBr · H-D-Val-CHA-Arg-DPA

5a. Cbo-Arg-DPA · HCl

In einem Dreihalsrundkolben von 1000 ml Inhalt wurden 34,48 g (0,1 Mol) getrocknetes Cbo-Arg-OH · HCl in einem Gemisch von 150 ml frisch destilliertem wasserfreiem DMF und 300 ml absolutem THF bei 20°C gelöst. Der auf −10°C gekühlten Lösung wurden unter Rühren und Feuchtigkeitsausschluß 10,2 g (0,1 Mol) $Et_3N$ zugesetzt. Dann wurde dem Gemisch innerhalb von 20 Minuten eine Lösung von 13,65 g (0,1 Mol) Chlorameisensäureisobutylester in 50 ml THF tropfenweise zugesetzt, wobei man die Reaktionstemperatur nie über −5°C steigen ließ. Nach einer zusätzlichen Reaktionszeit von 10 Minuten bei einer Temperatur von −10°C bis −5°C wurde dem Reaktionsgemisch eine Lösung von 20,92 g (0,1 Mol) 5-Amino-isophthalsäure-dimethylester in 75 ml DMF innerhalb von 30 Minuten tropfenweise zugesetzt, wobei man die Reaktionstemperatur immer unterhalb −5°C hielt. Man ließ das Reaktionsgemisch noch 1 Stunde bei −5°C weiterreagieren. Es wurde über Nacht bei 20°C gerührt und dann auf −15°C gekühlt, um das $Et_3N$ · HCl auskristallisieren zu lassen. Das gebildete $Et_3N$ · HCl wurde abfiltriert und mit wenig kaltem DMF nachgewaschen. Das Filtrat zusammen mit der Waschlösung wurde im Vakuum bei 50°C zur Trockne eingeengt. Der Rückstand wurde in 1000 ml 50%iger AcOH gelöst und durch Gelfiltrierung auf einer mit 50%iger AcOH äquilibrierten Säule von »Sephadex G-15« gereinigt. Diejenige Hauptfraktion des AcOH-Eluates, die sich durch Trypsinbehandlung unter Freisetzung von 5-Amino-isophthalsäure-dimethylester spalten ließ, wurde im Vakuum bei 40°C zur Trockne eingeengt. Nach Trocknung des Rückstandes im Vakuumtrockenschrank bei 50°C über $P_2O_5$ erhielt man 24,6 g (45,9% der Theorie) der amorphen Verbindung 5a, die im DSC im LMS C einheitlich war. Elementaranalyse und Berechnung aus der Bruttoformel $C_{24}H_{30}N_5O_7Cl$ ergaben die folgenden Werte: C = 53,21% (53,78%), H = 5,71% (5,64%), N = 13,20% (13,07%) und Cl = 6,52% (6,62%).

5b. 2 HBr · H-Arg-DPA

21,44 g (40 mMol) der Verbindung 5a wurden gemäß Beispiel 1b deblockiert. Nach Aufarbeitung wurde das erhaltene Rohprodukt in 250 ml MeOH gelöst und durch Gelfiltrierung auf einer Säule von »Sephadex LH-20« gereinigt. Diejenige Fraktion des MeOH-Eluates, die sich durch Trypsinbehandlung unter Freisetzung von 5-Amino-isophthalsäure-dimethylester spalten ließ, wurde im Vakuum zur Trockne eingeengt. Nach Trocknung des Rückstandes im Vakuumtrockenschrank bei 40°C über $P_2O_5$ erhielt man 19,63 g (93,1% der Theorie) der amorphen Verbindung 5b, die im DSC im LMS C einheitlich war. Elementaranalyse und Berechnung aus der Bruttoformel $C_{16}H_{25}N_5O_5Br_2$ ergaben die folgenden Werte: C = 36,82% (36,45%), H = 4,67% (4,78%), N = 13,45% (13,28%) und Br = 29,85% (30,31%).

5c. Cbo-CHA-Arg-DPA · HBr

5,27 g (10 mMol) der Verbindung 5b wurden gemäß Beispiel 1c mit 4,69 g (11 mMol) Cbo-CHA-OpNP umgesetzt. Das nach Aufarbeitung erhaltene Rohprodukt wurde in 200 ml 50%iger AcOH gelöst und auf einer Säule von »Sephadex G-15« gereinigt. Diejenige Fraktion des AcOH-Eluates, die sich durch

Trypsinbehandlung unter Freisetzung von 5-Amino-isophthalsäure-dimethylester spalten ließ, wurde im Vakuum bei 40°C zur Trockne eingeengt. Nach Trocknung des Rückstandes im Vakuumtrockenschrank bei 60°C über $P_2O_5$ erhielt man 6,06 g (82,6% der Theorie) der amorphen Verbindung 5c, die im DSC im LMS C einheitlich war. Elementaranalyse und Berechnung aus der Bruttoformel $C_{33}H_{45}N_6O_8Br$ ergaben die folgenden Werte: C = 53,74% (54,02%), H = 6,28% (6,18%), N = 11,90% (11,46%) und Br = 10,68% (10,89%).

5d. 2 HBr · H-CHA-Arg-DPA

5,87 g (8 mMol) der Verbindung 5c wurden gemäß Beispiel 1d mit 32 ml 2N HBr in Eisessig deblockiert. Das nach Aufarbeitung erhaltene Rohprodukt wurde in 100 ml MeOH gelöst und auf einer Säule von »Sephadex LH-20« gereinigt. Diejenige Fraktion des MeOH-Eluates, die sich durch Trypsinbehandlung unter Freisetzung von 5-Amino-isophthalsäure-dimethylester spalten ließ, wurde im Vakuum bei 30°C zur Trockne eingeengt. Nach Trocknung des Rückstandes im Vakuumtrockenschrank bei 40°C über $P_2O_5$ erhielt man 4,79 g (88,0% der Theorie) der amorphen Verbindung 5d, die im DSC im LMS C einheitlich war. Elementaranalyse und Berechnung aus der Bruttoformel $C_{25}H_{40}N_6O_6Br$ ergaben die folgenden Werte: C = 43,75% (44,13%), H = 5,88% (5,93%), N = 12,69% (12,35%) und Br = 23,22% (23,49%).

5e. Cbo-D-Val-CHA-Arg-DPA · HBr

3,40 g (5 mMol) der Verbindung 5d wurden gemäß Beispiel 1e mit 2,05 g (5,5 mMol) Cbo-D-Val-OpNP umgesetzt. Das nach Aufarbeitung erhaltene Rohprodukt wurde in 100 ml 50%iger AcOH gelöst und auf einer Säule von »Sephadex G-15« gereinigt. Diejenige Fraktion des AcOH-Eluates, die sich durch Trypsinbehandlung unter Freisetzung von 5-Amino-isophthalsäure-dimethylester spalten ließ, wurde im Vakuum bei 40°C zur Trockne eingeengt. Nach Trocknung des Rückstandes im Vakuumtrockenschrank bei 60°C über $P_2O_5$ erhielt man 3,25 g (78,1% der Theorie) der amorphen Verbindung 5e, die im DSC im LMS C einheitlich war. Elementaranalyse und Berechnung aus der Bruttoformel $C_{38}H_{54}N_7O_9Br$ ergaben die folgenden Werte: C = 53,95% (54,80%), H = 6,65% (6,54%), N = 12,07% (11,77%) und Br = 9,38% (9,59%).

5f. 2 HBr · H-D-Val-CHA-Arg-DPA

2,50 g (3 mMol) der Verbindung 5e wurden gemäß Beispiel 1f mit 12 ml 2N HBr in Eisessig deblockiert. Das nach Aufarbeitung erhaltene Rohprodukt wurde in 50 ml MeOH gelöst und auf einer Säule von »Sephadex LH-20« vorgereinigt. Diejenige Fraktion des MeOH-Eluates, die sich durch Trypsinbehandlung unter Freisetzung von 5-Amino-isophthalsäure-dimethylester spalten ließ, wurde im Vakuum bei 30°C zur Trockne eingeengt. Das vorgereinigte Produkt wurde in 50 ml 50%iger AcOH gelöst und durch Gelfiltrierung auf einer Säule von »Sephadex G-15« gereinigt. Die Hauptfraktion des AcOH-Eluates, die sich durch Trypsinbehandlung unter Freisetzung von 5-Amino-isophthalsäure-dimethylester spalten ließ, wurde im Vakuum bei 40°C zur Trockne eingeengt. Nach Trocknung des Rückstandes im Vakuumtrockenschrank bei 40°C über $P_2O_5$ erhielt man 1,93 g (82,6% der Theorie) der amorphen Verbindung 5f, die im DSC im LMS C einheitlich war. Elementaranalyse und Berechnung aus der Bruttoformel $C_{30}H_{49}N_7O_7Br_2$ ergaben die folgenden Werte: C = 46,84% (46,22%), H = 6,42% (6,34%), N = 12,16% (12,58%) und Br = 20,22% (20,50%).

Die Aminosäureanalyse ergab die zu erwartenden Aminosäuren im richtigen Verhältnis:

D-Val: 1,00 — Arg: 0,98 — CHA: 1,02.

Beispiel 6

2 HBr · H-D-CHG-Phe-Lys-DPA

6a. BOC-Lys(ε-Cbo)-DPA

In einem Dreihalsrundkolben von 1000 ml Inhalt wurden 38,05 g (0,1 Mol) getrocknetes BOC-Lys(ε-Cbo)-OH in einem Gemisch von frisch destilliertem wasserfreiem DMF und 300 ml absolutem THF bei 20°C gelöst. Der auf −10°C gekühlten Lösung wurde unter Rühren und Feuchtigkeitsausschluß eine Lösung von 10,2 g (0,1 Mol) $Et_3N$ in 75 ml THF zugesetzt. Dann wurde innerhalb 20 Minuten eine Lösung von 13,65 g (0,1 Mol) Chlorameisensäure-isobutylester in 50 ml THF zugetropft, wobei man die Reaktionstemperatur nie über −5°C steigen ließ. Nach einer Reaktionszeit

von etwa 10 Minuten bei −10°C bis −5°C wurde eine Lösung von 20,92 g (0,1 Mol) 5-Amino-isophthalsäure-dimethylester in 75 ml DMF innerhalb 30 Minuten zugetropft. Das Reaktionsgemisch wurde gemäß Beispiel 4a aufgearbeitet. Der erhaltene Rückstand wurde in 500 ml MeOH gelöst und durch Gelfiltrierung auf einer Säule von »Sephadex LH-20« gereinigt. Das MeOH-Eluat enthielt neben dem erünschten Produkt BOC-Lys(ε-Cbo)-DPA drei weitere Produkte, nämlich das Nebenprodukt N-[1,3-Dimethoxycarbonyl-phenyl-(5)]-karbaminsäure-isobutylester und die beiden Ausgangsprodukte BOC-Lys(ε-Cbo)-OH bzw. 5-Amino-isophthalsäure-dimethylester in drei verschiedenen Fraktionen. Die Fraktion, die das Produkt BOC-Lys(ε-Cbo)-DPA enthielt, wurde im Vakuum bei 30°C zur Trockne eingeengt. Nach Trocknung des Rückstandes im Vakuumtrockenschrank bei 50°C über $P_2O_5$ erhielt man 27,4 g (47,9% der Theorie) der kristallinen Verbindung 6a, die im DSC in den LMS A und B einheitlich war. Elementaranalyse und Berechnung aus der Bruttoformel $C_{29}H_{37}N_3O_9$ ergaben die folgenden Werte: C = 60,58% (60,93%), H = 6,53% (6,52%) und N = 7,48% (7,35%).

6b. $CF_3COOH$ · H-Lys(ε-Cbo)-DPA

22,87 g (40 mMol) der Verbindung 4a wurden gemäß Beispiel 2b mit 70 ml Trifluoressigsäure deblockiert. Das nach Aufarbeitung erhaltene Rohprodukt wurde in 250 ml MeOH gelöst und durch Gelfiltrierung auf einer Säule von »Sephadex LH-20« gereinigt. Die Hauptfraktion des MeOH-Eluates, die sich im DSC im LMS C einheitlich verhielt, wurde im Vakuum bei 30°C zur Trockne eingeengt. Nach Trocknung des Rückstandes im Vakuumtrockenschrank bei 40°C über $P_2O_5$ erhielt man 20,7 g (88,4% der Theorie) der amorphen Verbindung 6b. Elementaranalyse und Berechnung aus der Bruttoformel $C_{26}H_{30}N_3O_9F_3$ ergaben die folgenden Werte: C = 52,77% (53,33%), H = 5,25% (5,16%) und N = 7,02% (7,18%).

6c. BOC-Phe-Lys(ε-Cbo)-DPA

5,86 g (10 mMol) der Verbindung 6b wurden gemäß Beispiel 2c mit 4,25 g (11 mMol) BOC-Phe-OpNP umgesetzt. Das nach Aufarbeitung erhaltene Rohprodukt wurde in 100 ml MeOH gelöst und durch Gelfiltrierung auf einer Säule von »Sephadex LH-20« gereinigt. Die erste Hauptfraktion des MeOH-Eluates, die sich im DSC in den LMS A und B einheitlich verhielt, wurde im Vakuum bei 30°C zur Trockne eingeengt. Nach Trocknung des Rückstandes im Vakuumtrockenschrank bei 50°C erhielt man 5,87 g (81,7% der Theorie) der kristallinen Verbindung 6c. Elementaranalyse und Berechnung aus der Bruttoformel $C_{38}H_{46}N_4O_{10}$ ergaben die folgenden Werte: C = 63,82% (63,50%), H = 6,49% (6,45%) und N = 7,64% (7,80%).

6d. $CF_3COOH$ · Phe-Lys(ε-Cbo)-DPA

3,59 g (5 mMol) der Verbindung 6c wurden gemäß Beispiel 2d mit 15 ml Trifluoressigsäure deblockiert. Das nach Aufarbeitung erhaltene Rohprodukt wurde in 60 ml MeOH gelöst und durch Gelfiltrierung auf einer Säule von »Sephadex LH-20« gereinigt. Die Hauptfraktion des MeOH-Eluates, die sich im DSC im LMS C einheitlich verhielt, wurde im Vakuum bei 30°C zur Trockne eingeengt. Nach Trocknung des Rückstandes im Vakuumtrockenschrank bei 40°C über $P_2O_5$ erhielt man 3,40 g (92,8% der Theorie) der amorphen Verbindung 6d. Elementaranalyse und Berechnung aus der Bruttoformel $C_{35}H_{39}N_4O_{10}F_3$ ergaben die folgenden Werte: C = 57,11% (57,37%), H = 5,40% (5,36%) und N = 7,79% (7,65%).

6e. Cbo-D-CHG-Phe-Lys(ε-Cbo)-DPA

2,20 g (3 mMol) der Verbindung 6d wurden gemäß Beispiel 2e mit 1,36 g (3,3 mMol) Cbo-D-CHG-OpNP umgesetzt. Das nach Aufarbeitung erhaltene Rohprodukt wurde in 60 ml MeOH gelöst und durch Gelfiltrierung auf einer Säule von »Sephadex LH-20« gereinigt. Die erste Hauptfraktion des MeOH-Eluates, die sich im DSC in den LMS A und B einheitlich verhielt, wurde im Vakuum bei 30°C zur Trockne eingeengt. Nach Trocknung des Rückstandes im Vakuumtrockenschrank bei 60°C über $P_2O_5$ erhielt man 2,07 g (77,4% der Theorie) der teilweise kristallinen Verbindung 6e. Elementaranalyse und Berechnung aus der Bruttoformel $C_{49}H_{57}N_5O_{11}$ ergaben die folgenden Werte: C = 66,18% (65,98%), H = 6,52% (6,44%) und N = 7,59% (7,85%).

6f. 2 HBr · H-D-CHG-Phe-Lys-DPA

892 mg (1 mMol) der Verbindung 6e wurden gemäß Beispiel 2f mit 6 ml 2N HBr in Eisessig

deblockiert. Das nach Aufarbeitung erhaltene Rohprodukt wurde in 20 ml MeOH gelöst und auf einer Säule von »Sephadex LH-20« vorgereinigt. Die Fraktion des MeOH-Eluates, die sich durch Trypsinbehandlung unter Freisetzung von 5-Amino-isophthalsäure-dimethylester spalten ließ, wurde bei 30°C zur Trockne eingeengt. Zur weiteren Reinigung wurde das vorgereinigte Produkt in 30 ml 50%iger AcOH gelöst und durch Gelfiltrierung auf einer Säule von »Sephadex G-15« gereinigt. Die Hauptfraktion des AcOH-Eluates, die sich durch Trypsinbehandlung unter Freisetzung von 5-Amino-isophthalsäure-dimethylester spalten ließ, wurde im Vakuum bei 40°C zur Trockne eingeengt. Nach Trocknung des Rückstandes im Vakuumtrockenschrank bei 40°C über $P_2O_5$ erhielt man 602 mg (76,6% der Theorie) der amorphen Verbindung 6f, die im DSC im LMS C einheitlich war. Elementaranalyse und Berechnung aus der Bruttoformel $C_{33}H_{47}N_5O_7Br_2$ ergaben die folgenden Werte: C = 49,79% (50,45%), H = 6,10% (6,03%), N = 9,09% (8,92%) und Br = 19,87% (20,34%).

Die Aminosäureanalyse ergab die zu erwartenden Aminosäuren im richtigen Verhältnis:

Phe: 1,00 — Lys: 1,01 — D-CHG: 0,97.

Beispiel 7

2 HBr · H-D-Val-CHA-Arg-2-NA

7b. 2 HBr · H-Arg-2-NA

9,40 g (20 mMol) käufliches Cbo-Arg-2-NA · HCl wurden gemäß Beispiel 1b mit einer Lösung von 80 ml 2N HBr in Eisessig deblockiert. Das nach Aufarbeitung erhaltene Produkt wurde in 150 ml MeOH gelöst und auf einer Säule von »Sephadex LH-20« gereinigt. Diejenige Fraktion des MeOH-Eluates, die sich durch Trypsinbehandlung unter Freisetzung von 2-Naphthylamin spalten ließ, wurde im Vakuum bei 30° C zur Trockne eingeengt. Nach Trocknung des Rückstandes im Vakuumtrockenschrank bei 40° C über $P_2O_5$ erhielt man 8,60 g (93,2% der Theorie) der amrophen Verbindung 7b, die im DSC im LMS C einheitlich war. Elementaranalyse und Berechnung aus der Bruttoformel $C_{16}H_{23}N_5OBr_2$ ergaben die folgenden Werte: C = 42,08% (41,67%), H = 5,12% (5,03%), N = 14,68% (15,19%) und Br = 33,96% (34,65%).

7c. Cbo-CHA-Arg-2-NA · HBr

4,6 g (10 mMol) der Verbindung 7b wurden gemäß Beispiel 1c mit 4,69 g (11 mMol) Cbo-CHA-OpNP umgesetzt. Das nach Aufarbeitung erhaltene Rohprodukt wurde in 150 ml 50%iger AcOH gelöst und auf einer Säule von »Sephadex G-15« gereinigt. Diejenige Fraktion des AcOH-Eluates, die sich durch Trypsinbehandlung unter Freisetzung von 2-Naphthylamin spalten ließ, wurde im Vakuum bei 40°C zur Trockne eingeengt. Nach Trocknung des Rückstandes im Vakuumtrockenschrank bei 60°C über $P_2O_5$ erhielt man 5,31 g (79,5% der Theorie) der amorphen Verbindung 7c, die im DSC im LMS C einheitlich war. Elementaranalyse und Berechnung aus der Bruttoformel $C_{33}H_{43}N_6O_4Br$ ergaben die folgenden Werte: C = 59,18% (59,37%), H = 6,58% (6,49), N = 12,87% (12,59%) und Br = 11,55% (11,97%).

7d. 2 HBr · H-CHA-Arg-2-NA

4,67 g (7 mMol) der Verbindung 7c wurden gemäß Beispiel 1d mit 28 ml 2N HBr in Eisessig deblockiert. Das nach Aufarbeitung erhaltene Rohprodukt wurde in 100 ml MeOH gelöst und auf einer Säule von »Sephadex LH-20« gereinigt. Diejenige Fraktion des MeOH-Eluates, die sich durch Trypsinbehandlung unter Freisetzung von 2-Naphthylamin spalten ließ, wurde im Vakuum bei 30° C zur Trockne eingeengt. Nach Trocknung des Rückstandes im Vakuumtrockenschrank bei 40° C über $P_2O_5$ erhielt man 3,95 g (91,8% der Theorie) der amorphen Verbindung 7d, die im DSC im LMS C einheitlich war. Elementaranalyse und Berechnung aus der Bruttoformel $C_{25}H_{38}N_6O_2Br_2$ ergaben die folgenden Werte: C = 49,22% (48,87%), H = 6,30% (6,23%), N = 13,61% (13,68%) und Br = 25,84% (26,01%).

7e. Cbo-D-Val-CHA-Arg-2-NA · HBr

3,07 g (5 mMol) der Verbindung 7d wurden gemäß Beispiel 1e mit 2,05 g (5,5 mMol) Cbo-D-Val-OpNP umgesetzt. Das nach Aufarbeitung erhaltene Rohprodukt wurde in 100 ml 50%iger AcOH gelöst und auf einer Säule von »Sephadex G-15« gereinigt. Die erste Hauptfraktion des AcOH-Eluates, die sich durch Trypsinbehandlung unter Freisetzung von 2-Naphthylamin spalten ließ, wurde im Vakuum bei 40° C zur Trockne eingeengt und dann im Vakuumtrockenschrank bei 60° C über $P_2O_5$ getrocknet. Man erhielt 3,14 g (81,9% der Theorie) der amorphen Verbindung 7e, die im DSC im LMS C einheitlich war.

Elementaranalyse und Berechnung aus der Bruttoformel $C_{38}H_{52}N_7O_5Br$ ergaben die folgenden Werte: C = 58,92% (59,52%), H = 6,93% (6,84%), N = 13,02% (12,79%) und Br = 10,18% (10,42%).

7f. 2 HBr · H-D-Val-CHA · Arg-2-NA

1,53 g (2 mMol) der Verbindung 7e wurden gemäß Beispiel 1f mit 8 ml 2N HBr in Eisessig deblockiert. Das nach Aufarbeitung erhaltene Rohrpodukt wurde in 40 ml MeOH gelöst und auf einer Säule von »Sephadex LH-20« gereinigt. Die Hauptfraktion des MeOH-Eluates, die sich durch Trypsineinwirkung unter Bildung von 2-Maphthylamin spalten ließ, wurde im Vakuum bei 30° C zur Trockne eingeengt. Dieses Produkt wurde in 50 ml 50%iger AcOH gelöst und auf einer Säule von »Sephadex G-15« gereinigt. Die Hauptfraktion des AcOH-Eluates, die sich durch Trypsinbehandlung unter Entstehung von 2-Naphthylamin spalten ließ, wurde bei 40° C im Vakuum zur Trockne eingeengt. Nach Trocknung des Rückstandes im Vakuumtrockenschrank bei 40° C über $P_2O_5$ erhielt man 1,05 g (73,6% der Theorie) der amorphen Verbindung 7f, die im DSC im LMS C einheitlich war. Elementaranalyse und Berechnung aus der Brottoformel $C_{30}H_{47}N_7O_3Br_2$ ergaben die folgenden Werte: C = 50,16% (50,50%), H = 6,71% (6,64%), N = 14,00% (13,74%) und Br = 22,05% (22,40%).

Die Aminosäureanalyse ergab die zu erwartenden Aminosäuren im richtigen Verhältnis:

D-Val: 1,00 — Arg: 0,98 — CHA: 0,97.

## Beispiel 8

2 HBr · H-D-CHG-Phe-Lys-2-NA

8a. BOC · Lys(ε-Cbo)-2-NA

Gemäß Beispiel 4a wurden 38,05 g (0,1 Mol) BOC-Lys(ε-Cbo)-OH mit 14,32 g (0,1 Mol) 2-Naphthylamin zur Reaktion gebracht. Der nach Aufarbeitung erhaltene Rückstand wurde in 500 ml MeOH gelöst und auf einer Säule von »Sephadex LH-20« gereinigt. Aus dem MeOH-Eluat wurden neben dem erwünschten Produkt BOC-Lys(ε-Cbo)-2-NA drei weitere Produkte, nämlich das Nebenprodukt N-[1,3-Dimethoxy-phenyl-(5)]-karbaminsäure-isobutylester und die beiden Ausgangsprodukte BOC-Lys(ε-Cbo)-OH bzw. 2-Naphthylamin in drei verschiedenen Fraktionen erhalten. Die Fraktion, die BOC-Lys(ε-Cbo)-2-NA enthielt, wurde im Vakuum bei 30° C eingeengt. Nach Trocknung des Rückstandes im Vakuumtrockenschrank bei 50° C über $P_2O_5$ erhielt man 26,9 g (53,2% der Theorie) der kristallinen Verbindung 8a, die im DSC in den LMS A und B einheitlich war. Elementaranalyse und Berechnung aus der Brottuformel $C_{29}H_{35}N_3O_5$ ergaben die folgenden Werte: C = 68,23% (68,89%), H = 7,07% (6,98%) und N = 8,52% (8,31%).

8b. $CF_3COOH$ · H-Lys(ε-Cbo)-2-NA

20,22 g (40 mMol) der Verbindung 8a wurden gemäß Beispiel 2b mit 75 ml Trifluoressigsäure deblockiert. Das nach Aufarbeitung erhaltene Rohprodukt wurde in 250 ml MeOH gelöst und auf einer Säule von »Sephadex LH-20« gereinigt. Die Hauptfraktion des MeOH-Eluates, die sich im DSC im LMS C einheitlich verhielt, wurde im Vakuum bei 30° C zur Trockne eingeengt. Nach Trocknung des Rückstandes im Vakuumtrockenschrank bei 40° C über $P_2O_5$ erhielt man 18,29 g (88,0% der Theorie) der amorpen Verbindung 8b. Elementaranalyse und Berechnung aus der Bruttoformel $C_{26}H_{28}N_3O_5F_3$ ergaben die folgenden Werte: C = 59,70% (60,11%), H = 5,38% (5,43%) und N = 8,26% (8,09%).

8c. BOC-Phe-Lys(ε-Cbo)-2-NA

5,20 g (10 mMol) der Verbindung 8b wurden gemäß Beispiel 2c mit 4,25 g (11 mMol) BOC-Phe-OpNP umgesetzt. Das nach Aufarbeitung erhaltene Rohprodukt wurde in 100 ml MeOH gelöst und auf einer Säule von »Sephadex LH-20« gereinigt. Die erste Hauptfraktion des MeOH-Eluates, die sich im DSC in den LMS A und B einheitlich verhielt, wurde im Vakuum bei 30°C zur Trockne eingeengt. Der Rückstand wurde im Vakuumtrockenschrank bei 50° C über $P_2O_5$ getrocknet. Man erhielt 5,48 g (83,9% der Theorie) der teilweise kristallinen Verbindung 8c. Elementaranalyse und Berechnung aus der Bruttoformel $C_{38}H_{44}N_4O_6$ ergaben die folgenden Werte: C = 70,41% (69,92%), H = 6,74% (6,79%) und N = 8,69% (8,58%).

8d. $CF_3COOH$ · H-Phe-Lys(ε-Cbo)-2-NA

3,26 g (5 mMol) der Verbindung 8c wurden gemäß Beispiel 2d mit 17 ml Trifluoressigsäure deblockiert. Das nach Aufarbeitung erhaltene Rohprodukt wurde in 75 ml MeOH gelöst und auf einer Säule von »Sephadex LH-20« gereinigt. Die Hauptfraktion des MeOH-Eluates, die sich im DSC im LMS C einheitlich verhielt, wurde im Vakuum bei 30°C zur Trockne eingeengt. Nach Trocknung des Rückstandes im Vakuumtrockenschrank bei 40°C über $P_2O_5$ erhielt man 3,18 g (95,4% der Theorie) der amorphen Verbindung 8d. Elementraranalyse und Berechnung aus der Bruttoformel $C_{35}H_{37}N_4O_6F_3$ ergaben die folgenden Werte: C = 62,63% (63,05%), H = 5,66% (5,59%) und N = 8,19% (8,40%).

8e. Cbo-D-CHG-Phe-Lys(ε-Cbo)-2-NA

1,67 g (2,5 mMol) der Verbindung 8d wurden gemäß Beispiel 2e mit 1,14 g (2,76 mMol) Cbo-D-CHG-OpNP umgesetzt. Das nach Aufarbeitung erhaltene Rohprodukt wurde in 50 ml MeOH gelöst und auf einer Säule von »Sephadex LH-20« gereinigt. Die erste Hauptfraktion des MeOH-Eluates, die sich im DSC in den LMS A und B einheitlich verhielt, wurde im Vakuum bei 30°C zur Trockne eingeengt. Der Rückstand wurde im Vakuumtrockenschrank bei 60°C über $P_2O_5$ getrocknet. Man erhielt 1,58 g (76,5% der Theorie) der amorphen Verbindung 8e. Elementaranalyse und Berechnung aus der Bruttoformel $C_{49}H_{55}N_5O_7$ ergaben die folgenden Werte: C = 70,91% (71,25%), H = 6,66% (6,71%) und N = 8,69% (8,48%).

8f. 2 HBr · H-D-CHG-Phe-Lys-2-NA

1,24 g (1,5 mMol) der Verbindung 8e wurden gemäß Beispiel 2f mit 9 ml 2N HBr in Eisessig deblockiert. Das nach Aufarbeitung erhaltene Rohprodukt wurde in 25 ml MeOH gelöst und auf einer Säule von »Sephadex LH-20« vorgereinigt. Die Hauptfraktion des MeOH-Eluates, die sich durch Trypsineinwirkung unter Freisetzung von 2-Naphthylamin spalten ließ, wurde bei 30°C zur Trockne eingeengt. Das vorgereinigte Produkt wurde in 40 ml 50%iger AcOH gelöst und auf einer Säule von »Sephadex G-15« weitergereinigt. Die Hauptfraktion des AcOH-Eluates, die unter der Einwirkung von Trypsin 2-Naphthylamin bildete, wurde im Vakuum bei 40°C zur Trockne eingeengt. Nach Trocknung des Rückstandes im Vakuumtrockenschrank bei 40°C über $P_2O_5$ erhielt man 805 mg (74,6% der Theorie) der amorphen Verbindung 8f, die im DSC im LMS C einheitlich war. Elementaranalyse und Berechnung aus der Bruttoformel $C_{33}H_{45}N_5O_3Br_2$ ergaben die folgenden Werte: C = 54,73% (55,08%), H = 6,38% (6,30%), N = 10,05% (9,73%) und Br = 21,88% (22,21%).

Die Aminosäureanalyse ergab die zu erwartenden Aminosäuren im richtigen Verhältnis:

Phe: 1,00 — Lys: 0,99 — D-CHG: 0,98.

Beispiel 9

2 HBr · H-D-Val-CHA-Arg-4-MeO-2-NA

9b. 2 HBr · H-Arg-4-MeO-2-NA

10,0 g (20 mMol) käufliches Cbo-Arg-4-MeO-2-NA · HCl wurden gemäß Beispiel 1b mit 80 ml 2N HBr in Eisessig deblockiert. Das nach Aufarbeitung erhaltene Rohprodukt wurde in 150 ml MeOH gelöst und auf einer Säule von »Sephadex LH-20« gereinigt. Die Hauptfraktion des MeOH-Eluates, die sich durch Trypsinbehandlung unter Freisetzung von 4-Methoxy-2-naphthylamin spalten ließ, wurde im Vakuum bei 30°C zur Trockne eingeengt. Nach Trocknung des Rückstandes im Vakuumtrockenschrank bei 40°C über $P_2O_5$ erhielt man 8,98 g (91,4% der Theorie) der amorphen Verbindung 9b, die im DSC im LMS C einheitlich war. Elementaranalyse und Berechnung aus der Bruttoformel $C_{17}H_{25}N_5O_2Br_2$ ergaben die folgenden Werte: C = 41,22% (41,57%), H = 5,19% (5,13%), N = 14,40% (14,26%) und Br = 32,01% (32,53%).

9c. Cbo-CHA-Arg-4-MeO-2-NA · HBr

4,91 g (10 mMol) der Verbindung 9b wurden gemäß Beispiel 1c mit 4,69 g (11 mMol) Cbo-CHA-OpNP umgesetzt. Das nach Aufarbeitung erhaltene Rohprodukt wurde in 150 ml 50%iger AcOH gelöst und auf einer Säule von »Sephadex G-15« gereinigt. Die erste Hauptfraktion des AcOH-Eluates, die sich durch Trypsinbehandlung unter Freisetzung von 4-Methoxy-2-naphthylamin spalten ließ, wurde im Vakuum bei 40°C zur Trockne eingeengt. Nach Trocknung des Rückstandes im Vakuumtrockenschrank

bei 60° C über $P_2O_5$ erhielt man 5,36 g (76,8% der Theorie) der amorphen Verbindung 9c, die im DSC im LMS C einheitlich war. Elementaranalyse und Berechnung aus der Bruttoformel $C_{34}H_{45}N_6O_5Br$ ergaben die folgenden Werte: C = 58,85% (58,53%), H = 6,59% (6,50%), N = 11,91% (12,05%) und Br = 11,32% (11,45%).

9d. 2 HBr · H-CHA-Arg-4-MeO-2-NA

4,88 g (7 mMol) der Verbindung 9c wurden gemäß Beispiel 1d mit 28 ml 2N HBr in Eisessig deblockiert. Das nach Aufarbeitung erhaltene Rohprodukt wurde in 100 ml MeOH gelöst und auf einer Säule von »Sephadex LH-20« gereinigt. Die Hauptfraktion des MeOH-Eluates, die sich durch Trypsinbehandlung unter Bildung von 4-Methoxy-2-naphthylamin spalten ließ, wurde im Vakuum bei 30° C zur Trockne eingeengt. Nach Trocknung des Rückstandes im Vakuumtrockenschrank bei 40° C über $P_2O_5$ erhielt man 4,12 g (91,3% der Theorie) der amorphen Verbindung 9d, die im DSC im LMS C einheitlich war. Elementaranalyse und Berechnung aus der Bruttoformel $C_{26}H_{40}N_6O_3Br_2$ ergaben die folgenden Werte: C = 48,92% (48,46%), H = 6,36% (6,26%), N = 12,84% (13,04%) und Br = 24,33% (24,80%).

9e. Cbo-D-Val-CHA-Arg-4-MeO-2-NA · HBr

3,22 g (5 mMol) der Verbindung 9d wurden gemäß Beispiel 1e mit 2,05 g (5,5 mMol) Cbo-D-Val-OpNP umgesetzt. Das nach Aufarbeitung erhaltene Rohprodukt wurde in 125 ml 50%iger AcOH gelöst und auf einer Säule von »Sephadex G-15« gereinigt. Die erste Hauptfraktion des AcOH-Eluates, die sich durch Trypsinbehandlung unter Freisetzung von 4-Methoxy-2-naphthylamin spalten ließ, wurde im Vakuum bei 40° C zur Trockne eingeengt. Nach Trocknung des Rückstandes im Vakuumtrockenschrank bei 60° C über $P_2O_5$ erhielt man 3,15 g (79,1% der Theorie) der amorphen Verbindung 9e, die im DSC im LMS C einheitlich war. Elementaranalyse und Berechnung aus der Bruttoformel $C_{39}H_{54}N_7O_6Br$ ergaben die folgenden Werte: C = 58,35% (58,79%), H = 6,78% (6,83%), N = 12,68% (12,31%) und Br = 9,82% (10,03%).

9f. 2 HBr · H-D-Val-CHA-Arg-4-MeO-2-NA

1,59 g (2 mMol) der Verbindung 9e wurden gemäß Beispiel 1f mit 8 ml 2N HBr in Eisessig deblockiert. Das nach Aufarbeitung erhaltene Rohprodukt wurde in 40 ml MeOH gelöst und auf einer Säule von »Sephadex LH-20« vorgereinigt. Die Hauptfraktion des MeOH-Eluates, die sich durch Trypsinbehandlung unter Bildung von 4-Methoxy-2-naphthylamin spalten ließ, wurde bei 30° C zur Trockne eingeengt. Dieses vorgereinigte Produkt wurde in 60 ml 50%iger AcOH gelöst und auf einer Säule von »Sephadex G-15« gereinigt. Die Hauptfraktion des AcOH-Eluates, die sich durch Trypsinbehandlung unter Entstehung von 4-Methoxy-2-naphthylamin spalten ließ, wurde bei 40° C im Vakuum zur Trockne eingeengt. Nach Trocknung des Rückstandes im Vakuumtrockenschrank bei 40° C über $P_2O_5$ erhielt man 1,09 g (73,3% der Theorie) der amorphen Verbindung 9f, die im DSC im LMS C einheitlich war. Elementaranalyse und Berechnung aus der Bruttoformel $C_{31}H_{49}N_7O_4Br_2$ ergaben die folgenden Werte: C = 49,63% (50,07%), H = 6,70% (6,64%), N = 13,42% (13,19%) und Br = 21,22% (21,49%).

Die Aminosäureanalyse ergab die zu erwartenden Aminosäuren im richtigen Verhältnis:

D-Val: 1,00 — Arg: 1,01 — D-CHA: 0,98.

Beispiel 10

2 HBr · H-D-CHG-Phe-Lys-4-MeO-2-NA

10a. BOC-Lys(ε-Cbo)-4-MeO-2-NA

Gemäß Beispiel 4a wurden 9,51 g (25 mMol) BOC-Lys(ε-Cbo)-OH mit 4,33 g (25 mMol) 4-Methoxy-2-naphthylamin zur Reaktion gebracht. Der nach Aufarbeitung erhaltene Rückstand wurde in 175 ml MeOH gelöst und auf einer Säule von »Sephadex LH-20« gereinigt. Aus dem MeOH-Eluat wurden neben dem erwünschten Produkt BOC-Lys(ε-Cbo)-4-MeO-2-NA drei weitere Produkte, nämlich das Nebenprodukt N-(4-Methoxy-2-naphthyl)-karbaminsäure-isobutylester sowie die beiden Ausgangsprodukte BOC-Lys(ε-Cbo)-OH bzw. 4-Methoxy-2-naphthylamin in drei verschiedenen Fraktionen erhalten. Die das gewünschte Produkt enthaltende Fraktion wurde im Vakuum bei 30° C eingeengt. Nach Trocknung des Rückstandes im Vakuumtrockenschrank bei 50° C über $P_2O_5$ erhielt man 6,28 g (46,9% der Theorie) der teilweise kristallinen Verbindung 10a, die im DSC in den LMS A und

B einheitlich war. Elementaranalyse und Berechnung aus der Bruttoformel $C_{30}H_{37}N_3O_6$ ergaben die folgenden Werte: C = 66,83% (67,27%), H = 7,04% (6,96%) und N = 8,09% (7,85%).

10b. $CF_3COOH$ · H-Lys(ε-Cbo)-4-MeO-2-NA

5,36 g (10 mMol) der Verbindung 10a wurden gemäß Beispiel 2b mit 20 ml Trifluoressigsäure deblockiert. Das nach Aufarbeitung erhaltene Rohprodukt wurde in 75 ml MeOH gelöst und auf einer Säule von »Sephadex LH-20« gereinigt. Die Hauptfraktion des MeOH-Eluates, die sich im DSC im LMS C einheitlich verhielt, wurde im Vakuum bei 30°C zur Trockne eingeengt. Nach Trocknung des Rückstandes im Vakuumtrockenschrank bei 40°C über $P_2O_5$ erhielt man 5,10 g (92,8% der Theorie) der amorphen Verbindung 10b. Elementaranalyse und Berechnung aus der Bruttoformel $C_{27}H_{30}N_3O_6F$ ergaben die folgenden Werte: C = 58,66% (59,01%), H = 5,61% (5,50%) und N = 7,92% (7,65%).

10c. BOC-Phe-Lys(ε-Cbo)-4-MeO-2-NA

4,40 g (8 mMol) der Verbindung 10b wurden gemäß Beispiel 2c mit 3,40 g (8,8 mMol) BOC-Phe-OpNP umgesetzt. Das nach Aufarbeitung erhaltene Rohprodukt wurde in 75 ml MeOH gelöst und auf einer Säule von »Sephadex LH-20« gereinigt. Die erste Hauptfraktion des MeOH-Eluates, die sich im DSC in den LMS A und B einheitlich verhielt, wurde im Vakuum bei 30°C zur Trockne eingeengt. Nach Trocknung des Rückstandes im Vakuumtrockenschrank bei 50°C über $P_2O_5$ erhielt man 4,54 g (83,1% der Theorie) der amorphen Verbindung 10c. Elementaranalyse und Berechnung aus der Bruttoformel $C_{39}H_{46}N_4O_7$ ergaben die folgenden Werte: C = 68,24% (68,60%), H = 6,85% (6,79%) und N = 8,41% (8,21%).

10d. $CF_3COOH$ · H-Phe-Lys(ε-Cbo)-4-MeO-2-NA

3,41 g (5 mMol) der Verbindung 10c wurden gemäß Beispiel 2d mit 20 ml Trifluoressigsäure deblockiert. Das nach Aufarbeitung erhaltene Rohprodukt wurde in 80 ml MeOH gelöst und auf einer Säule von »Sephadex LH-20« gereinigt. Die Hauptfraktion des MeOH-Eluates, die sich im DSC im LMS C einheitlich verhielt, wurde im Vakuum bei 30°C zur Trockne eingeengt. Nach Trocknung des Rückstandes im Vakuumtrockenschrank bei 40°C über $P_2O_5$ erhielt man 3,29 g (94,4% der Theorie) der amorphen Verbindung 10d. Elementaranalyse und Berechnung aus der Bruttoformel $C_{36}H_{39}N_4O_7F_3$ ergaben die folgenden Werte: C = 61,82% (62,06%), H = 5,63% (5,64%) und N = 8,21% (8,04%).

10e. Cbo-D-CHG-Phe-Lys(ε-Cbo)-4-MeO-2-NA

1,74 g (2,5 mMol) der Verbindung 10d wurden gemäß Beispiel 2e mit 1,14 g (2,76 mMol) Cbo-D-CHG-OpNP umgesetzt. Das nach Aufarbeitung erhaltene Rohprodukt wurde in 60 ml MeOH gelöst und auf einer Säule von »Sephadex LH-20« gereinigt. Die erste Hauptfraktion des MeOH-Eluates, die sich im DSC in den LMS A und B einheitlich verhielt, wurde im Vakuum bei 30°C zur Trockne eingeengt. Nach Trocknung des Rückstandes im Vakuumtrockenschrank bei 60°C über $P_2O_5$ erhielt man 1,76 g (82,2% der Theorie) der amorphen Verbindung 10e. Elementaranalyse und Berechnung aus der Bruttoformel $C_{50}H_{57}N_5O_8$ ergaben die folgenden Werte: C = 69,75% (70,15%), H = 6,82% (6,71%) und N = 8,28% (8,18%).

10f. 2 HBr · H-D-CHG-Phe-Lys-4-MeO-2-NA

856 mg (1 mMol) der Verbindung 10e wurden gemäß Beispiel 2f mit 6 ml 2N HBr in Eisessig deblockiert. Das nach Aufarbeitung erhaltene Rohprodukt wurde in 20 ml AcOH gelöst und auf einer Säule von »Sephadex LH-20« vorgereinigt. Die Hauptfraktion des AcOH-Eluates, die sich durch Trypsinbehandlung unter Freisetzung von 4-Methoxy-2-naphthylamin spalten ließ, wurde bei 30°C zur Trockne eingeengt. Das vorgereinigte Produkt wurde in 30 ml 50%iger AcOH gelöst und auf einer Säule von »Sephadex G-15« gereinigt. Die Hauptfraktion des AcOH-Eluates, die unter der Einwirkung von Trypsin 4-Methoxy-2-naphthylamin bildete, wurde im Vakuum bei 40°C zur Trockne eingeengt. Nach Trocknung des Rückstandes im Vakuumtrockenschrank bei 40°C über $P_2O_5$ erhielt man 553 mg (73,8% der Theorie) der amorphen Verbindung 10f, die im DSC im LMS C einheitlich war. Elementaranalyse und Berechnung aus der Bruttoformel $C_{34}H_{47}N_5O_4Br_2$ ergaben die folgenden Werte: C = 54,09% (54,48%), H = 6,31% (6,32%), N = 9,52% (9,34%) und Br = 20,86% (21,32%).

Die Aminosäureanalyse ergab die zu erwartenden Aminosäuren im richtigen Verhältnis:

Phe: 1,00 — Lys: 1,02 — D-CHG: 0,99.

Nach den in den vorangehenden Beispielen beschriebenen Methoden wurde eine Reihe weiterer Tripeptidderivate hergestellt, welche in der Tabelle 1 zusammengestellt sind.

Die zur Herstellung der in Tabelle 1 aufgeführten Tripeptidderivate verwendeten Di- und Tripeptidzwischenprodukte sind in den Tabellen 2 und 3 zusammengestellt.

Tabelle 1

| Beispiel | Endprodukt | Ausgangsprodukte (mMol) | Methode (Beispiel) Ausbeute (%) | Elementaranalyse gef. % | ber. % | Aminosäureanalyse |
|---|---|---|---|---|---|---|
| 11 | 2 HBr · H-D-CHG-Leu-Arg-pNA<br>$C_{28}H_{44}N_8O_5Br_2$ | 11e (1 mMol)<br>2N HBr/AcOH | (1f)<br>88,2 | C 43,72<br>H 6,31<br>N 16,28<br>Br 22,23 | 44,08<br>6,26<br>15,82<br>22,56 | CHG : Leu : Arg<br>0,97 : 1,00 : 0,98 |
| 12 | 2 HBr · H-D-Val-CHA-Arg-pNA<br>$C_{26}H_{44}N_8O_5Br_2$ | 12e (1 mMol)<br>2N HBr/AcOH | (1f)<br>86,4 | C 43,60<br>H 6,41<br>N 16,15<br>Br 22,21 | 44,08<br>6,26<br>15,82<br>22,56 | Val : CHA : Arg<br>1,00 : 0,96 : 1,02 |
| 13 | 2 HBr · H-D-Ile-CHA-Arg-pNA<br>$C_{27}H_{46}N_8O_5Br_2$ | 13e (1,5 mMol)<br>2N HBr/AcOH | (1f)<br>88,9 | C 44,48<br>H 6,53<br>N 16,09<br>Br 21,85 | 44,88<br>6,42<br>15,51<br>22,12 | Ile : CHA : Arg<br>1,00 : 0,98 : 0,99 |
| 14 | 2 HBr · H-D-Val-CHA-Lys-pNA<br>$C_{28}H_{44}N_6O_5Br_2$ | 14e (0,8 mMol)<br>2N HBr/AcOH | (2f)<br>91,0 | C 45,30<br>H 6,58<br>N 12,69<br>Br 23,17 | 45,89<br>6,52<br>12,35<br>23,49 | Val : CHA : Lys<br>1,00 : 1,03 : 0,99 |
| 15 | 2 HBr · H-D-Ile-CHA-LYs-pNA<br>$C_{27}H_{46}N_6O_5Br_2$ | 15e (1,2 mMol)<br>2N HBr/AcOH | (2f)<br>90,3 | C 46,10<br>H 6,75<br>N 11,81<br>Br 22,75 | 46,69<br>6,68<br>12,10<br>23,01 | Ile : CHA : Lys<br>1,00 : 0,98 : 1,02 |
| 16 | 2 HBr · H-D-CHG-Phe-Arg-pNA<br>$C_{29}H_{42}N_8O_5Br_2$ | 16e (1 mMol)<br>2N HBr/AcOH | (1f)<br>85,6 | C 46,22<br>H 5,78<br>N 14,82<br>Br 21,33 | 46,91<br>5,70<br>15,09<br>21,52 | CHG : Phe : Arg<br>0,97 : 1,00 : 1,01 |
| 17 | 2 HBr · H-D-CHT-Phe-Arg-pNA<br>$C_{30}H_{44}N_8O_6Br_2$ | 17e (1,25 mMol)<br>2N HBr/AcOH | (1f)<br>79,4 | C 45,95<br>H 5,80<br>N 14,70<br>Br 20,55 | 46,64<br>5,74<br>14,51<br>20,69 | CHT : Phe : Arg<br>0,96 : 1,00 : 1,03 |

Fortsetzung

| Beispiel | Endprodukt | Ausgangsprodukte (mMol) | Methode (Beispiel) Ausbeute (%) | Elementaranalyse gef. % | ber. % | Aminosäureanalyse |
|---|---|---|---|---|---|---|
| 18 | 2 HBr · H-D-Ph'Gly-CHA-Arg-pNA<br>$C_{29}H_{42}N_8O_5Br_2$ | 18e (1,1 mMol)<br>2N HBr/AcOH | (1f)<br>82,6 | C 46,54<br>H 5,73<br>N 15,25<br>Br 21,28 | 46,91<br>5,70<br>15,09<br>21,52 | Ph'Gly : CHA : Arg<br>1,00 : 0,99 : 1,00 |
| 19 | 2 HBr · H-D-CHA-Pro-Arg-pNA<br>$C_{26}H_{42}N_8O_5Br_2$ | 19e (1 mMol)<br>2N HBr/AcOH | (1f)<br>86,4 | C 44,15<br>H 6,06<br>N 16,13<br>Br 22,44 | 44,20<br>5,99<br>15,86<br>22,62 | CHA : Pro : Arg<br>0,98 : 0,99 : 1,00 |
| 20 | 2 HBr · H-D-CHG-CHA-Lys-pNA<br>$C_{29}H_{48}N_6O_5Br_2$ | 20e (1 mMol)<br>2N HBr/AcOH | (2f)<br>92,4 | C 47,81<br>H 6,72<br>N 11,59<br>Br 21,83 | 48,34<br>6,71<br>11,66<br>22,18 | CHG : CHA : Lys<br>1,02 : 0,99 : 1,00 |
| 21 | 2 HBr · H-D-CHG-Leu-Lys-pNA<br>$C_{26}H_{44}N_6O_5Br_2$ | 21e (0,9 mMol)<br>2N HBr/AcOH | (2f)<br>92,5 | C 45,35<br>H 6,60<br>N 12,67<br>Br 23,17 | 45,89<br>6,52<br>12,35<br>23,49 | CHG : Leu : Lys<br>0,99 : 1,00 : 1,02 |
| 22 | 2 HBr · H-D-CHT-Pro-Arg-pNA<br>$C_{26}H_{42}N_8O_6Br_2$ | 23e (0,75 mMol)<br>2N HBr/AcOH | (1f)<br>82,4 | C 43,05<br>H 5,96<br>N 15,70<br>Br 21,88 | 43,22<br>5,86<br>15,51<br>22,12 | CHT : Pro : Arg<br>1,00 : 1,02 : 1,00 |
| 23 | 2 HBr · H-D-CHG-Pro-Arg-pNA<br>$C_{25}H_{40}N_8O_5Br_2$ | 23e (1,2 mMol)<br>2N HBr/AcOH | (1f)<br>84,8 | C 42,80<br>H 5,92<br>N 16,44<br>Br 22,70 | 43,36<br>5,82<br>16,18<br>23,08 | CHG : Pro : Arg<br>1,01 : 1,02 : 1,00 |
| 24 | 2 HBr · H-D-CHT-Pip-Arg-pNA<br>$C_{27}H_{44}N_8O_6Br_2$ | 24e (0,65 mMol)<br>2N HBr/AcOH | (1f)<br>82,6 | C 43,96<br>H 6,09<br>N 15,77<br>Br 21,53 | 44,03<br>6,02<br>15,22<br>21,70 | CHT : Pip : Arg<br>0,99 : 0,96 : 1,00 |

Fortsetzung

| Beispiel | Endprodukt | Ausgangsprodukte (mMol) | Methode (Beispiel) Ausbeute (%) | Elementaranalyse | gef. % | ber. % | Aminosäureanalyse |
|---|---|---|---|---|---|---|---|
| 25 | 2 AcOH · H-D-CHA-Pro-Lys-pNA<br>$C_{30}H_{48}N_6O_9$ | 25e (0,75 mMol)<br>2N HBr/AcOH | (2f)<br>87,5 | C<br>H<br>N | 56,09<br>7,65<br>13,48 | 56,59<br>7,60<br>13,20 | CHA : Pro : Lys<br>0,99 : 0,98 : 1,00 |
| 26 | 2 AcOH · H-D-Val-CHG-Lys-pNA<br>$C_{29}H_{48}N_6O_9$ | 26e (0,75 mMol)<br>2N HBr/AcOH | (2f)<br>88,8 | C<br>H<br>N | 56,02<br>7,82<br>13,59 | 55,75<br>7,74<br>13,45 | Val : CHG : Lys<br>1,00 : 0,98 : 1,01 |
| 27 | 2 AcOH · H-D-CHG-Pro-Lys-pNA<br>$C_{29}H_{46}N_6O_9$ | 27e (0,75 mMol)<br>2N HBr/AcOH | (2f)<br>85,4 | C<br>H<br>N | 55,49<br>7,55<br>13,78 | 55,93<br>7,45<br>13,50 | CHG : Pro : Lys<br>0,97 : 0,99 : 1,00 |
| 28 | 2 AcOH · H-D-CHT-Pro-Lys-pNA<br>$C_{30}H_{48}N_6O_{10}$ | 28e (0,75 mMol)<br>2N HBr/AcOH | (2f)<br>79,8 | C<br>H<br>N | 55,01<br>7,44<br>13,18 | 55,20<br>7,41<br>12,88 | CHT : Pro : Lys<br>0,96 : 0,99 : 1,00 |
| 29 | 2 AcOH · H-D-Ph'Gly-CHA-Lys-pNA<br>$C_{33}H_{48}N_6O_9$ | 29e (0,75 mMol)<br>2N HBr/AcOH | (2f)<br>75,5 | C<br>H<br>N | 59,25<br>7,18<br>12,66 | 58,91<br>7,19<br>12,49 | Ph'Gly : CHA : Lys<br>0,99 : 0,98 : 1,00 |
| 30 | 2 AcOH · H-D-Val-CHT-Lys-pNA<br>$C_{30}H_{50}N_6O_{10}$ | 30e (0,75 mMol)<br>2N HBr/AcOH | (2f)<br>84,4 | C<br>H<br>N | 54,90<br>7,78<br>13,05 | 55,03<br>7,70<br>12,84 | Val : CHT : Lys<br>1,00 : 0,98 : 1,01 |
| 31 | 2 AcOH · H-D-Ph'Gly-CHT-Lys-pNA<br>$C_{33}H_{48}N_6O_{10}$ | 31e (0,75 mMol)<br>2H HBr/AcOH | (2f)<br>73,8 | C<br>H<br>N | 57,18<br>7,09<br>12,40 | 57,54<br>7,02<br>12,20 | Ph'Gly : CHT : Lys<br>0,98 : 0,96 : 1,00 |
| 32 | 2 HBr · H-D-Leu-CHA-Arg-pNA<br>$C_{27}H_{46}N_8O_5Br_2$ | 32e (1 mMol)<br>2N HBr/AcOH | (1f)<br>85,2 | C<br>H<br>N<br>Br | 44,55<br>6,53<br>15,85<br>21,92 | 44,88<br>6,42<br>15,51<br>22,12 | Leu : CHA : Arg<br>1,00 : 0,98 : 0,99 |

Fortsetzung

| Beispiel | Endprodukt | Ausgangsprodukte (mMol) | Methode (Beispiel) Ausbeute (%) | Elementaranalyse gef. % | ber. % | Aminosäureanalyse |
|---|---|---|---|---|---|---|
| 33 | 2 HBr · H-D-Nleu-CHA-Arg-pNA<br>$C_{27}N_{46}N_8O_5Br_2$ | 33e (1 mMol)<br>2H HBr/AcOH | (1f)<br>86,0 | C 44,75<br>H 6,49<br>N 15,79<br>Br 21,82 | 44,88<br>6,42<br>15,51<br>22,12 | Nleu : CHA : Arg<br>1,00 : 0,97 : 0,99 |
| 34 | 2 HBr · H-D-Nval-CHA-Arg-pNA<br>$C_{26}H_{44}N_8O_5Br_2$ | 34e (1 mMol)<br>2N HBr/AcOH | (1f)<br>87,1 | C 44,39<br>H 6,28<br>N 16,09<br>Br 22,33 | 44,08<br>6,26<br>15,82<br>22,56 | Nval : CHA : Arg<br>1,00 : 0,98 : 0,98 |
| 35 | 2 HBr · H-D-Phe-CHA-Arg-pNA<br>$C_{30}H_{44}N_8O_5Br_2$ | 35e (1 mMol)<br>2N HBr/AcOH | (1f)<br>82,3 | C 47,47<br>H 5,92<br>N 15,08<br>Br 20,85 | 47,63<br>5,86<br>14,81<br>21,12 | Phe : CHA : Arg<br>1,00 : 0,98 : 1,01 |
| 36 | 2 HBr · H-D-Ala-CHA-Arg-pNA<br>$C_{24}H_{40}N_8O_5Br_2$ | 36e (1 mMol)<br>2N HBr/AcOH | (1f)<br>86,0 | C 41,97<br>H 5,92<br>N 16,59<br>Br 23,11 | 42,36<br>5,93<br>16,47<br>23,49 | Ala : CHA : Arg<br>1,00 : 0,97 : 0,99 |
| 37 | 2 HBr · H-D-But-CHA-Arg-pNA<br>$C_{25}H_{42}N_8O_5Br_2$ | 37e (1 mMol)<br>2N HBr/AcOH | (1f)<br>81,9 | C 43,18<br>H 6,16<br>N 16,27<br>Br 22,73 | 43,24<br>6,10<br>16,14<br>23,01 | But : CHA : Arg<br>1,00 : 0,98 : 1,01 |
| 38 | 2 HBr · H-D-CHG-Tyr-Arg-pNA<br>$C_{29}H_{42}N_8O_6Br_2$ | 38e (0,5 mMol)<br>2N HBr/AcOH | (1f)<br>77,8 | C 45,77<br>H 5,64<br>N 15,01<br>Br 20,84 | 45,92<br>5,58<br>14,77<br>21,07 | CHG : Tyr : Arg<br>0,97 : 1,00 : 0,99 |
| 39 | 2 HBr · H-D-CHA-Tyr-Arg-pNA<br>$C_{30}h_{46}N_8O_6Br_2$ | 39e (0,5 mMol)<br>2N HBr/AcOH | (1f)<br>72,1 | C 46,39<br>H 5,73<br>N 14,60<br>Br 20,50 | 46,64<br>5,74<br>14,51<br>20,69 | CHA : Tyr : Arg<br>0,98 : 1,00 : 1,00 |

Fortsetzung

| Beispiel | Endprodukt | Ausgangsprodukte (mMol) | Methode (Beispiel) Ausbeute (%) | Elementaranalyse gef. % | ber. % | Aminosäureanalyse |
|---|---|---|---|---|---|---|
| 40 | 2 HBr · H-D-Nval-Tyr-Arg-pNA<br>$C_{26}H_{38}N_8O_6Br_2$ | 40e (0,5 mMol)<br>2N HBr/AcOH | (1f)<br>70,5 | C 43,29<br>H 5,31<br>N 15,82<br>Br 22,07 | 43,47<br>5,33<br>15,60<br>22,24 | Nval : Tyr : Arg<br>1,02 : 1,00 : 0,98 |
| 41 | 2 HBr · H-D-CHT-Tyr-Arg-pNA<br>$C_{30}H_{44}N_8O_7Br_2$ | 41e (0,5 mMol)<br>2N HBr/AcOH | (1f)<br>69,9 | C 45,20<br>H 5,68<br>N 14,39<br>Br 20,05 | 45,69<br>5,62<br>14,21<br>20,27 | CHT : Tyr : Arg<br>0,96 : 1,00 : 0,99 |
| 42 | 2 HBr · H-D-Phe-Tyr-Arg-pNA<br>$C_{30}H_{38}N_8O_6Br_2$ | 42e (0,5 mMol)<br>2N HBr/AcOH | (1f)<br>73,0 | C 47,51<br>H 5,07<br>N 14,88<br>Br 20,46 | 47,01<br>5,00<br>14,62<br>20,85 | Phe : Tyr : Arg<br>1,00 : 1,01 : 0,98 |
| 43 | 2 HBr · H-D-Ph'Gly-Tyr-Arg-pNA<br>$C_{29}H_{36}N_8O_6Br_2$ | 43e (0,5 mMol)<br>2N HBr/AcOH | (1f)<br>67,8 | C 46,57<br>H 4,90<br>N 15,13<br>Br 20,98 | 46,30<br>4,82<br>14,89<br>21,24 | Ph'Gly : Tyr : Arg<br>0,97 : 1,00 : 0,99 |
| 44 | 2 HBr · H-D-CHG-Ala-Arg-pNA<br>$C_{23}H_{38}N_8O_5Br_2$ | 44e (1 mMol)<br>2N HBr/AcOH | (1f)<br>83,4 | C 41,66<br>H 5,74<br>N 17,09<br>Br 23,90 | 41,45<br>5,75<br>16,82<br>23,98 | CHG : Ala : Arg<br>0,96 : 1,00 : 0,99 |
| 45 | 2 HBr · H-D-CHA-Ala-Arg-pNA<br>$C_{24}H_{40}N_8O_5Br_2$ | 45e (1 mMol)<br>2N HBr/AcOH | (1f)<br>88,1 | C 42,54<br>H 5,98<br>N 16,60<br>Br 23,19 | 42,36<br>5,93<br>16,47<br>23,49 | CHA : Ala : Arg<br>0,98 : 1,00 : 1,00 |
| 46 | 2 HBr · H-D-CHT-Leu-Arg-pNA<br>$C_{27}H_{46}N_8O_6Br_2$ | 46e (1 mMol)<br>2N HBr/AcOH | (1f)<br>80,7 | C 43,80<br>H 6,33<br>N 15,42<br>Br 21,37 | 43,91<br>6,28<br>15,17<br>21,64 | CHT : Leu : Arg<br>0,96 : 1,00 : 0,99 |

Fortsetzung

| Beispiel | Endprodukt | Ausgangsprodukte (mMol) | Methode (Beispiel) Ausbeute (%) | Elementaranalyse gef. % | ber. % | Aminosäureanalyse |
|---|---|---|---|---|---|---|
| 47 | 2 HBr · H-D-CHA-Leu-Arg-pNA<br>$C_{27}H_{46}N_8O_5Br_2$ | 47e (1 mMol)<br>2N HBr/AcOH | (1f)<br>84,5 | C 44,38<br>H 6,41<br>N 15,75<br>Br 21,85 | 44,88<br>6,42<br>15,51<br>22,12 | CHA : Leu : Arg<br>0,97 : 1,00 : 1,01 |
| 48 | 2 HBr · H-D-Leu-Ph'Gly-Arg-pNA<br>$C_{26}H_{38}N_8O_5Br_2$ | 48e (0,5 mMol)<br>2N HBr/AcOH | (1f)<br>82,3 | C 44,18<br>H 5,50<br>N 16,18<br>Br 22,48 | 44,46<br>5,45<br>15,95<br>22,75 | Leu : Ph'Gly : Arg<br>1,00 : 0,97 : 0,98 |
| 49 | 2 HBr · H-D-Nval-Ph'Gly-Arg-pNA<br>$C_{25}H_{36}N_8O_5Br_2$ | 49e (0,5 mMol)<br>2N HBr/AcOH | (1f)<br>79,8 | C 43,24<br>H 5,33<br>N 16,39<br>Br 22,87 | 43,62<br>5,27<br>16,28<br>23,21 | Nval : Ph'Gly : Arg<br>1,00 : 0,98 : 1,01 |
| 50 | 2 HBr · H-D-Ala-Ph'Gly-Arg-pNA<br>$C_{23}H_{32}N_8O_5Br_2$ | 50e (0,5 mMol)<br>2N HBr/AcOH | (1f)<br>82,1 | C 41,46<br>H 4,93<br>N 17,19<br>Br 23,92 | 41,83<br>4,88<br>16,97<br>24,20 | Ala : Ph'Gly : Arg<br>1,00 : 0,98 : 0,99 |
| 51 | 2 HBr · H-D-CHA-Ph'Gly-Arg-pNA<br>$C_{29}H_{42}N_8O_5Br_2$ | 51e (0,5 mMol)<br>2N HBr/AcOH | (1f)<br>77,6 | C 46,75<br>H 5,73<br>N 15,40<br>Br 21,22 | 46,91<br>5,70<br>15,09<br>21,52 | CHA : Ph'Gly : Arg<br>0,97 : 0,98 : 1,00 |
| 52 | 2 HBr · H-D-CHT-Ph'Gly-Arg-pNA<br>$C_{29}H_{42}N_8O_6Br_2$ | 52e (0,5 mMol)<br>2N HBr/AcOH | (1f)<br>76,4 | C 45,64<br>H 5,61<br>N 15,00<br>Br 20,69 | 45,92<br>5,58<br>14,77<br>21,07 | CHT : Ph'Gly : Arg<br>0,96 : 0,99 : 1,00 |
| 53 | 2 HBr · H-D-CHG-Ph'Gly-Arg-pNA<br>$C_{28}H_{40}N_8O_5Br_2$ | 53e (0,5 mMol)<br>2N HBr/AcOH | (1f)<br>75,9 | C 46,02<br>H 5,59<br>N 15,67<br>Br 21,48 | 46,16<br>5,53<br>15,38<br>21,94 | CHG : Ph'Gly : Arg<br>0,98 : 0,98 : 1,00 |

Fortsetzung

| Beispiel | Endprodukt | Ausgangsprodukte (mMol) | Methode (Beispiel) Ausbeute (%) | Elementaranalyse gef. % | ber. % | Aminosäureanalyse |
|---|---|---|---|---|---|---|
| 54 | 2 HBr · H-D-CHG-CHG-Arg-pNA<br>$C_{28}H_{46}N_8O_5Br_2$ | 54e (1 mMol)<br>2N HBr/AcOH | (1f)<br>84,6 | C 45,65<br>H 6,38<br>N 15,44<br>Br 21,52 | 45,78<br>6,31<br>15,26<br>21,76 | CHG : Arg<br>1,93 : 1,00 |
| 55 | 2 HBr · H-D-Phe-CHG-Arg-pNA<br>$C_{29}H_{42}N_8O_5Br_2$ | 55e (1 mMol)<br>2N HBr/AcOH | (1f)<br>88,4 | C 46,70<br>H 5,68<br>N 15,27<br>Br 21,33 | 46,91<br>5,70<br>15,09<br>21,52 | Phe : CHG : Arg<br>1,00 : 0,98 : 1,01 |
| 56 | 2 HBr · H-D-Ph'Gly-CHG-Arg-pNA<br>$C_{28}H_{40}N_8O_5Br_2$ | 56e (1 mMol)<br>2N HBr/AcOH | (1f)<br>82,7 | C 46,18<br>H 5,60<br>N 15,62<br>Br 21,77 | 46,16<br>5,53<br>15,38<br>21,94 | Ph'Gly : CHG : Arg<br>0,98 : 0,98 : 1,00 |
| 57 | 2 HBr · H-D-Ph'Gly-Phe-Arg-pNA<br>$C_{29}H_{36}N_8O_5Br_2$ | 57e (0,5 mMol)<br>2N HBr/AcOH | (1f)<br>80,0 | C 47,08<br>H 5,00<br>N 15,38<br>Br 21,61 | 47,29<br>4,93<br>15,22<br>21,70 | Ph'Gly : Phe : Arg<br>0,97 : 1,00 : 0,99 |
| 58 | 2 HBr · H-D-CHA-Phe-Arg-pNA<br>$C_{30}H_{44}N_8O_5Br_2$ | 58e (0,5 mMol)<br>2N HBr/AcOH | (1f)<br>82,5 | C 47,32<br>H 5,84<br>N 14,90<br>Br 20,88 | 47,63<br>5,86<br>14,81<br>21,12 | CHA : Phe : Arg<br>0,96 : 1,00 : 0,99 |
| 59 | 2 HBr · H-D-CHG-Pip-Arg-pNA<br>$C_{26}H_{42}N_8O_5Br_2$ | 59e (0,5 mMol)<br>2N HBr/AcOH | (1f)<br>77,7 | C 43,87<br>H 6,04<br>N 16,18<br>Br 22,40 | 44,20<br>5,99<br>15,86<br>22,62 | CHG : Pip : Arg<br>0,98 : 0,96 : 1,00 |

Fortsetzung

| Beispiel | Endprodukt | Ausgangsprodukte (mMol) | Methode (Beispiel) Ausbeute (%) | Elementaranalyse | gef. % | ber. % | Aminosäureanalyse |
|---|---|---|---|---|---|---|---|
| 60 | 2 HBr · H-D-CHA-Pip-Arg-pNA<br>$C_{27}H_{44}N_8O_5Br_2$ | 60e (0,5 mMol)<br>2N HBr/AcOH | (1f)<br>76,9 | C<br>H<br>N<br>Br | 44,76<br>6,18<br>15,73<br>22,01 | 45,01<br>6,16<br>15,55<br>22,18 | CHA : Pip : Arg<br>0,98 : 0,95 : 1,00 |
| 61 | 2 HBr · H-D-CHG-Nleu-Arg-pNA<br>$C_{26}H_{44}N_8O_5Br_2$ | 61e (1 mMol)<br>2N HBr/AcOH | (1f)<br>85,8 | C<br>H<br>N<br>Br | 43,68<br>6,30<br>16,02<br>22,33 | 44,08<br>6,26<br>15,82<br>22,56 | CHG : Nleu : Arg<br>0,97 : 1,00 : 0,99 |
| 62 | 2 HBr · H-D-CHA-Nleu-Arg-pNA<br>$C_{27}H_{46}N_8O_5Br_2$ | 62e (1 mMol)<br>2N HBr/AcOH | (1f)<br>87,4 | C<br>H<br>N<br>Br | 44,59<br>6,45<br>15,62<br>21,87 | 44,88<br>6,42<br>15,51<br>22,12 | CHA : Nleu : Arg<br>0,98 : 1,00 : 0,98 |
| 63 | 2 HBr · H-D-CHA-Nval-Arg-pNA<br>$C_{26}H_{44}N_8O_5Br_2$ | 63e (0,5 mMol)<br>2N HBr/AcOH | (1f)<br>90,2 | C<br>H<br>N<br>Br | 43,90<br>6,32<br>16,18<br>22,39 | 44,08<br>6,26<br>15,82<br>22,56 | CHA : Nval : Arg<br>0,97 : 1,00 : 0,99 |
| 64 | 2 HBr · H-D-CHG-Nval-Arg-pNA<br>$C_{25}H_{42}N_8O_5Br_2$ | 64e (0,5 mMol)<br>2N HBr/AcOH | (1f)<br>88,5 | C<br>H<br>N<br>Br | 43,20<br>6,15<br>16,36<br>22,78 | 43,24<br>6,10<br>16,14<br>23,01 | CHG : Nval : Arg<br>0,96 : 1,00 : 1,01 |
| 65 | 2 HBr · H-D-Nval-CHA-Lys-pNA<br>$C_{26}H_{44}N_6O_5Br_2$ | 65e (1 mMol)<br>2N HBr/AcOH | (2f)<br>76,4 | C<br>H<br>N<br>Br | 45,73<br>6,61<br>12,48<br>23,18 | 45,89<br>6,52<br>12,35<br>23,49 | Nval : CHA : Lys<br>1,00 : 0,98 : 0,99 |
| 66 | 2 HBr · H-D-But-CHA-Lys-pNA<br>$C_{25}H_{42}N_6O_5Br_2$ | 66e (1 mMol)<br>2N HBr/AcOH | (2f)<br>74,6 | C<br>H<br>N<br>Br | 44,75<br>6,39<br>12,67<br>23,68 | 45,05<br>6,35<br>12,61<br>23,98 | But : CHA : Lys<br>1,02 : 0,98 : 1,00 |

Fortsetzung

| Beispiel | Endprodukt | Ausgangsprodukte (mMol) | Methode (Beispiel) Ausbeute (%) | Elementaranalyse gef. % | | Elementaranalyse ber. % | Aminosäureanalyse |
|---|---|---|---|---|---|---|---|
| 67 | 2 HBr · H-D-Leu-CHA-Lys-pNA<br>$C_{27}H_{46}N_6O_5Br_2$ | 67e (1 mMol)<br>2N HBr/AcOH | (2f)<br>78,0 | C<br>H<br>N<br>Br | 46,39<br>6,63<br>12,27<br>22,81 | 46,69<br>6,68<br>12,10<br>23,01 | Leu : CHA : Lys<br>1,00 : 0,97 : 1,01 |
| 68 | 2 HBr · H-D-Nleu-CHA-Lys-pNA<br>$C_{27}H_{46}N_6O_5Br_2$ | 68e (1 mMol)<br>2N HBr/AcOH | (2f)<br>77,6 | C<br>H<br>N<br>Br | 46,88<br>6,72<br>12,33<br>22,75 | 46,69<br>6,68<br>12,10<br>23,01 | Nleu : CHA : Lys<br>0,99 : 0,97 : 1,00 |
| 69 | 2 HBr · H-D-CHG-But-Arg-pNA<br>$C_{24}H_{40}N_8O_5Br_2$ | 69e (1 mMol)<br>2N HBr/AcOH | (1f)<br>84,7 | C<br>H<br>N<br>Br | 42,18<br>6,00<br>16,52<br>23,18 | 42,36<br>5,93<br>16,47<br>23,49 | CHG : But : Arg<br>0,97 : 0,99 : 1,00 |
| 70 | 2 HBr · H-D-CHA-But-Arg-pNA<br>$C_{25}H_{42}N_8O_5Br_2$ | 70e (1 mMol)<br>2N HBr/AcOH | (1f)<br>88,5 | C<br>H<br>N<br>Br | 42,96<br>6,12<br>16,25<br>22,69 | 43,24<br>6,10<br>16,14<br>23,01 | CHA : But : Arg<br>0,98 : 0,98 : 1,00 |
| 71 | 2 HBr · H-D-CHT-But-Arg-pNA<br>$C_{25}H_{42}N_8O_6Br_2$ | 71e (1 mMol)<br>2N HBr/AcOH | (1f)<br>82,3 | C<br>H<br>N<br>Br | 41,89<br>6,01<br>15,96<br>22,15 | 42,26<br>5,96<br>15,77<br>22,49 | CHT : But : Arg<br>0,96 : 0,99 : 1,00 |

Tabelle 2

| Beispiel | Dipeptid-Zwischenprodukt | Ausgangsprodukt (mMol) | Methode (Beispiel) Ausbeute (%) | Elementaranalyse | gef. % | ber. % |
|---|---|---|---|---|---|---|
| 11c | Cbo-Leu-Arg-pNA · HBr<br>$C_{26}H_{36}N_7O_6Br$ | 1b (5 mMol)<br>Cbo-LeuOpNP<br>(5,5 mMol) | (1c)<br>82,4 | C<br>H<br>N<br>Br | 50,75<br>5,95<br>16,10<br>12,69 | 50,16<br>5,83<br>15,75<br>12,84 |
| 11d | 2 HBr · H · Leu-Arg-pNA<br>$C_{18}H_{31}N_7O_4Br_2$ | 11c (3 mMol)<br>2N HBr/AcOH | (1d)<br>90,3 | C<br>H<br>N<br>Br | 38,42<br>5,55<br>17,84<br>27,67 | 37,98<br>5,49<br>17,23<br>28,07 |
| 14c | BOC-CHA-Lys(ε-Cbo)-pNA<br>$C_{34}H_{47}N_5O_8$ | 2b (5 mMol)<br>BOC-CHA-OpNP<br>(5,5 mMol) | (2c)<br>84,2 | C<br>H<br>N | 63,05<br>7,35<br>10,98 | 62,46<br>7,25<br>10,71 |
| 14d | $CF_3COOH$ · H-CHA-Lys(ε-Cbo)-pNA<br>$C_{31}H_{40}N_5O_8F_3$ | 14c (3 mMol)<br>6ml $CF_3COOH$ | (2d)<br>91,6 | C<br>H<br>N | 56,08<br>6,15<br>11,01 | 55,77<br>6,04<br>10,49 |
| 16c | Cbo-Phe-Arg-pNA · HBr<br>$C_{29}H_{34}N_7O_6Br$ | 1b (5 mMol)<br>Cbo-Phe-OpNP<br>(5,5 mMol) | (1c)<br>84,8 | C<br>H<br>N<br>Br | 53,45<br>5,30<br>15,15<br>11,95 | 53,05<br>5,22<br>14,94<br>12,17 |
| 16d | 2 HBr · H-Phe-Arg-pNA<br>$C_{21}H_{29}N_7O_4Br_2$ | 16c (3 mMol)<br>2N HBr/AcOH | (1d)<br>93,5 | C<br>H<br>N<br>Br | 41,38<br>4,78<br>16,50<br>26,15 | 41,81<br>4,85<br>16,25<br>26,49 |
| 19e | Cbo-Pro-Arg-pNA · HBr<br>$C_{25}H_{32}N_7O_6Br$ | 1b (5 mMol)<br>Cbo-Pro-OpNP<br>(5,5 mMol) | (1c)<br>88,1 | C<br>H<br>N<br>Br | 49,95<br>5,40<br>16,19<br>12,82 | 49,51<br>5,32<br>16,17<br>13,17 |
| 19d | 2 HBr · H-Pro-Arg-pNA<br>$C_{17}H_{27}N_7O_4Br_2$ | 19c (3 mMol)<br>2N HBr/AcOH | (1d)<br>92,0 | C<br>H<br>N<br>Br | 37,41<br>5,03<br>18,15<br>28,50 | 36,91<br>4,92<br>17,72<br>28,88 |
| 21c | BOC-Leu-Lys(ε-Cbo)-pNA<br>$C_{31}H_{43}N_5O_8$ | 2b (5 mMol)<br>BOC-Leu-OpNP<br>(5,5 mMol) | (2c)<br>87,3 | C<br>H<br>N | 61,10<br>7,15<br>11,88 | 60,67<br>7,06<br>11,41 |
| 21d | $CF_3COOH$ · H-Leu-Lys(ε-Cbo)-pNA<br>$C_{28}H_{36}N_5O_8F_3$ | 21c (3 mMol)<br>6ml $CF_3COOH$ | (2d)<br>90,9 | C<br>H<br>N | 53,40<br>5,85<br>11,50 | 53,58<br>5,78<br>11,16 |
| 54c | Cbo-CHG-Arg-pNA · HBr<br>$C_{28}H_{38}N_7O_6Br$ | 1b (5 mMol)<br>Cbo-CHG-OpNP<br>(5,5 mMol) | (1c)<br>79,6 | C<br>H<br>N<br>Br | 52,20<br>6,09<br>15,55<br>12,05 | 51,85<br>5,91<br>15,12<br>12,32 |
| 54d | 2 HBr · H-CHG-Arg-pNA<br>$C_{20}H_{33}N_7O_4Br_2$ | 54c (3 mMol)<br>2N HBr/AcOH | (1d)<br>88,4 | C<br>H<br>N<br>Br | 40,95<br>5,53<br>17,06<br>26,58 | 40,35<br>5,59<br>16,47<br>26,84 |

Fortsetzung

| Beispiel | Dipeptid-Zwischenprodukt | Ausgangsprodukt (mMol) | Methode (Beispiel) Ausbeute (%) | Elementaranalyse | gef. % | ber. % |
|---|---|---|---|---|---|---|
| 24c | Cbo-Pip-Arg-pNA · HBr<br>$C_{26}H_{34}N_7O_6Br$ | 1b (5 mMol)<br>Cbo-Pip-OpNP<br>(5,5 mMol) | (1c)<br>82,3 | C<br>H<br>N<br>Br | 50,80<br>5,50<br>16,19<br>12,66 | 50,33<br>5,52<br>15,80<br>12,88 |
| 24d | 2 HBr · H-Pip-Arg-pNA<br>$C_{18}H_{29}N_7O_4Br_2$ | 24c (2,5 mMol)<br>2N HBr/AcOH | (1d)<br>91,0 | C<br>H<br>N<br>Br | 38,49<br>5,14<br>17,90<br>27,86 | 38,11<br>5,15<br>17,28<br>28,17 |
| 25c | BOC-Pro-Lys(ε-Cbo)-pNA<br>$C_{30}H_{39}N_5O_8$ | 2b (5 mMol)<br>BOC-Pro-OpNP<br>(5,5 mMol) | (2c)<br>88,5 | C<br>H<br>N | 59,82<br>6,65<br>11,94 | 60,29<br>6,58<br>11,72 |
| 25d | $CF_3COOH$ · H-Pro-Lys(ε-Cbo)-pNA<br>$C_{27}H_{32}N_5O_8F_3$ | 25c (3 mMol)<br>6ml $CF_3COOH$ | (2d)<br>93,0 | C<br>H<br>N | 52,91<br>5,32<br>11,66 | 53,03<br>5,27<br>11,45 |
| 26c | BOC-CHG-Lys(ε-Cbo)-pNA<br>$C_{33}H_{45}N_5O_8$ | 2b (5 mMol)<br>BOC-CHG-OpNP<br>(5,5 mMol) | (2c)<br>84,6 | C<br>H<br>N | 61,50<br>7,08<br>11,18 | 61,95<br>7,09<br>10,95 |
| 26d | $CF_3COOH$ · H-CHG-Lys(ε-Cbo)-pNA<br>$C_{30}H_{38}N_5O_8F_3$ | 26c (3 mMol)<br>6ml $CF_3COOH$ | (2d)<br>90,5 | C<br>H<br>N | 54,75<br>5,93<br>11,08 | 55,12<br>5,86<br>10,71 |
| 30c | BOC-CHT-Lys(ε-Cbo)-pNA<br>$C_{34}H_{47}N_5O_9$ | 2b (5 mMol)<br>BOC-CHT-OpNP<br>(5,5 mMol) | (2c)<br>84,3 | C<br>H<br>N | 60,48<br>7,11<br>10,76 | 60,97<br>7,07<br>10,46 |
| 30d | $CF_3COOH$ · H-CHT-Lys(ε-Cbo)-pNA<br>$C_{31}H_{40}N_5O_9F_3$ | 30c (3 mMol)<br>6ml $CF_3COOH$ | (2d)<br>90,8 | C<br>H<br>N | 54,08<br>6,00<br>10,18 | 54,46<br>5,90<br>10,24 |
| 38c | Cbo-Tyr(OBzl)-Arg-pNA · HBr<br>$C_{36}H_{40}N_7O_7Br$ | 1b (5 mMol)<br>Cbo-Tyr(OBzl)-OpNP<br>(5,5 mMol) | (1c)<br>77,6 | C<br>H<br>N<br>Br | 56,08<br>5,35<br>12,95<br>10,15 | 56,69<br>5,29<br>12,86<br>10,48 |
| 38d | 2 HBr · H-Tyr-Arg-pNA<br>$C_{21}H_{29}N_7O_5Br_2$ | 38c (5 mMol)<br>2N HBr/AcOH | (1d)<br>83,7 | C<br>H<br>N<br>Br | 40,63<br>4,77<br>16,08<br>25,38 | 40,73<br>4,72<br>15,83<br>25,80 |
| 44c | Cbo-Ala-Arg-pNA · HBr<br>$C_{23}H_{30}N_7O_6Br$ | 1b (5 mMol)<br>Cbo-Ala-OpNP<br>(5,5 mMol) | (1c)<br>88,5 | C<br>H<br>N<br>Br | 47,19<br>5,22<br>17,18<br>13,60 | 47,59<br>5,21<br>16,89<br>13,77 |
| 44d | 2 HBr · H-Ala-Arg-pNA<br>$C_{15}H_{25}N_7O_4Br_2$ | 44c (3 mMol)<br>2N HBr/AcOH | (1d)<br>90,8 | C<br>H<br>N<br>Br | 34,01<br>4,76<br>18,85<br>29,88 | 34,17<br>4,78<br>18,60<br>30,31 |

Fortsetzung

| Beispiel | Dipeptid-Zwischenprodukt | Ausgangsprodukt (mMol) | Methode (Beispiel) Ausbeute (%) | Elementaranalyse | gef. % | ber. % |
|---|---|---|---|---|---|---|
| 48c | Cbo-Ph'Gly-Arg-pNA · HBr<br>$C_{28}H_{32}N_7O_6Br$ | 1b (5 mMol)<br>Cbo-Ph'Gly-OpNP<br>(5,5 mMol) | (1c)<br>86,7 | C<br>H<br>N<br>Br | 52,00<br>4,99<br>15,17<br>12,23 | 52,34<br>5,02<br>15,26<br>12,44 |
| 48d | 2 HBr · H-Ph'Gly-Arg-pNA<br>$C_{20}H_{27}N_7O_4Br_2$ | 48c (3 mMol)<br>2N HBr/AcOH | (1d)<br>93,4 | C<br>H<br>N<br>Br | 40,38<br>4,66<br>16,92<br>26,84 | 40,76<br>4,62<br>16,64<br>27,12 |
| 61c | Cbo-Nleu-Arg-pNA · HBr<br>$C_{26}H_{36}N_7O_6Br$ | 1b (5 mMol)<br>Cbo-Nleu-OpNP<br>(5,5 mMol) | (1c)<br>90,0 | C<br>H<br>N<br>Br | 49,82<br>5,88<br>16,03<br>12,62 | 50,16<br>5,83<br>15,75<br>12,84 |
| 61d | 2 HBr · H-Nleu-Arg-pNA<br>$C_{28}H_{31}N_7O_4Br_2$ | 61c (3 mMol)<br>2N HBr/AcOH | (1d)<br>94,6 | C<br>H<br>N<br>Br | 48,52<br>4,57<br>14,50<br>22,91 | 48,78<br>4,53<br>14,22<br>23,18 |
| 63c | Cbo-Nval-Arg-pNA · HBr<br>$C_{25}H_{34}N_7O_6Br$ | 1b (5 mMol)<br>Cbo-Nval-OpNP<br>(5,5 mMol) | (1c)<br>90,8 | C<br>H<br>N<br>Br | 49,13<br>5,69<br>16,38<br>13,00 | 49,35<br>5,63<br>16,11<br>13,13 |
| 63d | 2 HBr · H-Nval-Arg-pNA<br>$C_{17}H_{29}N_7O_4Br_2$ | 63c (3 mMol)<br>2N HBr/AcOH | (1d)<br>96,0 | C<br>H<br>N<br>Br | 36,45<br>5,30<br>18,01<br>28,55 | 36,77<br>5,26<br>17,66<br>28,78 |
| 69c | Cbo-But-Arg-pNA · HBr<br>$C_{24}H_{32}N_7O_6Br$ | 1b (5 mMol)<br>Cbo-But-OpNP<br>(5,5 mMol) | (1c)<br>91,6 | C<br>H<br>N<br>Br | 48,09<br>5,46<br>16,83<br>13,22 | 48,49<br>5,43<br>16,49<br>13,44 |
| 69d | 2 HBr · H-But-Arg-pNA<br>$C_{16}H_{27}N_7O_4Br_2$ | 69c (3 mMol)<br>2N HBr/AcOH | (1d)<br>94,2 | C<br>H<br>N<br>Br | 35,50<br>5,08<br>18,40<br>29,08 | 35,51<br>5,03<br>18,12<br>29,53 |

Tabelle 3

| Beispiel | Tripeptid-Zwischenprodukt | Ausgangsprodukt (mMol) | Methode (Beispiel) Ausbeute (%) | Elementaranalyse | gef. % | ber. % |
|---|---|---|---|---|---|---|
| 11e | Cbo-D-CHG-Leu-Arg-pNA · HBr<br>$C_{34}H_{49}N_8O_7Br$ | 11d (2 mMol)<br>Cbo-D-CHG-OpNP<br>(2,2 mMol) | (1e)<br>78,6 | C<br>H<br>N<br>Br | 53,04<br>6,49<br>15,15<br>10,22 | 53,67<br>6,48<br>14,71<br>10,49 |
| 12e | Cbo-D-Val-CHA-Arg-pNA · HBr<br>$C_{34}H_{49}N_8O_7Br$ | 1d (2 mMol)<br>Cbo-D-Val-OpNP<br>(2,2 mMol) | (1e)<br>82,7 | C<br>H<br>N<br>Br | 52,88<br>6,54<br>15,03<br>10,25 | 53,61<br>6,48<br>14,71<br>10,49 |
| 13e | Cbo-D-Ile-CHA-Arg-pNA · HBr<br>$C_{35}H_{51}N_8O_7Br$ | 1d (2 mMol)<br>Cbo-D-Ile-OpNP<br>(2,2 mMol) | (1e)<br>83,3 | C<br>H<br>N<br>Br | 53,80<br>6,64<br>14,75<br>10,11 | 54,19<br>6,63<br>14,45<br>10,30 |
| 14e | Cbo-D-Val-CHA-Lys(ε-Cbo)-pNA<br>$C_{42}H_{54}N_6O_9$ | 14d (2 mMol)<br>Cbo-D-Val-OpNP<br>(2,2 mMol) | (2e)<br>85,8 | C<br>H<br>N | 63,74<br>7,09<br>10,93 | 64,10<br>6,92<br>10,68 |
| 15e | Cbo-D-Ile-CHA-Lys(ε-Cbo)-pNA<br>$C_{43}H_{56}N_6O_9$ | 14d (2 mMol)<br>Cbo-D-Ile-OpNP<br>(2,2 mMol) | (2e)<br>86,9 | C<br>H<br>N | 64,11<br>7,13<br>10,60 | 64,48<br>7,05<br>10,49 |
| 16e | Cbo-D-CHG-Phe-Arg-pNA · HBr<br>$C_{37}H_{47}N_8O_7Br$ | 16d (1,5 mMol)<br>Cbo-D-CHG-OpNP<br>(1,65 mMol) | (1e)<br>84,1 | C<br>H<br>N<br>Br | 55,21<br>6,01<br>14,23<br>9,85 | 55,85<br>5,95<br>14,08<br>10,04 |
| 17e | Cbo-D-CHT-Phe-Arg-pNA · HBr<br>$C_{38}H_{49}N_8O_8Br$ | 16d (1,5 mMol)<br>Cbo-D-CHT-OpNP<br>(1,65 mMol) | (1e)<br>81,0 | C<br>H<br>N<br>Br | 54,96<br>5,99<br>13,75<br>9,39 | 55,27<br>5,98<br>13,57<br>9,68 |
| 18e | Cbo-D-Ph'Gly-CHA-Arg-pNA · HBr<br>$C_{37}H_{47}N_8O_7Br$ | 1d (2 mMol)<br>Cbo-D-Ph'Gly-OpNP<br>(2,2 mMol) | (1e)<br>82,7 | C<br>H<br>N<br>Br | 54,86<br>6,04<br>14,38<br>9,81 | 55,85<br>5,95<br>14,08<br>10,04 |
| 19e | Cbo-D-CHA-Pro-Arg-pNA · HBr<br>$C_{34}H_{47}N_8O_7Br$ | 19d (2 mMol)<br>Cbo-D-CHA-OpNP<br>(2,2 mMol) | (1e)<br>80,5 | C<br>H<br>N<br>Br | 53,41<br>6,30<br>15,08<br>10,24 | 53,75<br>6,24<br>14,75<br>10,52 |
| 20e | Cbo-D-CHG-CHA-Lys(ε-Cbo)-pNA<br>$C_{45}H_{58}N_6O_9$ | 14d (1,5 mMol)<br>Cbo-D-CHG-OpNP<br>(1,65 mMol) | (2e)<br>88,2 | C<br>H<br>N | 64,84<br>7,11<br>10,36 | 65,36<br>7,07<br>10,16 |
| 21e | Cbo-D-CHG-Leu-Lys(ε-Cbo)-pNA<br>$C_{42}H_{54}N_6O_9$ | 21d (2,5 mMol)<br>Cbo-D-CHG-OpNP<br>(2,75 mMol) | (2e)<br>90,2 | C<br>H<br>N | 63,73<br>7,00<br>10,85 | 64,10<br>6,92<br>10,68 |
| 22e | Cbo-D-CHT-Pro-Arg-pNA · HBr<br>$C_{34}H_{47}N_8O_8Br$ | 19d (1,5 mMol)<br>Cbo-D-CHT-OpNP<br>(1, 65mMol) | (1e)<br>83,3 | C<br>H<br>N<br>Br | 51,96<br>5,13<br>14,87<br>10,09 | 52,64<br>6,11<br>14,45<br>10,30 |

Fortsetzung

| Beispiel | Tripeptid-Zwischenprodukt | Ausgangsprodukt (mMol) | Methode (Beispiel) Ausbeute (%) | Elementaranalyse | gef. % | ber. % |
|---|---|---|---|---|---|---|
| 23e | Cbo-D-CHG-Pro-Arg-pNA · HBr<br>$C_{33}H_{45}N_8O_7Br$ | 19d (2 mMol)<br>Cbo-D-CHG-OpNP<br>(2,2 mMol) | (1e)<br>85,5 | C<br>H<br>N<br>Br | 53,04<br>6,17<br>15,49<br>10,45 | 53,15<br>6,08<br>15,03<br>10,72 |
| 24e | Cbo-D-CHT-Pip-Arg-pNA · HBr<br>$C_{35}H_{49}N_8O_8Br$ | 24d (1,5 mMol)<br>Cbo-D-CHT-OpNP<br>(1,65 mMol) | (1e)<br>78,1 | C<br>H<br>N<br>Br | 52,54<br>6,27<br>14,38<br>9,85 | 53,23<br>6,25<br>14,19<br>10,12 |
| 25e | Cbo-D-CHA-Pro-Lys(ε-Cbo)-pNA<br>$C_{42}H_{52}N_6O_9$ | 25d (2 mMol)<br>Cbo-D-CHA-OpNP<br>(2,2 mMol) | (2e)<br>82,5 | C<br>H<br>N | 64,54<br>6,75<br>10,92 | 64,27<br>6,68<br>10,71 |
| 26e | Cbo-D-Val-CHG-Lys(ε-Cbo)-pNA<br>$C_{41}H_{52}N_6O_9$ | 26d (1,5 mMol)<br>Cbo-D-Val-OpNP<br>(1,65 mMol) | (2e)<br>86,0 | C<br>H<br>N | 63,88<br>6,79<br>11,18 | 63,71<br>6,78<br>10,87 |
| 27e | Cbo-D-CHG-Pro-Lys(ε-Cbo)-pNA<br>$C_{41}H_{50}N_6O_9$ | 25d (2 mMol)<br>Cb-D-CHG-OpNP<br>(2,2 mMol) | (2e)<br>88,4 | C<br>H<br>N | 63,73<br>6,61<br>11,18 | 63,88<br>6,54<br>10,90 |
| 28e | Cbo-D-CHT-Pro-Lys(ε-Cbo)-pNA<br>$C_{42}H_{52}N_6O_{10}$ | 25d (2 mMol)<br>Cbo-D-CHT-OpNP<br>(2,2 mMol) | (2e)<br>80,8 | C<br>H<br>N | 62,61<br>6,58<br>10,70 | 62,98<br>6,54<br>10,49 |
| 29e | Cbo-D-Ph'Gly-CHA-Lys(ε-Cbo)-pNA<br>$C_{45}H_{52}N_6O_9$ | 14d (2 mMol)<br>Cbo-D-Ph'Gly-OpNP<br>(2,2 mMol) | (2e)<br>76,7 | C<br>H<br>N | 65,59<br>6,41<br>10,33 | 65,84<br>6,38<br>10,24 |
| 30e | Cbo-D-Val-CHT-Lys(ε-Cbo)-pNA<br>$C_{42}H_{54}N_6O_{10}$ | 30d (1,5 mMol)<br>Cbo-D-Val-OpNP<br>(1,65 mMol) | (2e)<br>77,9 | C<br>H<br>N | 62,80<br>6,87<br>10,61 | 62,83<br>6,78<br>10,47 |
| 31e | Cbo-D-Ph'Gly-CHT-Lys(ε-Cbo)-pNA<br>$C_{45}H_{52}N_6O_{10}$ | 30d (1,5 mMol)<br>Cbo-D-Ph'Gly-OpNP<br>(1,65 mMol) | (2e)<br>75,4 | C<br>H<br>N | 64,40<br>6,27<br>10,28 | 64,58<br>6,26<br>10,04 |
| 32e | Cbo-D-Leu-CHA-Arg-pNA · HBr<br>$C_{35}H_{51}N_8O_7Br$ | 1d (2 mMol)<br>Cbo-D-Leu-OpNP<br>(2,2 mMol) | (1e)<br>79,8 | C<br>H<br>N<br>Br | 53,95<br>6,70<br>14,66<br>10,14 | 54,19<br>6,63<br>14,45<br>10,30 |
| 33e | Cbo-D-Nleu-CHA-Arg-pNA · HBr<br>$C_{35}H_{51}N_8O_7Br$ | 1d (2 mMol)<br>Cbo-D-Nleu-OpNP<br>(2,2 mMol) | (1e)<br>80,4 | C<br>H<br>N<br>Br | 54,08<br>6,73<br>14,58<br>10,08 | 54,19<br>6,63<br>14,45<br>10,30 |
| 34e | Cbo-D-Nval-CHA-Arg-pNA · HBr<br>$C_{34}H_{49}N_8O_7Br$ | 1d (2 mMol)<br>Cbo-D-Nval-OpNP<br>(2,2 mMol) | (1e)<br>82,6 | C<br>H<br>N<br>Br | 53,28<br>6,55<br>14,93<br>10,25 | 53,61<br>6,48<br>14,71<br>10,49 |
| 35e | Cbo-D-Phe-CHA-Arg-pNA · HBr<br>$C_{38}H_{49}N_8O_7Br$ | 1d (2 mMol)<br>Cbo-D-Phe-OpNP<br>(2,2 mMol) | (1e)<br>84,5 | C<br>H<br>N<br>Br | 56,23<br>6,18<br>14,10<br>9,75 | 56,36<br>6,10<br>13,84<br>9,87 |

Fortsetzung

| Beispiel | Tripeptid-Zwischenprodukt | Ausgangsprodukt (mMol) | Methode (Beispiel) Ausbeute (%) | Elementaranalyse | gef. % | ber. % |
|---|---|---|---|---|---|---|
| 36e | Cbo-D-Ala-CHA-Arg-pNA · HBr | 1d (2 mMol) | (1e) | C | 52,01 | 52,39 |
| | $C_{32}H_{45}N_8O_7Br$ | Cbo-D-Ala-OpNP | 88,2 | H | 6,19 | 6,18 |
| | | (2,2 mMol) | | N | 15,44 | 15,27 |
| | | | | Br | 10,62 | 10,89 |
| 37e | Cbo-D-But-CHA-Arg-pNA · HBr | 1d (2 mMol) | (1e) | C | 52,88 | 53,01 |
| | $C_{33}H_{47}N_8O_7Br$ | Cbo-D-But-OpNP | 87,6 | H | 6,40 | 6,34 |
| | | (2,2 mMol) | | N | 15,28 | 14,99 |
| | | | | Br | 10,53 | 10,69 |
| 38e | Cbo-D-CHG-Tyr-Arg-pNA · HBr | 38d (1 mMol) | (1e) | C | 54,59 | 54,75 |
| | $C_{37}H_{47}N_8O_8Br$ | Cbo-D-CHG-OpNP | 74,1 | H | 5,88 | 5,84 |
| | | (1,1 mMol) | | N | 14,05 | 13,81 |
| | | | | Br | 9,57 | 9,84 |
| 39e | Cbo-D-CHA-Tyr-Arg-pNA · HBr | 38d (1 mMol) | (1e) | C | 54,96 | 55,27 |
| | $C_{38}H_{49}N_8O_8Br$ | Cbo-D-CHA-OpNP | 70,6 | H | 6,04 | 5,98 |
| | | (1,1 mMol) | | N | 13,77 | 13,57 |
| | | | | Br | 9,51 | 9,68 |
| 40e | Cbo-D-Nval-Tyr-Arg-pNA · HBr | 38d (1 mMol) | (1e) | C | 52,70 | 52,92 |
| | $C_{34}H_{43}N_8O_8Br$ | Cbo-D-Nval-OpNP | 69,8 | H | 5,64 | 5,62 |
| | | (1,1 mMol) | | N | 14,82 | 14,52 |
| | | | | Br | 10,12 | 10,35 |
| 41e | Cbo-D-CHT-Tyr-Arg-pNA · HBr | 38d (1 mMol) | (1e) | C | 54,18 | 54,22 |
| | $C_{38}H_{49}N_8O_9Br$ | Cbo-D-CHT-OpNP | 67,8 | H | 5,93 | 5,87 |
| | | (1,1 mMol) | | N | 13,29 | 13,31 |
| | | | | Br | 9,35 | 9,49 |
| 42e | Cbo-D-Phe-Tyr-Arg-pNA · HBr | 38d (1 mMol) | (1e) | C | 55,38 | 55,68 |
| | $C_{38}H_{43}N_8O_8Br$ | Cbo-D-Phe-OpNP | 70,5 | H | 5,34 | 5,29 |
| | | (1,1 mMol) | | N | 13,88 | 13,67 |
| | | | | Br | 9,60 | 9,75 |
| 43e | Cbo-D-Ph'Gly-Tyr-Arg-pNA · HBr | 38d (1 mMol) | (1e) | C | 54,96 | 55,16 |
| | $C_{37}H_{41}N_8O_8Br$ | Cbo-D-Ph'Gly-OpNP | 74,0 | H | 5,19 | 5,13 |
| | | (1,1 mMol) | | N | 14,11 | 13,91 |
| | | | | Br | 9,84 | 9,92 |
| 44e | Cbo-D-CHG-Ala-Arg-pNA · HBr | 44d (1,5 mMol) | (1e) | C | 51,66 | 51,74 |
| | $C_{31}H_{43}N_8O_7Br$ | Cbo-D-CHG-OpNP | 86,7 | H | 6,06 | 6,02 |
| | | (1,65 mMol) | | N | 15,80 | 15,57 |
| | | | | Br | 10,96 | 11,10 |
| 45e | Cbo-D-CHA-Ala-Arg-pNA · HBr | 44d (1,5 mMol) | (1e) | C | 52,09 | 52,39 |
| | $C_{32}H_{45}N_8O_7Br$ | Cbo-D-CHA-OpNP | 88,4 | H | 6,22 | 6,18 |
| | | (1,65 mMol) | | N | 15,55 | 15,27 |
| | | | | Br | 10,70 | 10,89 |
| 46e | Cbo-D-CHT-Leu-Arg-pNA · HBr | 11d (2 mMol) | (1e) | C | 52,78 | 53,09 |
| | $C_{35}H_{51}N_8O_8Br$ | Cbo-D-CHT-OpNP | 79,4 | H | 6,51 | 6,49 |
| | | (2,2 mMol) | | N | 14,30 | 14,15 |
| | | | | Br | 9,92 | 10,09 |

Fortsetzung

| Beispiel | Tripeptid-Zwischenprodukt | Ausgangsprodukt (mMol) | Methode (Beispiel) Ausbeute (%) | Elementaranalyse | gef. % | ber. % |
|---|---|---|---|---|---|---|
| 47e | Cbo-D-CHA-Leu-Arg-pNA · HBr<br>$C_{35}H_{51}N_8O_7Br$ | 11d (2 mMol)<br>Cbo-D-CHA-OpNP<br>(2,2 mMol) | (1e)<br>86,0 | C<br>H<br>N<br>Br | 53,88<br>6,65<br>14,75<br>10,18 | 54,19<br>6,63<br>14,45<br>10,30 |
| 48e | Cbo-D-Leu-Ph'Gly-Arg-pNA · HBr<br>$C_{34}H_{43}N_8O_7Br$ | 48d (1 mMol)<br>Cbo-D-Leu-OpNP<br>(1,1 mMol) | (1e)<br>79,5 | C<br>H<br>N<br>Br | 53,80<br>5,76<br>15,08<br>10,41 | 54,04<br>5,74<br>14,83<br>10,57 |
| 49e | Cbo-D-Nval-Ph'Gly-Arg-pNA · HBr<br>$C_{33}H_{41}N_8O_7Br$ | 48d (1 mMol)<br>Cbo-D-Nval-OpNP<br>(1,1 mMol) | (1e)<br>80,4 | C<br>H<br>N<br>Br | 53,19<br>5,62<br>15,24<br>10,65 | 53,44<br>5,57<br>15,11<br>10,77 |
| 50e | Cbo-D-Ala-Ph'Gly-Arg-pNA · HBr<br>$C_{31}H_{37}N_8O_7Br$ | 48d (1 mMol)<br>Cbo-D-Ala-OpNP<br>(1,1 mMol) | (1e)<br>81,6 | C<br>H<br>N<br>Br | 52,00<br>5,29<br>15,95<br>11,03 | 52,18<br>5,23<br>15,70<br>11,20 |
| 51e | Cbo-D-CHA-Ph'Gly-Arg-pNA · HBr<br>$C_{37}H_{47}N_8O_7Br$ | 48d (1 mMol)<br>Cbo-D-CHA-OpNP<br>(1,1 mMol) | (1e)<br>78,5 | C<br>H<br>N<br>Br | 55,80<br>6,01<br>14,33<br>9,88 | 55,85<br>5,95<br>14,08<br>10,04 |
| 52e | Cbo-D-CHT-Ph'Gly-Arg-pNA · HBr<br>$C_{37}H_{47}N_8O_8Br$ | 48d (1 mMol)<br>Cbo-D-CHT-OpNP<br>(1,1 mMol) | (1e)<br>74,6 | C<br>H<br>N<br>Br | 54,85<br>5,90<br>14,08<br>9,70 | 54,75<br>5,84<br>13,81<br>9,84 |
| 53e | Cbo-D-CHG-Ph'Gly-Arg-pNA · HBr<br>$C_{36}H_{45}N_8O_7Br$ | 48d (1 mMol)<br>Cbo-D-CHG-OpNP<br>(1,1 mMol) | (1e)<br>77,0 | C<br>H<br>N<br>Br | 55,39<br>5,86<br>14,53<br>10,08 | 55,31<br>5,80<br>14,34<br>10,22 |
| 54e | Cbo-D-CHG-CHG-Arg-pNA · HBr<br>$C_{36}H_{51}N_8O_7Br$ | 54d (1,5 mMol)<br>Cbo-D-CHG-OpNP<br>(1,65 mMol) | (1e)<br>82,6 | C<br>H<br>N<br>Br | 54,99<br>6,60<br>14,41<br>9,89 | 54,89<br>6,53<br>14,23<br>10,14 |
| 55e | Cbo-D-Phe-CHG-Arg-pNA · HBr<br>$C_{37}H_{47}N_8O_7Br$ | 54d (1,5 mMol)<br>Cbo-D-Phe-OpNP<br>(1,65 mMol) | (1e)<br>88,7 | C<br>H<br>N<br>Br | 55,78<br>6,01<br>14,22<br>9,88 | 55,85<br>5,95<br>14,08<br>10,04 |
| 56e | Cbo-D-Ph'Gly-CHG-Arg-pNA · HBr<br>$C_{36}H_{45}N_8O_7Br$ | 54d (1,5 mMol)<br>Cbo-D-Ph'Gly-OpNP<br>(1,65 mMol) | (1e)<br>83,5 | C<br>H<br>N<br>Br | 55,45<br>5,86<br>14,55<br>10,09 | 55,31<br>5,80<br>14,34<br>10,22 |
| 57e | Cbo-D-Ph'Gly-Phe-Arg-pNA · HBr<br>$C_{37}H_{41}N_8O_7Br$ | 16d (1 mMol)<br>Cbo-D-Ph'Gly-OpNP<br>(1,1 mMol) | (1e)<br>86,0 | C<br>H<br>N<br>Br | 56,18<br>5,24<br>14,33<br>9,95 | 56,27<br>5,23<br>14,19<br>10,12 |

Fortsetzung

| Beispiel | Tripeptid-Zwischenprodukt | Ausgangsprodukt (mMol) | Methode (Beispiel) Ausbeute (%) | Elementaranalyse | gef. % | ber. % |
|---|---|---|---|---|---|---|
| 58e | Cbo-D-CHA-Phe-Arg-pNA · HBr<br>$C_{38}H_{49}N_8O_7Br$ | 16d (1 mMol)<br>Cbo-D-CHA-OpNP<br>(1,1 mMol) | (1e)<br>85,6 | C<br>H<br>N<br>Br | 56,18<br>6,12<br>14,01<br>9,81 | 56,36<br>6,10<br>13,84<br>9,87 |
| 59e | Cbo-D-CHG-Pip-Arg-pNA · HBr<br>$C_{34}H_{47}N_8O_7Br$ | 24d (1 mMol)<br>Cbo-D-CHG-OpNP<br>(1,1 mMol) | (1e)<br>42,1 | C<br>H<br>N<br>Br | 53,53<br>6,27<br>14,95<br>10,39 | 53,75<br>6,24<br>14,75<br>10,52 |
| 60e | Cbo-D-CHA-Pip-Arg-pNA · HBr<br>$C_{35}H_{49}N_8O_7Br$ | 24d (1 mMol)<br>Cbo-D-CHA-OpNP<br>(1,1 mMol) | (1e)<br>44,8 | C<br>H<br>N<br>Br | 54,17<br>6,41<br>14,62<br>10,13 | 54,33<br>6,38<br>14,48<br>10,33 |
| 61e | Cbo-D-CHG-Nleu-Arg-pNA · HBr<br>$C_{34}H_{49}N_8O_7Br$ | 61d (2 mMol)<br>Cbo-D-CHG-OpNP<br>(2,2 mMol) | (1e)<br>86,7 | C<br>H<br>N<br>Br | 53,43<br>6,53<br>14,95<br>10,28 | 53,61<br>6,48<br>14,71<br>10,49 |
| 62e | Cbo-D-CHA-Nleu-Arg-pNA · HBr<br>$C_{35}H_{51}N_8O_7Br$ | 61d (2 mMol)<br>Cbo-D-CHA-OpNP<br>(2,2 mMol) | (1e)<br>83,5 | C<br>H<br>N<br>Br | 53,88<br>6,71<br>14,59<br>10,21 | 54,19<br>6,63<br>14,45<br>10,30 |
| 63e | Cbo-D-CHA-Nval-Arg-pNA · HBr<br>$C_{34}H_{49}N_8O_7Br$ | 63d (1 mMol)<br>Cbo-D-CHA-OpNP<br>(1,1 mMol) | (1e)<br>86,0 | C<br>H<br>N<br>Br | 53,48<br>6,46<br>14,80<br>10,31 | 53,61<br>6,48<br>14,71<br>10,49 |
| 64e | Cbo-D-CHG-Nval-Arg-pNA · HBr<br>$C_{33}H_{47}N_8O_7Br$ | 63d (1 mMol)<br>Cbo-D-CHG-OpNP<br>(1,1 mMol) | (1e)<br>88,8 | C<br>H<br>N<br>Br | 52,88<br>6,35<br>15,18<br>10,49 | 53,01<br>6,34<br>14,99<br>10,69 |
| 65e | Cbo-D-Nval-CHA-Lys($\varepsilon$-Cbo)-pNA<br>$C_{42}H_{54}N_6O_9$ | 14d (1,5 mMol)<br>Cbo-D-Nval-OpNP<br>(1,65 mMol) | (2e)<br>90,6 | C<br>H<br>N | 63,90<br>6,98<br>10,82 | 64,10<br>6,92<br>10,68 |
| 66e | Cbo-D-But-CHA-Lys($\varepsilon$-Cbo)-pNA<br>$C_{41}H_{52}N_6O_9$ | 14d (1,5 mMol)<br>Cbo-D-But-OpNP<br>(1,65 mMol) | (2e)<br>91,4 | C<br>H<br>N | 63,48<br>6,82<br>10,99 | 63,71<br>6,78<br>10,87 |
| 67e | Cbo-D-Leu-CHA-Lys($\varepsilon$-Cbo)-pNA<br>$C_{43}H_{56}N_6O_9$ | 14d (1,5 mMol)<br>Cbo-D-Leu-OpNP<br>(1,65 mMol) | (2e)<br>88,5 | C<br>H<br>N | 64,28<br>7,11<br>10,75 | 64,48<br>7,05<br>10,49 |
| 68e | Cbo-D-Nleu-CHA-Lys($\varepsilon$-Cbo)-pNA<br>$C_{43}H_{56}N_6O_9$ | 14d (1,5 mMol)<br>Cbo-D-Nleu-OpNP<br>(1,65 mMol) | (2e)<br>88,9 | C<br>H<br>N | 64,18<br>7,08<br>10,60 | 64,48<br>7,05<br>10,49 |
| 69e | Cbo-D-CHG-But-Arg-pNA · HBr<br>$C_{32}H_{45}N_8O_7Br$ | 69d (2 mMol)<br>Cbo-D-CHG-OpNP<br>(2,2 mMol) | (1e)<br>84,7 | C<br>H<br>N<br>Br | 52,19<br>6,17<br>15,35<br>10,70 | 52,39<br>6,18<br>15,27<br>10,89 |

Fortsetzung

| Beispiel | Tripeptid-Zwischenprodukt | Ausgangsprodukt (mMol) | Methode (Beispiel) Ausbeute (%) | Elementaranalyse | gef. % | ber. % |
|---|---|---|---|---|---|---|
| 70e | Cbo-D-CHA-But-Arg-pNA · HBr $C_{33}H_{47}N_8O_7Br$ | 69d (2 mMol) Cbo-D-CHA-OpNP (2,2 mMol) | (1e) 80,6 | C | 52,87 | 53,01 |
| | | | | H | 6,41 | 6,34 |
| | | | | N | 15,17 | 14,99 |
| | | | | Br | 10,55 | 10,69 |
| 71e | Cbo-D-CHT-But-Arg-pNA · HBr $C_{33}H_{47}N_8O_8Br$ | 69d (2 mMol) Cbo-D-CHT-OpNP (2,2 mMol) | (1e) 83,3 | C | 51,72 | 51,90 |
| | | | | H | 6,25 | 6,20 |
| | | | | N | 14,88 | 14,67 |
| | | | | Br | 10,37 | 10,46 |

Beispiel 72

2 AcOH · H-D-Nval-CHA-Arg-pNA

7,09 g (10 mMol) 2 HBr · E-D-Nval-CHA-Arg-pNA (hergestellt gemäß Beispiel 34) wurden in 75 ml 60%igem wäßrigem MeOH gelöst. Die Lösung wurde auf eine Säule von »Amberlite« JRA-401 in der Acetatform gegeben. Die Säule wurde mittels 60%igem wäßrigem MeOH eluiert, wobei durch Ionenaustausch HBr durch AcOH ersetzt wurde. Das Eluat wurde im Vakuum bei 40°C zur Trockne eingeengt. Nach dem Trocknen im Vakuumtrockenschrank bei 40°C über $P_2O_5$ erhielt man 6,33 g bromidfreies 2 AcOH · H-D-Nval-CHA-Arg-pNA (98,5% der Theorie).

Nach dieser Methode kann man aus dem obengenannten Tripeptidderivat entsprechend andere Salze mit organischen Säuren, z. B. Ameisen-, Propion-, Oxal-, Wein-, Zitronen-, Milch-, Benzoe-, Chlorbenzoe-, Salicyl- oder Phthalsäure, herstellen. Man kann als Ionenaustauscher z. B. »Amberlite« JRA-401 in der Hydrochloridform verwenden und in die gewünschte Säuresalzform überführen, indem man den genannten Ionenaustauscher durch Behandlung mit Natronlauge in die basische OH-Form überführt und dann mit einer Lösung eines 1 : 1-Gemisches der gewünschten organischen Säure und deren Natriumsalz in 60%igem wäßrigem MeOH behandelt.

Die quantitativen Enzymbestimmungen mittels der erfindungsgemäßen Tripeptidsubstrate können folgendermaßen durchgeführt werden:

1. Bestimmung von Urinkallikrein

1 ml Urin und 1 ml TRIS-Imidazol-Puffer mit pH 7,9 und Ionenstärke 1,0 werden 5 Min. bei 37°C inkubiert und dann zentrifugiert, um Sedimente zu entfernen.

In einer Plastikküvette werden 1,4 ml dest. $H_2O$ von 37°C und 0,4 ml des Zentrifugats gut gemischt. Zu dieser Mischung gibt man 0,2 ml einer $2 \times 10^{-3}$ molaren wäßrigen Substratlösung und durchmischt die Komponenten schnell. Dieses Gemisch wird genau 15 Min. bei 37°C inkubiert. Dann wird dem Reaktionsgemisch 0,2 ml Eisessig zugemischt, um die Enzymreaktion zu unterbrechen. Zur Farbmessung verwendet man eine Vergleichsprobe (Blindprobe), die aus den gleichen Komponenten zusammengesetzt ist, wobei jedoch der Eisessig vor Zugabe des Substrates zugesetzt wird, um die Enzymreaktion zu verhindern. Dann wird bei 405 nm photometrisch oder spektrophotometrisch die Menge der gebildeten farbigen Verbindung p-Nitroanilin aus der Differenz zwischen der Blindprobe und der zu bestimmenden Probe ermittelt. Aus dem ermittelten Wert wird die Urinkallikreinaktivität im Urin nach der folgenden Formel berechnet:

$$\frac{\Delta\, OD_{15\,Min.} \times V \times 1000 \times F}{15\ Min. \times v \times \varepsilon_{pNA}} = mU/ml\ Urin.$$

| | | |
|---|---|---|
| $\Delta OD$ | = | Zunahme der optischen Dichte bei 405 nm nach 15 Min. |
| V | = | Totalvolumen des Bestimmungsansatzes = 2,2 ml |
| 1000 | = | Umrechnungsfaktor zur Umwandlung von U in mU |
| F | = | Verdünnungsfaktor des Urins (2) |
| v | = | Volumen der Probe = 0,4 ml |
| $\varepsilon_{pNA}$ | = | Extinktionskoeffizient des p-Nitroanilins (10 400 $Mol^{-1} \times cm^{-1}$) bei 405 nm dividiert durch 1000 = 10,4 $mMol^{-1} \times cm^{-1}$ |

Die Berechnung des Urinkallikreins im Urin kann auch durch eine kontinuierliche Messung des entstehenden p-Nitroanilins durchgeführt werden. Diese Methode ist nachstehend für die Bestimmung von Glandulärkallikrein im Sputum beschrieben.

Im Urin ist neben dem Urinkallikrein auch Urokinase als proteolytisches Enzym vorhanden, welches gegebenenfalls die erfindungsgemäßen Substrate ebenfalls, wenn auch nur geringfügig, spalten kann. Bei der oben beschriebenen Bestimmungsmethode mißt man somit die Summe der Aktivitäten von Urinkallikrein und Urokinase. Um die genaue Urinkallikreinaktivität zu erhalten, muß man die Urokinaseaktivität abziehen, welche dadurch bestimmt werden kann, daß man in einem Vergleichsversuch durch Zugabe von 0,075 Einheiten Trypsininhibitor (Trypsininhibitor aus Rinderlunge) pro ml Puffer die Urinkallikreinaktivität vollständig hemmt und nur die Urokinaseaktivität mißt.

2. Bestimmung des Glandulärkallikreins im Sputum

Man mischt 0,5 ml Sputum mit 2 ml TRIS-Imidazol-Puffer (Ionenstärke 1,0) und präinkubiert die Mischung 5 Min. bei 37° C. Das Inkubat wird zentrifugiert. In eine Testküvette wird 1,5 ml dest. $H_2O$ von 37° C vorgelegt und mit 0,25 ml des Zentrifugats versetzt. Die Komponenten werden gut gemischt. Dann wird 0,2 ml $2 \times 10^{-3}$ molare wäßrige Substratlösung zugesetzt und gemischt. Dann wird bei 405 nm die Extinktionsänderung des kontinuierlich freigesetzten p-Nitroanilins mittels eines Schreibers während 5—10 Min. verfolgt. Aus dem ermittelten $\Delta$OD pro Min. wird mitteils der nachstehenden Formel die Kallikreinaktivität pro ml Sputum in mU errechnet:

$$\frac{\Delta\, OD_{Min.} \times V \times 1000 \times F}{v \times \varepsilon_{pNA}} = \text{mU/ml Sputum}.$$

F = 5
V = 1,95
v = 0,25
1 U (Einheit) = Enzymmenge, die während 1 Min. 1 µMol Substrat unter optimalen oder sonst festgelegten Bedingungen in bezug auf pH, Ionenstärke, Temperatur und Substratkonzentration zu spalten vermag.

In Pankreassaft liegt das Pankreaskallikrein hauptsächlich in Form von Präkallikrein vor und kann erst nach Aktivierung, z. B. mittels Trypsin, bestimmt werden. Das Trypsin wird dabei nach der Aktivierung des Präkallikreins mittels Soyabohnentrypsininhibitor (SBTI) gehemmt. In diesem Aktivierungsgemisch läßt sich der Kallikreingehalt nach einer der oben beschriebenen Methoden bestimmen.

3. Bestimmung von Plasmin

Man mischt 1,7 ml TRIS-Imidazol-Puffer (pH 7,5, Ionenstärke 0,2) bei 37° C mit 0,1 ml einer Lösung von Plasmin in 25%igem Glycerin und inkubiert die Mischung während 1 Min. bei 37° C. Dann gibt man der Mischung 0,2 ml einer wäßrigen $2 \times 10^{-3}$ molaren Substratlösung von 37° C zu und durchmischt das Ganze schnell. Man ermittelt dann kontinuierlich die pro Zeiteinheit aus dem Substrat freigesetzte Menge des p-Nitroanilins. Aus dem pro Minute ermittelten Wert berechnet man dann die Plasminaktivität pro ml Probe in mU nach der folgenden Formel:

$$\frac{\Delta\, E/Min. \times V \times 1000}{v \times \varepsilon_{pNA}} = \text{mU/ml Probe}.$$

$\Delta E$ = pro Minute freigesetzte Menge des Spaltproduktes
V = Totalvolumen des Testansatzes
v = Volumen der Probe
$\varepsilon_{pNA}$ = Extinktionskoeffizient des p-Nitroanilins (10 400 $Mol^{-1} \times cm^{-1}$) bei 405 nm dividiert durch 1000

4. Bestimmung von Antiplasmin in Humanplasma

0,1 ml von mit TRIS-Imidazol-Puffer im Verhältnis 1 : 20 verdünntem Plasma wird mit 0,02 ml einer Lösung von 1,25 CU Humanplasmin (Präparat der Firma AB Kabi, Stockholm, Schwefen) und 50 ATU Hirudin (Präparat der Firma Pentapharm AG, Basel, Schweiz) pro ml in 25%igem Glycerin gemischt. Die Mischung wird während 90 Sek. bei 37° C inkubiert. Man mischt das Inkubat mit 1,7 ml

TRIS-Imidazol-Puffer von 37°C (pH 7,5, Ionenstärke 0,2) und dann mit 0,2 ml $2 \times 10^{-3}$ molarer wäßriger Substratlösung. Dann mißt man kontinuierlich die pro Zeiteinheit aus dem Substrat freigesetzte Menge des p-Nitroanilins. Aus dem ermittelten Wert berechnet man in der oben angegebenen Weise die restliche Plasminaktivität.

In einem Blindversuch wird das Plasma durch die entsprechende Menge Puffer ersetzt, sonst aber in der oben beschriebenen Weise gearbeitet. Die ermittelte Plasminaktivität entspricht der eingesetzten Menge Plasmin. Man ermittelt die Antiplasminaktivität aus der Differenz zwischen der im Blindversuch bestimmten Plasminaktivität und der im Test mit Plasma bestimmten restlichen Plasminaktivität nach folgender Formel:

$$\frac{(\Delta E_{Blindprobe} - \Delta E_{Plasmaprobe})/Min. \times V \times F \times 1000}{v \times \varepsilon_{pNA}} = mIU/ml\ Plasma.$$

F = Verdünnungsfaktor des Plasmas (20)

Mit den erfindungsgemäßen Substraten kann man ferner Plasminogen in Humanplasma bestimmen, indem man in einem Puffersystem das im Plasma vorhandene Plasminogen mittels Urokinase oder Streptokinase in Plasmin überführt und die Menge des entstandenen Plasmins mittels eines erfindungsgemäßen Substrates nach der oben beschriebenen Plasminbestimmungsmethode bestimmt. Aus dem für Plasmin ermittelten Wert ergibt sich die im Plasma ursprünglich vorhandene Menge Plasminogen, da bei der Aktivierung aus 1 Molekül Plasminogen 1 Molekül Plasmin entsteht.

In der nachfolgenden Tabelle 4 ist die Suszeptibilität einiger der erfindungsgemäßen Substrate gegenüber Organ- oder Glandulärkallikreinen, Plasmin und Thrombin angegeben.

Tabelle 4

Aktivität von Urinkallikrein in 1 ml menschlichem Urin*), Submandibularis-Kallikrein in 1 ml Sputum, Humanplasmin und humanem NIH-Thrombin, gemessen mittels der erfindungsgemäßen Substrate bei konstanter Substrat- und Enzymkonzentration. Zum Vergleich (ausgenommen Thrombin) sind die entsprechenden Werte für die vorbekannten, im Handel erhältlichen Substrate

2 HCl · H-D-Val-Leu-Arg-pNA (A) und 2 HCl · H-D-Val-Leu-Lys-pNA (B)

(s. DOS 2 629 067) angeführt. Substratkonzentration $2 \times 10^{-4}$ molar.

| | Menge des durch 1 ml Humanurin, 1 ml Humansputum, 1 CU-Einheit Humanplasmin und 1 NIH-Einheit Thrombin pro Min. freigesetzten Spaltproduktes $R-NH_2$ in Nanomol | | | |
|---|---|---|---|---|
| | Urinkallikrein | Submandibularis-Kallikrein | Humanplasmin | Humanthrombin |
| A | 0,90 | 12,2 | 83 | |
| B | 0,30 | 5,7 | 347 | |
| Substrate gem. Beispiel | | | | |
| 1 | 0,84 | 12,9 | 699 | 8,27 |
| 2 | 0,80 | 25,6 | 1150 | |
| 3 | 2,55 | 31,2 | 430 | |
| 4 | 0,60 | 15,7 | 1120 | |
| 5 | 2,42 | 28,5 | 408 | |
| 6 | 0,72 | 24,8 | 1215 | |
| 7 | 2,56 | 31,1 | 428 | |

Fortsetzung

| | Menge des durch 1 ml Humanurin, 1 ml Humansputum, 1 CU-Einheit Humanplasmin und 1 NIH-Einheit Thrombin pro Min. freigesetzten Spaltproduktes R—$NH_2$ in Nanomol | | | |
|---|---|---|---|---|
| | Urinkallikrein | Submandibularis-Kallikrein | Humanplasmin | Humanthrombin |
| Substrate gem. Beispiel | | | | |
| 8 | 0,58 | 17,6 | 1180 | |
| 9 | 2,38 | 25,4 | 388 | |
| 10 | 0,61 | 14,9 | 1165 | |
| 11 | 0,60 | 11,6 | 381 | 33,9 |
| 12 | 2,75 | 30,5 | 421 | 4,0 |
| 13 | 2,67 | 36,6 | 446 | 7,44 |
| 14 | 1,02 | 6,7 | 505 | 0,35 |
| 15 | 2,50 | 23,1 | 440 | 0,39 |
| 16 | 3,31 | 32,2 | 1200 | 17,1 |
| 17 | 0,77 | 7,4 | 860 | 18,7 |
| 18 | 0,67 | 8,5 | 1200 | 4,53 |
| 19 | 1,40 | 8,1 | 1200 | 54,4 |
| 20 | 0,28 | 4,4 | 500 | 0,99 |
| 21 | 0,40 | 2,2 | 460 | 1,20 |
| 22 | 1,20 | 21,4 | 690 | 45,8 |
| 23 | 0,33 | 12,9 | 511 | 96,3 |
| 24 | 0 | 9,9 | 122 | 80,4 |
| 25 | 0,71 | 4,4 | 639 | 4,54 |
| 26 | 0 | 1,3 | 61 | 0,27 |
| 27 | 0 | 1,9 | 231 | 3,78 |
| 28 | 0,20 | 3,8 | 613 | 0,8 |
| 29 | 0,10 | 0,9 | 837 | 0,4 |
| 30 | 1,40 | 16,5 | 532 | 0,4 |
| 31 | 0,10 | 0,9 | 894 | 0,5 |
| 32 | 3,11 | 35,9 | 1069 | 6,8 |
| 33 | 2,08 | 28,1 | 1179 | 7,3 |

Fortsetzung

| | Menge des durch 1 ml Humanurin, 1 ml Humansputum, 1 CU-Einheit Humanplasmin und 1 NIH-Einheit Thrombin pro Min. freigesetzten Spaltproduktes $R-NH_2$ in Nanomol | | | |
|---|---|---|---|---|
| | Urinkallikrein | Submandibularis-Kallikrein | Humanplasmin | Humanthrombin |
| Substrate gem. Beispiel | | | | |
| 34 | 3,12 | 41,3 | 969 | 5,1 |
| 35 | 2,14 | 28,5 | 814 | 3,9 |
| 36 | 1,73 | 22,4 | 433 | 1,5 |
| 37 | 2,99 | 31,2 | 623 | 2,8 |
| 38 | 0,49 | 3,9 | 969 | 2,0 |
| 39 | 0,49 | 5,2 | 1407 | 8,3 |
| 40 | 0,42 | 4,9 | 1219 | 1,8 |
| 41 | 0,59 | 10,9 | 1252 | 9,5 |
| 42 | 0,26 | 6,1 | 843 | 2,6 |
| 43 | 0,19 | 7,0 | 818 | 1,0 |
| 44 | 0,72 | 9,5 | 239 | 158 |
| 45 | 0,66 | 10,6 | 389 | 163,5 |
| 46 | 0,33 | 11,7 | 596 | 58,1 |
| 47 | 0,64 | 8,8 | 555 | 57,6 |
| 48 | 0,67 | 25,8 | 422 | 27,4 |
| 49 | 0,58 | 22,9 | 382 | 19,2 |
| 50 | 0,42 | 23,5 | 141 | 4,2 |
| 51 | 0,64 | 29,4 | 559 | 120,2 |
| 52 | 0,45 | 31,1 | 591 | 112,1 |
| 53 | 0,38 | 22,1 | 446 | 63,7 |
| 54 | 0,33 | 5,9 | 101 | 7,5 |
| 55 | 0,33 | 4,9 | 60 | 3,7 |
| 56 | 0,25 | 7,2 | 26 | 2,4 |
| 57 | 0,42 | 7,0 | 841 | 2,4 |
| 58 | 0,5 | 9,7 | 1261 | 25,8 |
| 59 | 1,92 | 14,2 | 259 | 142,5 |

Fortsetzung

| | Menge des durch 1 ml Humanurin, 1 ml Humansputum, 1 CU-Einheit Humanplasmin und 1 NIH-Einheit Thrombin pro Min. freigesetzten Spaltproduktes $R{-}NH_2$ in Nanomol | | | |
|---|---|---|---|---|
| | Urinkallikrein | Submandibularis-Kallikrein | Humanplasmin | Humanthrombin |
| Substrate gem. Beispiel | | | | |
| 60 | 0,53 | 9,8 | 228 | 114,2 |
| 61 | 0,53 | 13,5 | 739 | 41,0 |
| 62 | 0,45 | 15,0 | 899 | 77,6 |
| 63 | 0,35 | 14,2 | 574 | 97,1 |
| 64 | 0,38 | 24,2 | 488 | 67,4 |
| 65 | 1,87 | 18,04 | 655 | 6,9 |
| 66 | 2,22 | 21,0 | 532 | 3,6 |
| 67 | 1,73 | 19,3 | 778 | 3,4 |
| 68 | 0,77 | 8,4 | 695 | 3,9 |
| 69 | 0,51 | 14,5 | 439 | 102,9 |
| 70 | 0,51 | 18,1 | 478 | 108,9 |
| 71 | 0,64 | 18,2 | 463 | 114,2 |

*) Urinprobe erhalten durch Mischen von je 100 ml Morgenurin gesunder Personen.

Bei der Messung des durch die enzymatische Hydrolyse des Substrates gebildeten Spaltproduktes $R-NH_2$ geht man von der Voraussetzung aus, daß das Spaltprodukt ein UV-Spektrum besitzt, das von demjenigen des Substrates verschieden und in Richtung höherer Wellenlängen verschoben ist. Die Absorption des Substrates bei 405 nm ist praktisch Null. p-Nitroanilin als Spaltprodukt weist ein Absorptionsmaximum bei 380 nm und einen molaren Extinktionskoeffizienten von 13 200 auf. Bei 405 nm ist der Extinktionskoeffizient nur wenig niedriger, d. h. 10 400. Der Grad der enzymatischen Hydrolyse des Substrates, welcher der Menge des abgespaltenen p-Nitroanilins proportional ist, läßt sich durch spektrophotometrische Messung bei 405 nm bestimmen. Auch bei Anwesenheit eines Überschusses an Substrat wird die Messung bei 405 nm nicht gestört.

Bei den Substraten, die als chromogene Gruppe eine 2-Naphthylamino-, 4-Methoxy-2-naphthylamino-, 4-Methyl-cumaryl-(7)-amino- oder 1,3-Di(methoxycarbonyl)-phenyl-(5)-amino-Gruppe enthalten, wird die Menge des Spaltproduktes $R-H$ durch Fluoreszenzspektrophotometrie gemessen. In einem aus Enzym, Puffer und Substrat bestehenden Testsystem mißt man kontinuierlich das energieärmere emittierte Licht bei 400–470 nm, nachdem man das gebildete fluoreszierende Spaltprodukt mit energiereicherem Licht erregt hat. Die pro Zeiteinheit gebildete Menge Spaltprodukt ist ein Maß für die vorhandene Enzymaktivität. 1 µMol Spaltprodukt pro Minute entspricht definitionsgemäß 1 Enzym-Einheit, bezogen auf ein gegebenes Substrat.

**Patentansprüche für die Vertragsstaaten: BE, CH, DE, FR, GB, IT, LI, LU, NL, SE**

1. Tripeptidderivate der Formel

H–D–X–Y–Z–R I

in welcher Z eine Arginyl- oder Lysyl-Gruppe, R eine p-Nitrophenylamino-, 2-Naphthylamino-, 4-Methoxy-2-naphthylamino-, 4-Methyl-cumaryl-(7)-amino-, 1,3-Di(methoxycarbonyl)-phenyl-(5)-amino-, Chinonylamino- oder Nitrochinonylamino-Gruppe, X eine Cyclohexylglycyl-, Cyclohexylalanyl- oder p-Hydroxycyclohexylalanyl-Gruppe und Y eine Alanyl-, α-Aminobutyryl-, Valyl-, Norvalyl-, Leucyl-, Norleucyl-, Isoleucyl-, Prolyl- oder Pipecolinoyl-Gruppe darstellen, und deren Salze mit Säuren.

2. Tripeptidderivate der im Anspruch 1 angegebenen Formel, in welchen Z und R die im Anspruch 1 definierte Bedeutung besitzen und X eine Cyclohexylglycyl-, Cycylohexylalanyl-, p-Hydroxycyclohexylalanyl-, Phenylalanyl- oder Phenylglycyl-Gruppe und Y eine Phenylalanyl-, Phenylglycyl- oder Tyrosyl-Gruppe darstellen, und deren Salze mit Säuren.

3. Tripeptidderivate der im Anspruch 1 angegebenen Formel, in welchen Z und R die im Anspruch 1 definierte Bedeutung besitzen und X eine Alanyl-, α-Aminobutyryl-, Valyl-, Norvalyl-, Leucyl-, Norleucyl- oder Isoleucyl-Gruppe und Y eine Cyclohexylglycyl-, Cyclohexylalanyl-, p-Hydroxycyclohexylalanyl-, Phenylglycyl- oder Tyrosyl-Gruppe darstellen, und deren Salze mit Säuren.

4. Tripeptidderivate der im Anspruch 1 angegebenen Formel, in welchen Z und R die im Anspruch 1 definierte Bedeutung besitzen und X eine Phenylalanyl-, Phenylglycyl- oder Cyclohexylglycyl-Gruppe und Y eine Cyclohexylalanyl-, Cyclohexylglycyl- oder p-Hydroxycyclohexylalanyl-Gruppe darstellen, und deren Salze mit Säuren.

5. Tripeptidderivate gemäß Anspruch 1, in welchen das an Z gebundene Dipeptidfragment eine CHG-Ala-, CHG-But-, CHG-Norval-, CHG-Leu-, CHG-Norleu-, CHG-Pro-, CHG-Pip-, CHA-Ala-, CHA-But-, CHA-Norval-, CHA-Leu-, CHA-Norleu-, CHA-Pro-, CHA-Pip-, HCA-But-, HCA-Leu-, HCA-Pro- oder HCA-Pip-Gruppe ist.

6. Tripeptidderivate gemäß Anspruch 2, in welchen das an Z gebundene Dipeptidfragment eine CHG-Phe-, CHG-Ph'Gly-, CHG-Tyr-, CHA-Phe-, CHA-Ph'Gly-, CHA-Tyr-, HCA-Phe-, HCA-Ph'Gly-, HCA-Tyr-, Phe-Tyr-, Ph'Gly-Phe- oder Ph'Gly-Tyr-Gruppe ist.

7. Tripeptidderivate gemäß Anspruch 3, in welchen das an Z gebundene Dipeptidfragment eine Ala-CHA-, Als-Ph'Gly-, But-CHA-, Val-CHG-, Val-CHA-, Val-HCA-, Norval-CHA-, Norval-Ph'Gly-, Norval-Tyr-, Leu-CHA-, Leu-Ph'Gly-, Norleu-CHA- oder Ile-CHA-Gruppe ist.

8. Tripeptidderivate gemäß Anspruch 4, in welchen das an Z gebundene Dipeptidfragment eine Phe-CHA-, Phe-CHG-, Ph'Gly-CHA-, Ph'Gly-CHG-, Ph'Gly-HCA-, CHG-CHA- oder CHG-CHG-Gruppe ist.

9. Tripeptidderivate gemäß Ansprüchen 1 bis 4, dadurch gekennzeichnet, daß sie mit einer Mineralsäure, z. B. HCl, HBr, $H_2SO_4$ oder $H_3PO_4$, oder einer organischen Säure, z. B. Ameisensäure, Essigsäure, Propionsäure, Trimethylessigsäure, Methoxyessigsäure, Trichlor- oder Trifluoressigsäure, Glykokollsäure, Milchsäure, Oxalsäure, Malonsäure, Zitronensäure, Benzoesäure, einer im Kern substituierten aromatischen Säure, wie Toluylsäuren, Chlor- oder Brombenzoesäuren, Methoxybenzoesäuren und Aminobenzoesäuren, oder Phthalsäure, protonisiert sind.

10. Verfahren zur quantitativen Bestimmung von proteolytischen Enzymen der Enzymklasse E.C. 3.4.21., welche natürliche Peptidketten an der Carboxylseite des Arginins oder Lysins spalten, in Medien, welche die genannten Enzyme enthalten oder in welchen die letzteren gebildet oder verbraucht werden, dadurch gekennzeichnet, daß man das genannte Medium mit einem Tripeptidderivat der Formel I gemäß den Ansprüchen 1 bis 4 zur Reaktion bringt und die Menge des durch die hydrolytische Einwirkung des Enzyms auf das Tripeptidderivat gebildeten farbigen oder fluoreszierenden Spaltproduktes R–H durch photometrische, spektrophotometrische, fluoreszenzspektrophotometrische oder elektrochemische Methoden mißt.

11. Verfahren gemäß Anspruch 10, dadurch gekennzeichnet, daß man Organ- oder Glandulärkallikreine in menschlichen Körperflüssigkeiten, z. B. Urin, Pankreassaft, Darmschleim, Milchdrüsensekret, Schweißdrüsensekret, Sputum oder Blut, bestimmt.

12. Verfahren gemäß Anspruch 10, dadurch gekennzeichnet, daß man Plasmin in Blut oder Blutplasma bestimmt.

13. Verfahren gemäß Anspruch 10, dadurch gekennzeichnet, daß man Thrombin in Blut oder Blutplasma bestimmt.

**Claims for the contracting states: BE, CH, DE, FR, GB, IT, LI, LU, NL, SE**

1. Tripeptide derivatives having the formula

H–D–X–Y–Z–R I

wherein Z represents an arginyl or lysyl group, R represents a p-nitrophenylamino, 2-naphthylamino, 4-methoxy-2-maphthylamino, 4-methyl-coumaryl-(7)-amino, 1,3-di(methoxycarbonyl)-phenyl-(5)-amino, quinonylamino or nitroquinonylamino group, X represents a cyclohexylglycyl, cyclohexylalanyl or p-hydroxycyclohexylalanyl group, and Y represents an alanyl, α-aminobutyryl, valyl, norvalyl, leucyl, norleucyl, isoleucyl, prolyl or pipecolinoyl group, and salts thereof with acids.

2. Tripeptide derivatives having the formula given in claim 1, wherein Z and R have the meaning defined in claim 1, and Y represents a cyclohexylglycyl, cyclohexylalanyl, p-hydroxycyclohexylalanyl, phenylalanyl or phenylglycyl group, and Y represents a phenylalanyl, phenylglycyl or tyrosyl group, and salts thereof with acids.

3. Tripeptide derivatives having the formula given in claim 1, wherein Z and R have the meaning defined in claim 1, and X represents an alanyl, α-aminobutyryl, valyl, norvalyl, leucyl, norleucyl or isoleucyl group, and Y represents a cyclohexylglycyl, cyclohexylalanyl, p-hydroxycyclohexylalanyl, phenylglycyl or tyrosyl group, and salts thereof with acids.

4. Tripeptide derivatives having the formula given in claim 1, wherein Z and R have the meaning defined in claim 1, and X represents a phenylalanyl, phenylglycyl or cyclohexylglycyl group, and Y represents a cyclohexylalanyl, cyclohexylglycyl or p-hydroxycyclohexylalanyl group, and salts thereof with acids.

5. Tripeptide derivatives according to claim 1, wherein the dipeptide fragment attached to Z is a CHG-Ala, CHG-But, CHG-Norval, CHG-Leu, CHG-Norleu, CHG-Pro, CHG-Pip, CHA-Ala, CHA-But, CHA-Norval, CHA-Leu, CHA-Norleu, CHA-Pro, CHA-Pip, HCA-But, HCA-Leu, HCA-Pro or HCA-Pip group.

6. Tripeptide derivatives according to claim 2, wherein the dipeptide fragment attached to Z is a CHG-Phe, CHG-Ph'Gly, CHG-Tyr, CHA-Phe, CHA-Ph'Gly, CHA-Tyr, HCA-Phe, HCA-Ph'Gly, HCA-Tyr, Phe-Tyr, Ph'Gly-Phe or Ph'Gly-Tyr group.

7. Tripeptide derivatives according to claim 3, wherein the dipeptide fragment attached to Z is an Ala-CHA, Ala-Ph'Gly, But-CHA, Val-CHG, Val-CHA, Val-HCA, Norval-CHA, Norval-Ph'Gly, Norval-Tyr, Leu-CHA, Leu-Ph'Gly, Norleu-CHA or Ile-CHA group.

8. Tripeptide derivatives according to claim 4, wherein the dipeptide fragment attached to Z is a Phe-CHA, Phe-CHG, Ph'Gly-CHA, Ph'Gly-CHG, Ph'Gly-HCA, CHG-CHA or CHG-CHG group.

9. Tripeptide derivatives according to claims 1 to 4, characterized by the fact that they are protonated with a mineral acid, e. g. HCl, HBr, $H_2SO_4$ or $H_3PO_4$, or an organic acid, e. g. formic acid, acetic acid, propionic acid, trimethylacetic acid, methoxyacetic acid, trichloro or trifluoroacetic acid, aminoacetic acid, lactic acid, oxalic acid, malonic acid, citric acid, benzoic acid, an aromatic acid substituted in the nucleus such as toluic acids, chloro- or bromobenzoic acids, methoxybenzoic acids and aminobenzoic acids, or phthalic acid.

10. Method for quantitatively assaying proteloytic enzymes of enzyme class E.C. 3.4.21., which split natural peptide chains on the carboxyl side of arginine or lysine, in media which contain the said enzymes or in which the latter are formed or consumed, characterized by the fact that the said medium is reacted with a tripeptide derivative of formula I according to claims 1 to 4, and that the quantity of the coloured or fluorescent split product R – H formed by the hydrolytic action of the enzyme on the tripeptide derivative is measured by photomeric, spectrophotometric, fluorescence spectrophotometric or electrochemical methods.

11. Method according to claim 10, characterized by the fact that organ or glandular kallikreins are assayed in human body fluids, e. g. urine, pancreatic juice, intestinal mucosa, milk gland secretion, sweat gland secretion, sputum or blood.

12. Method according to claim 10, characterized by the fact that plasmin is assayed in blood or blood plasma.

13. Method according to claim 10, characterized by the fact that thrombin is assayed in blood or blood plasma.

**Revendications pour les Etats contractants: BE, CH, DE, FR, GB, IT, LI, LU, NL, SE**

1. Dérivés tripeptidiques ayant la formule

$$H-D-X-Y-Z-R \qquad I$$

dans laquelle Z représente un groupe arginyle ou lysyle, R représente un groupe p-nitrophénylamino, 2-naphtylamino, 4-méthoxy-2-naphtylamino, 4-méthyl-coumaryl-(7)-amino, 1,3-di(méthoxycarbonyl)-phényl-(5)-amino, quinonylamino ou nitroquinonylamino, X représente un groupe cyclohexylglycyle, cyclohexylalanyle ou p-hydroxycyclohexylalanyle et Y représente un groupe alanyle, α-aminobutyryle, valyle, norvalyle, leucyle, norleucyle, isoleucyle, prolyle ou pipécolinoyle, et leurs sels avec des acides.

2. Dérivés tripeptidiques ayant la formule indiquée dans la revendication 1, dans lesquels Z et R ont la signification définie dans la revendication 1 et X représente un groupe cyclohexylglycyle, cyclohexylalanyle, p-hydroxycyclohexylalanyle, phénylalanyle ou phénylglycyle et Y représente un

groupe phénylalanyle, phénylglycyle ou tyrosyle, et leurs sels avec des acides.

3. Dérivés tripeptidiques ayant la formule indiquée dans la revendication 1, dans lesquels Z et R ont la signification définie dans la revendication 1 et X représente un groupe alanyle, α-aminobutyryle, valyle, norvalyle, leucyle, norleucyle ou isoleucyle et Y représente un groupe cyclohexylglycyle, cyclohexylalanyle, p-hydroxycyclohexylalanyle, phénylglycyle ou tyrosyle, et leurs sels avec des acides.

4. Dérivés tripeptidiques ayant la formule indiquée dans la revendication 1, dans lesquels Z et R ont la signification définie dans la revendication 1 et X représente un groupe phénylalanyle, phénylglycyle ou cyclohexylglycyle et Y représente un groupe cyclohexylalanyle, cyclohexylglycyle ou p-hydroxycyclohexylalanyle, et leurs sels avec des acides.

5. Dérivés tripjeptidiques selon la revendication 1 dans lesquels le fragment dipeptidique lié à Z est un groupe CHG-Als, CHG-But, CHG-Norval, CHG-Leu, CHG-Norleu, CHG-Pro, CHG-Pip, CHA-Ala, CHA-But, CHA-Norval, CHA-Leu, CHA-Norleu, CHA-Pro, CHA-Pip, HCA-But, HCA-Leu, HCA-Pro, ou HCA-Pip.

6. Dérivés tripeptidiques selon la revendication 2 dans lesquels le fragment dipeptidique lié à Z est un groupe CHG-Phe, CHG-Ph'Gly, CHG-Tyr, CHA-Phe, CHA-Ph'Gly, CHA-Tyr, HCA-Phe, HCA-Ph'Gly, HCA-Tyr, Phe-Tyr, Ph'Gly-Phe ou Ph'Gly-Tyr.

7. Dérivés tripeptidiques selon la revendication 3 dans lesquels le fragment dipeptidique lié à Z est un groupe Ala-CHA, Ala-Ph'Gly, But-CHA, Val-CHG, Val-CHA, Val-HCA, Norval-CHA, Norval-Ph'Gly, Norval-Tyr, Leu-CHA, leu-Ph'Gly, Norleu-CHA ou Ile-CHA.

8. Dérivés tripeptidiques selon la revendication 4 dans lesquels le fragment dipeptidique lié à Z est un groupe Phe-CHA, Phe-CHG, Ph'Gly-CHA, Ph'Gly-CHG, Ph'Gly-HCA, CHG-CHA ou CHG-CHG.

9. Dérivés tripeptidiques selon les revendications 1 à 4, caractérisés en ce qu'ils sont protonisés par un acide minéral, p. ex. HCl, HBr, $H_2SO_4$ ou $H_3PO_4$, ou un acide organique, tel que l'acide formique, acétique, propionique, triméthylacétique, méthoxyacétique, trichloro ou trifluoroacétique, aminoacétique, lactique, oxalique, malonique, citrique, benzoïque, un acide aromatique substitué dans le noyau, tel que les acides toluyliques, chloro ou bromobenzoïques, méthoxybenzoïques et aminobenzoïques, ou l'acide phtalique.

10. Procédé pour la détermination quantitative d'enzymes protéolytiques de la classe d'enzymes E.C. 3.4.21., lesquesl scindent les chaînes peptidiques naturelles sur le côté carboxylique de l'arginine ou de la lysine dans des milieux comprenant lesdits enzymes ou dans lesquels ce derniers sont formés ou consommés, caractérisé en ce que l'on fait réagir ledit milieu avec un dérivé tripeptidique ayant la formule I selon les revendications 1 à 4 et que l'on mesure la quantité du produit de scission coloré ou fluorescent R−H formé par l'action hydrolytique de l'enzyme sur le dérivé tripeptidique par des méthodes photométriques, spectrophotométriques, spectrophotométriques de fluorescence ou électrochimiques.

11. Procédé selon la revendication 10, caractérisé en ce que l'on détermine des kallikréines organiques ou glandulaires dans des humeurs humaines, p. ex. de l'urine, du jus pancréatique, les muqueuses intestinales, la sécrétion des glandes lactiques, la sécrétion des glandes sudoripares, la salive et le sang.

12. Procédé selon la revendication 10, caractérisé en ce que l'on détermine la plasmine dans le sang ou le plasma sanguin.

13. Procédé selon la revendication 10, caractérisé en ce que l'on détermine la thrombine dans le sang ou le plasma sanguin.

**Patentansprüche für den Vertragsstaat AT**

1. Verfahren zur quantitativen Bestimmung von proteolytischen Enzymen der Enzymklasse E.C. 3.4.21., welche natürliche Peptidketten an der Carboxylseite des Arginins oder Lysins spalten, in Medien, welche die genannten Enzyme enthalten oder in welchen die letzteren gebildet oder verbraucht werden, dadurch gekennzeichnet, daß man das genannte Medium mit einem Tripeptidderivat der Formel

$$H-D-X-Y-Z-R \qquad I$$

in welcher Z eine Arginyl- oder Lysylgruppe und R eine p-Nitrophenylamino-, 2-Naphthylamino-, 4-Methoxy-2-naphthylamino-, 4-Methyl-cumaryl-(7)-amino-, 1,3-Di(methoxycarbonyl)-phenyl-(5)-amino-, Chinonylamino- oder Nitrochinonylamino-Gruppe darstellen, und

a) X eine Cyclohexylglycyl-, Cyclohexylalanyl- oder p-Hydroxycyclohexylalanyl-Gruppe und Y eine Alanyl-, α-Aminobutyryl-, Valyl-, Norvalyl-, Leucyl-, Norleucyl-, Isoleucyl-, Prolyl- oder Pipecolinoyl-Gruppe darstellen, oder
b) X eine Cyclohexylglycyl-, Cyclohexylalanyl-, p-Hydroxycyclohexylalanyl-, Phenylalanyl- oder Phenylglycyl-Gruppe und Y eine Phenylalanyl-, Phenylglycyl- oder Tyrosyl-Gruppe darstellen,

oder

c) X eine Alanyl-, α-Aminobutyryl-, Valyl-, Norvalyl-, Leucyl-, Norleucyl- oder Isoleucyl-Gruppe und Y eine Cyclohexylglycyl-, Cyclohexylalanyl-, p-Hydroxycyclohexylalanyl-, Phenylglycyl- oder Tyrosyl-Gruppe darstellen, oder
d) X eine Phenylalanyl-, Phenylglycyl- oder Cyclohexylglycyl-Gruppe und Y eine Cyclohexylalanyl-,

Cyclohexylglycyl- oder p-Hydroxycyclohexylalanyl-Gruppe darstellen,

oder einem Säureadditionssalz davon zur Reaktion bringt und die Menge des durch die hydrolytische Einwirkung des Enzyms auf das Tripeptidderivat gebildeten farbigen oder fluoreszierenden Spaltproduktes R–H durch photometrische, spektrophotometrische, fluoreszenzspektrophotometrische oder elektrochemische Methoden mißt.

2. Verfahren nach Anspruch 1, dadurch gekennzeichnet, daß man ein Tripeptidderivat verwendet, welches mit einer Mineralsäure, z. B. HCl, HBr, $H_2SO_4$ oder $H_3PO_4$, oder einer organischen Säure, z. B. Ameisensäure, Essigsäure, Propionsäure, Trimethylessigsäure, Methoxyessigsäure, Trichlor- oder Trifluoressigsäure, Glykokollsäure, Milchsäure, Oxalsäure, Malonsäure, Zitronensäure, Benzoesäure, einer im Kern substituierten aromatischen Säure, wie Toluylsäuren, Chlor- oder Brombenzoesäuren, Methoxybenzoesäuren und Aminobenzoesäuren, oder Phthalsäure, protonisiert ist.

3. Verfahren nach Anspruch 1, dadurch gekennzeichnet, daß man ein Tripeptidderivat verwendet, in welchem das an Z gebundene Dipeptidfragment eine CHG-Ala-, CHG-But, CHG-Norval-, CHG-Leu-, CHG-Norleu-, CHG-Pro-, CHG-Pip-, CHA-Ala-, CHA-But-, CHA-Norval-, CHA-Leu-, CHA-Norleu-, CHA-Pro-, CHA-Pip-, HCA-But-, HCA-Leu-, HCA-Pro- oder HCA-Pip-Gruppe, oder eine CHG-Phe-, CHG-Ph'Gly-, CHG-Tyr-, CHA-Phe-, CHA-Ph'Gly-, CHA-Tyr-, HCA-Phe-, HCA-Ph'Gly-, HCA-Tyr-, Phe-Tyr-, Ph'Gly-Phe- oder Ph'Gly-Tyr-Gruppe, oder eine Ala-CHA-, Ala-Ph'Gly-, But-CHA-, Val-CHG-, Val-CHA-, Val-HCA-, Norval-CHA-, Norval-Ph'Gly-, Norval-Tyr-, Leu-CHA-, Leu-Ph'Gly-, Norleu-CHA- oder Ile-CHA-Gruppe, oder eine Phe-CHA-, Phe-CHG-, Ph'Gly-CHA-, Ph'Gly-CHG, Ph'Gly-HCA-, CHG-CHA- oder CHG-CHG-Gruppe ist.

4. Verfahren nach Anspruch 1, dadurch gekennzeichnet, daß man Organ- oder Glandulärkallikreine in menschlichen Körperflüssigkeiten, z. B. Urin, Pankreassaft, Darmschleim, Milchdrüsensekret, Schweißdrüsensekret, Sputum oder Blut, bestimmt.

5. Verfahren nach Anspruch 1, dadurch gekennzeichnet, daß man Plasmin in Blut oder Blutplasma bestimmt.

6. Verfahren nach Anspruch 1, dadurch gekennzeichnet, daß man Thrombin in Blut oder Blutplasma bestimmt.

**Claims for the Contracting-State AT**

1. Method for quantitatively assaying proteolytic enzymes of enzyme class E.C. 3.4.21., which split natural peptide chains on the carboxyl side of arginie or lysine, in media which contain the said enzymes or in which the latter are formed or consumed, characterized by the fact that the said medium is reacted with a tripeptide derivative having the formula

$$H-D-X-Y-Z-R \qquad I$$

wherein Z represents an arginyl or lysyl group, and R represents a p-nitrophenylamino, 2-naphthylamino, 4-methoxy-2-naphthylamino, 4-methyl-coumaryl-(7)-amino, 1,3-di(methoxycarbonyl)-phenyl-(5)-amino, quinonylamino or nitroquinonylamino group, and

a) X represents a cyclohexylglycyl, cyclohexylalanyl or p-hydroxycyclohexylalanyl group and Y an alanyl, α-aminobutyryl, valyl, norvalyl, leucyl, norleucyl, isoleucyl, prolyl or pipecolinoyl group, or
b) X represents a cyclohexylglycyl, cyclohexylalanyl, p-hydroxycyclohexylalanyl, phenylalanyl or phenylglycyl group and Y represents a phenylalanyl, phenylglycyl or tyrosyl group, or
c) X represents an alanyl, α-aminobutyryl, valyl, norvalyl, leucyl, norleucyl, or isoleucyl group, and Y represents a cyclohexylglycyl, cyclohexylalanyl, p-hydroxycyclohexylalanyl, phenylglycyl or tyrosyl group, or
d) X represents a phenylalanyl, phenylglycyl or cyclohexylglycyl group and Y represents a cyclohexylalanyl, cyclohexylglycyl or p-hydroxycyclohexylalanyl group, or an acid addition salt thereof, and that the quantity of the coloured or fluorescent split product R–H formed by the hydrolytic action of the enzyme on the tripeptide derivative is measured by photometric, spectrophotometric, fluorescence spectrophotometric or electrochemical methods.

2. Method according to claim 1, characterized by using a tripeptide derivative which is protonated with a mineral acid, e. g. HCl, HBr, $H_2SO_4$ or $H_3PO_4$, or an organic acid, e. g. formic acid, acetic acid, propionic acid, trimethylacetic acid, methoxyacetic acid, trichloro or trifluoroacetic acid, aminoacetic

acid, lactic acid, oxalic acid, malonic acid, citric acid, benzoic acid, an aromatic acid substituted in the nucleus such as toluic acids, chloro- or bromobenzoic acids, methoxybenzoic acids and aminobenzoic acids, or phthalic acid.

3. Method according to claim 1, characterized by using a tripeptide derivative wherein the dipeptide fragment attached to Z is a CHG-Ala, CHG-But, CHG-Norval, CHG-Leu, CHG-Norleu, CHG-Pro, CHG-Pip, CHA-Ala, CHA-But, CHA-Norval, CHA-Leu, CHA-Norleu, CHA-Pro, CHA-Pip, HCA-But, HCA-Leu, HCA-Pro or HCA-Pip group, or a CHG-Phe, CHG-Ph'-Gly, CHG-Tyr, CHA-Phe, CHA-Ph'Gly, CHA-Tyr, HCA-Phe, HCA-Ph'Gly, HCA-Tyr, Phe-Tyr, Ph'Gly-Phe or Ph'Gly-Tyr group, or an Ala-CHA, Ala-Ph'Gly, But-CHA, Val-CHG, Val-CHA, Val-HCA, Norval-CHA, Norval-Ph'Gly, Norval-Tyr, Leu-CHA, Leu-Ph'Gly, Norleu-CHA or Ile-CHA group, or a Phe-CHA, Phe-CHG, Ph'Gly-CHA, Ph'Gly-CHG, Ph'Gly-HCA, CHG-CHA or CHG-CHG group.

4. Method according to claim 1, characterized by the fact that organ or glandular kallikreins are assayed in human body fluids, e. g. urine, pancreatic juice, intestinal mucosa, milk gland secretion, sweat gland secretion, sputum or blood.

5. Method according to claim 1, characterized by the fact that plasmin is assayed in blood or blood plasma.

6. Method according to claim 1, characterized by the fact that thrombin is assayed in blood or blood plasma.

**Revendications pour l'Etat AT**

1. Procédé pour la détermination quantitative d'enzymes protéolytiques de la classe d'enzymes E.C. 3.4.21., lesquels scindent les chaînes peptidiques naturelles sur le côté carboxylique de l'arginine ou de la lysine, dans des milieux comprenant lesdits enzymes ou dans lesquels ces derniers sont formés ou consommés, caractérisé en ce que l'on fait réagir ledit milieu avec un dérivé tripeptidique ayant la formule

$$H-D-X-Y-Z-R \qquad I$$

dans laquelle Z représente un groupe arginyle ou lysyle et R représente un groupe p-nitrophénylamino, 2-naphtylamino, 4-méthoxy-2-naphtylamino, 4-méthyl-coumaryl-(7)-amino, 1,3-di(méthoxycarbonyl)-phényl-(5)-amino, quinonylamino ou nitroquinonylamino et

a) X représente un groupe cyclohexylglycyle, cyclohexylalanyle ou p-hydroxycyclohexylalanyle et Y représente un groupe alanyle, $\alpha$-aminobutyryle, valyle, norvalyle, leucyle, norleucyle, isoleucyle, prolyle ou pipécolinoyle ou

b) X représente un groupe cyclohexylglycyle, cyclohexylalanyle, p-hydroxycyclohexylalanyle, phénylalanyle ou phénylglycyle et Y représente un groupe phénylalanyle, phénylglycyle ou tyrosyle ou

c) X représente un groupe alanyle, $\alpha$-aminobutyryle, valyle, norvalyle, leucyle, norleucyle ou isoleucyle et Y représente un groupe cyclohexylglycyle, cyclohexylalanyle, p-hydroxycyclohexylalanyle, phénylglycyle ou tyrosyle ou

d) X représente un groupe phénylalanyle, phénylglycyle ou cyclohexylglycyle et Y représente un groupe cyclohexylalanyle, cyclohexylglycyle ou p-hydroxycyclohexylalanyle, ou un de ses sels d'addition avec un acide, et que l'on mesure la quantité du produit de scission coloré ou fluorescent R – H formé par l'action hydrolytique de l'enzyme sur le dérivé tripeptidique par des méthodes photométriques, spectrophotométriques, spectrophotométriques de fluorescence ou électrochimiques.

2. Procédé selon la revendication 1, caractérisé en ce qu'on utilise un dérivé tripeptidique protonisé par un acide minéral, p. ex. HCl, HBr, $H_2SO_4$ ou $H_3PO_4$, ou un acide organique, tel que l'acide formique, acétique, propionique, triméthylacétique, méthoxyacétique, trichloro ou trifluoroacétique, aminoacétique, lactique, oxalique, malonique, citrique, benzoïque, un acide aromatique substitué dans le noyau, tels que les acides toluiques, chloro ou bromobenzoïques, méthoxybenzoïques et aminobenzoïques, ou l'acide phtalique.

3. Procédé selon la revendication 1, caractérisé en ce qu'on utilise un dérivé tripeptidique dans lequel le fragment dipeptidique lié à Z est un groupe CHG-Ala, CHG-But, CHG-Norval, CHG-Leu, CHG-Norleu, CHG-Pro, CHG-Pip, CHA-Ala, CHA-But, CHA-Norval, CHA-Leu, CHA-Norleu, CHA-Pro, CHA-Pip, HCA-But, HCA-Leu, HCA-Pro ou HCA-Pip ou un groupe CHG-Phe, CHG-Ph'Gly, CHG-Tyr, CHA-Phe, CHA-Ph'Gly, CHA-Tyr, HCA-Phe, HCA-Ph'Gly, HCA-Tyr, Phe-Tyr, Ph'Gly-Phe ou Ph'Gly-Tyr ou un groupe Ala-CHA, Ala-Ph'Gly, But-CHA, Val-CHG, Val-CHA, Val-HCA, Norval-CHA, Norval-Ph'Gly, Norval-Tyr, Leu-CHA, Leu-Ph'Gly, Norleu-CHA ou Ile-CHA ou un groupe Phe-CHA, Phe-CHG, Ph'Gly-CHA, Ph'Gly-CHG, Ph'Gly-HCA, CHG-CHA ou CHG-CHG.

4. Procédé selon la revendication 1, caractérisé en ce qu'on détermine des kallikréines d'organes ou

glandulaires dans des humeurs humaines, p. ex. de l'urine, du jus pancréatique, les muqueuses intestinales, la sécrétion des glandes lactiques, la sécrétion des glandes sudoripares, la salive et le sang.

5. Procédé selon la revendication 1, caractérisé en ce qu'on détermine la plasmine dans le sang ou le plasma sanguin.

6. Procédé selon la revendication 1, caractérisé en ce qu'on détermine la thrombine dans le sang ou le plasma sanguin.